# EUROPEAN PATENT APPLICATION

(11) **EP 4 582 441 A2**
(43) Date of publication of application: **09.07.2025**
(21) Application number: 24219623.6
(22) Date of filing: 12.12.2024
(51) Int. Cl.: C07K 14/605, C07K 14/575, C07K 14/705, A61K 38/00

(54) **MULTIMERIC POLYPEPTIDES AND USES THEREOF**

(30) Priority: 12.12.2023 US 202363609169 P
(71) Applicant: Amide Technologies, Waltham Massachusetts 02451 (US)
(72) Inventor: GLEASON, Patrick R., Waltham 02451 (US); JEDHE, Ganesh, Waltham 02451 (US); SIMON, Mark David, Waltham 02451 (US); PENTELUTE, Bradley L., Waltham 02451 (US)
(74) Representative: Pearl Cohen Zedek Latzer Baratz UK LLP

(57) **Abstract**

The present disclosure provides multimeric polypeptides comprising at least one G-coupled polypeptide receptor (GPCR) ligand for treatment of various conditions and diseases.

## Description

### FIELD OF THE INVENTION

Described herein are multimeric polypeptides comprising at least one G-coupled polypeptide receptor (GPCR) ligand for treatment of various conditions and diseases.

### BACKGROUND

G-protein coupled receptors (GPCRs) are transmembrane proteins with seven alpha-helical segments separated by alternating intracellular and extracellular loop regions. The primary function of GPCRs is the transduction of extracellular stimuli into intracellular signals. GPCRs undertake a plethora of essential physiological functions and are targets for numerous approved drugs and those under development. Currently, approximately thirty to forty percent of marketed pharmaceuticals target GPCRs.

It is toward preparing new compounds targeting GPCRs for improved safety and efficacy that the disclosure is directed.

### SUMMARY

In one aspect, a multimeric polypeptide is provided comprising:
at least two polypeptide units, wherein each polypeptide unit is covalently linked to at least one other polypeptide unit via a linker moiety;
wherein at least one polypeptide is a G-coupled polypeptide receptor (GPCR) ligand.

In some embodiments, the multimeric polypeptide comprises two, three, four, five, or at least six polypeptide units. In some embodiments, each of the polypeptide units comprises a different amino acid sequence from each of the other polypeptide units. In some embodiments, at least one of the polypeptide units comprises the same amino acid sequence as another polypeptide unit.

In some embodiments, each of the polypeptide units is bound to a linker moiety. In some embodiments, the linker moiety comprises a linear or branched structure comprising a plurality of reactive sites that covalently link the at least two polypeptides. In some embodiments, the plurality of reactive sites of the linker moiety comprise a combination of one or more independent amine, thiol, or halogen reactive sites. In some embodiments, the plurality of reactive sites of the linker moiety comprise one or more azides or alkynes. In some embodiments,, the plurality of reactive sites of the linker moiety comprise one or more orthogonal protecting groups that may be removed independently of other protecting groups.

In some embodiments, the linker moiety comprises a polymer linker. In some embodiments, the linker moiety comprises an alpha amino acid, a beta-amino acid or a gamma-amino acid. In some embodiments, the linker moiety comprises a D-amino acid. In some embodiments, the linker moiety comprises an ethylene glycol oligomer. In some embodiments, the linker moiety comprises one or more independent PEG4, PEG8, PEG20, 2x(GGGGS), 4x(GGGGS), 8xG, GSAGSAAGSGEF, A-(EAAAK)-A, A-3x(EAAAK)-A, 7x(AP), KESGSVSSEQLAQFRSLD or EGKSSGSGSESKST.

In some embodiments, the multimeric polypeptide comprising at least one non-peptidic molecule is covalently linked to any portion of any of the polypeptide units or the linker moiety. In some embodiments, the non-peptidic molecule is linked by an amido-PEG-acid or maleimide-PEG-acid.

In some embodiments, the at least one non-peptidic molecule is a sodium-glucose cotransporter-2 (SGLT-2) inhibitor. In some embodiments, the at least one non-peptidic molecule is a lipid, albumin or antibody. In some embodiments, the at least one non-peptidic molecule is a lipid. In some embodiments, the lipid is bound to a lysine.

In some embodiments, at least one polypeptide comprises at least one modified amino acid, wherein the modification is any of acetylation, amidation, C-linked glycosylation, citrullination, crotonylation, formylation, glutarylation, glutathionylation, hydroxylation, lipidation, malonylation, methylation, myristoylation, N-linked glycosylation, O-linked glycosylation, oxidation, palmitoylation, phosphorylation, pyrrolidone carboxylic acid, S-nitrosylation, sulfation, or sumoylation.

In some embodiments, at least one of the polypeptide units is N-terminus acetylated.

In some embodiments, at least one of the polypeptide units comprises one or more non-canonical amino acid. In some embodiments, the non-canonical amino acid is 2-aminoisobutyric acid.

In some embodiments, the SGLT-2 inhibitor is represented by Formula XIII 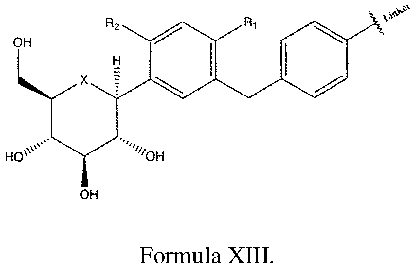 wherein
"Linker" refers to a linker covalently linking to the polypeptide unit
R₁ = -H, -OH, or a halogen;
R₂ = -H, -OH, or a halogen; and
X = S or O.

In some embodiments, the GPCR ligand is to GLP-1R, GIPR, or CRLR.

In some embodiments, the multimeric polypeptide comprises polypeptide sequences with greater than about 70% sequence homology to the respective native GPCR ligand.

In some embodiments, the multimeric polypeptide comprises polypeptide sequences of:
a. GLP-1 and GIP;
b. GLP-1 and an amylin variant; or
c. GLP-1, GIP, and an amylin variant;

optionally wherein at least one lysine of one or more polypeptides is lipidated, and
optionally wherein a conjugate molecule is bound thereto, and wherein each GPCR ligand
has greater than about 70% homology with the respective native GPCR ligand.

In some embodiments, at least one or all of the polypeptide units comprise native GPCR ligands.

In some embodiments, the GLP-1 polypeptide unit comprises a peptide with at least about 70% sequence homology to Ac-HXEGT FTSDV SSYLE GQAAK EFIAW LVKGR G;
wherein Ac refers to an N-terminal acetyl, X refers to 2-aminoisobutyric acid or alanine, and K refers to an optionally lipidated lysine residue.

In some embodiments, the GLP-1 polypeptide unit comprises:
Ac-H[Aib]EGTFTSDVSSYLEGQAAKEFIAWLVKGR.

In some embodiments, the GLP-1 polypeptide unit comprises:
Ac-HAEGTFTSDVSSYLEGQAAKEFIAWLVKGR.

In some embodiments, the GLP-1 polypeptide unit comprises:
H[Aib]EGTFTSDVSSYLEGQAAKEFIAWLVKGR.

In some embodiments, the GLP-1 polypeptide unit comprises:
HAEGTFTSDVSSYLEGQAAKEFIAWLVKGR.

In some embodiments, the GIP polypeptide unit comprises a peptide with at least about 70% sequence homology to
Ac-YXEGT FISDY SIAMD KIHQQ DFVNW LLAQK GKKND WKHNI TQ;
wherein Ac refers to an N-terminal acetyl, X refers to 2-aminoisobutyric acid or alanine, and K refers to an optionally lipidated lysine residue.

In some embodiments, the GIP polypeptide unit comprises:
Ac-Y[Aib]EGTFISDYSIAMDKIHQQDFVNWLLAQKGKKNDWKHNITQ.

In some embodiments, the GIP polypeptide unit comprises:
Ac-YAEGTFISDYSIAMDKIHQQDFVNWLLAQKGKKNDWKHNITQ.

In some embodiments, the GIP polypeptide unit comprises:
Y[Aib]EGTFISDYSIAMDKIHQQDFVNWLLAQKGKKNDWKHNITQ.

In some embodiments, the GIP polypeptide unit comprises:
YAEGTFISDYSIAMDKIHQQDFVNWLLAQKGKKNDWKHNITQ.

In some embodiments, the amylin polypeptide unit comprises a peptide with at least about 70% sequence homology to
Ac-KCNTA TCATQ RLAEF LRHSS NNFGP ILPPT NVGSN TP
wherein Ac refers to an N-terminal acetyl, and K refers to an optionally lipidated lysine residue.

In some embodiments, the multimeric polypeptide is: 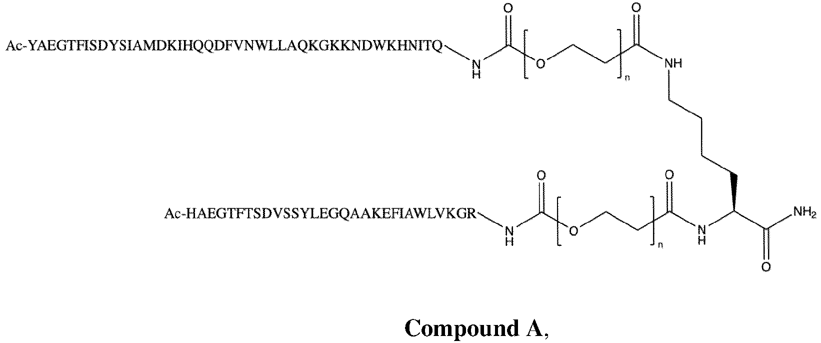 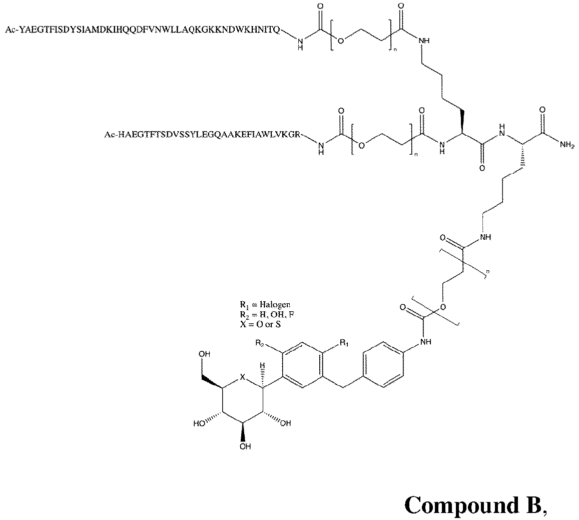 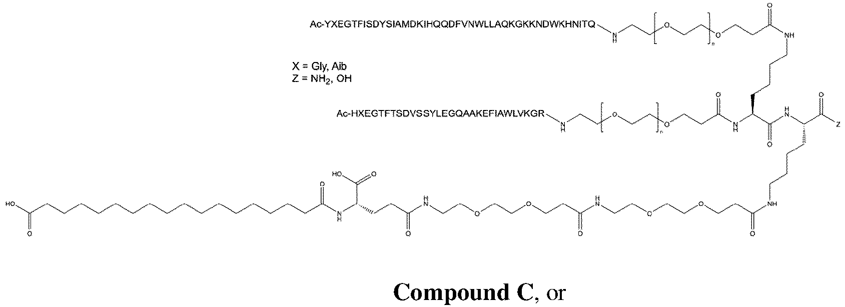 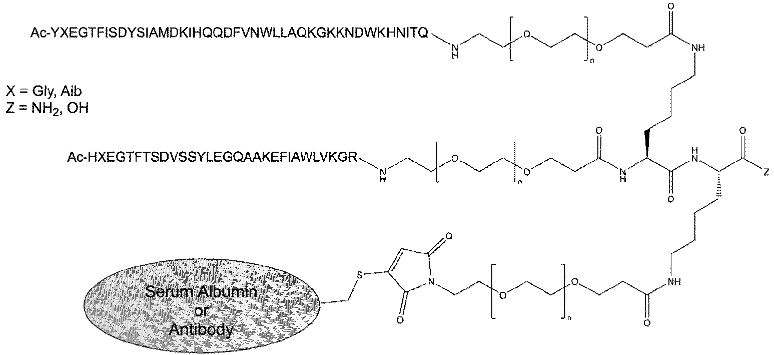 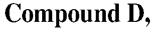 wherein n is any number selected from between 1 to 20.

### In some embodiments, the multimeric polypeptide is represented by the formula of Compound A-1

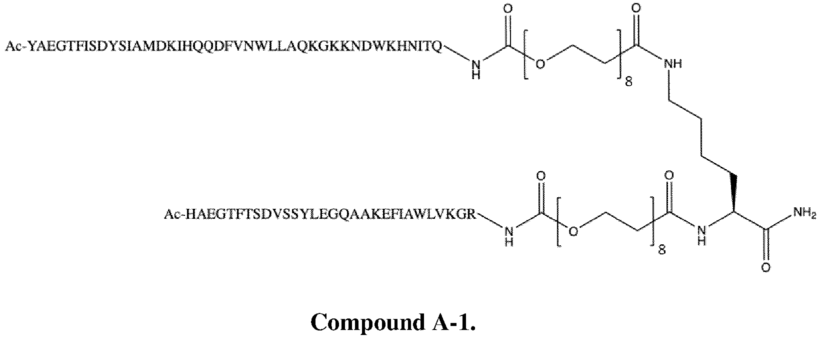

In some embodiments, the multimeric polypeptide is represented by any one of Formulas I through XII:

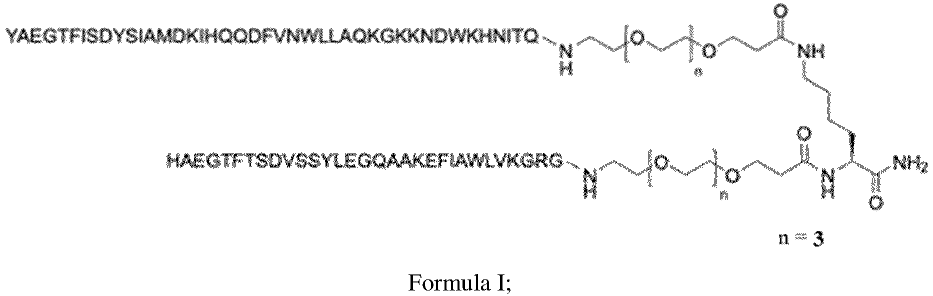

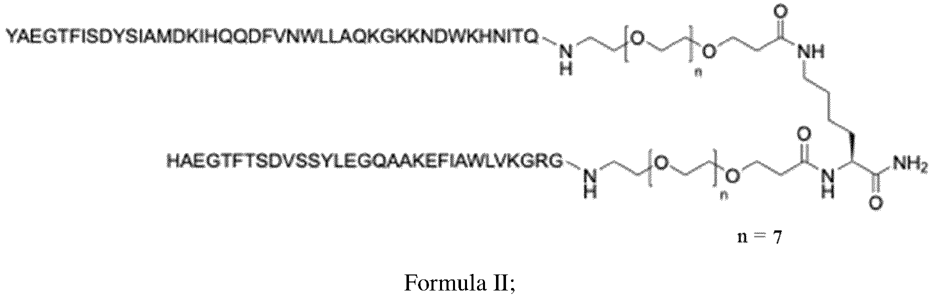

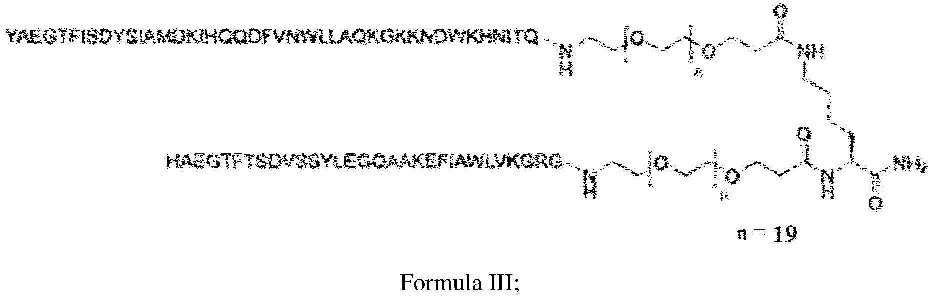

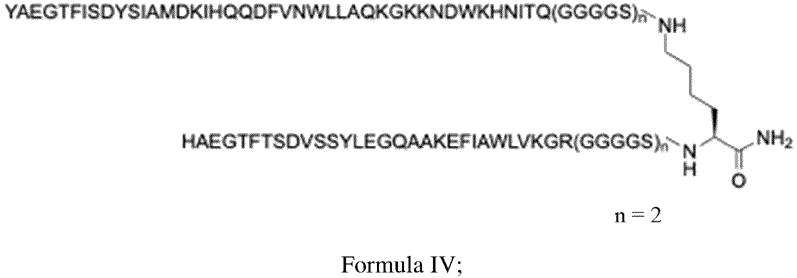

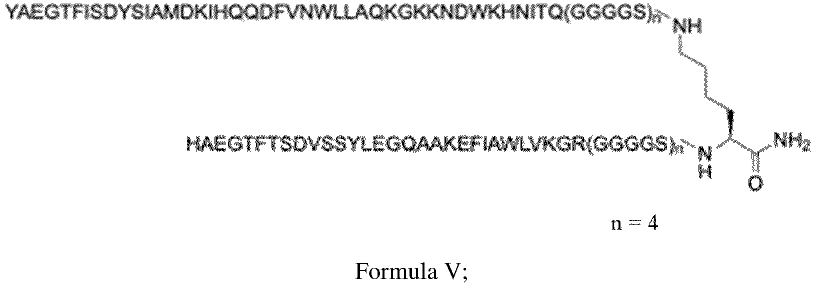

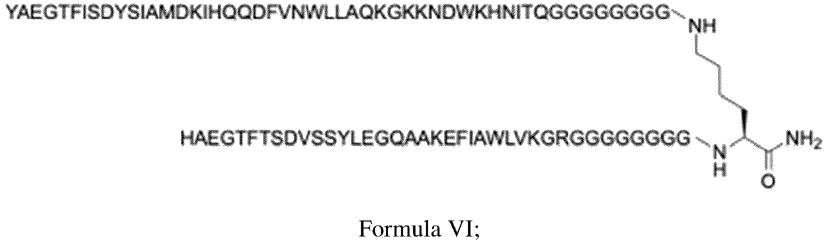

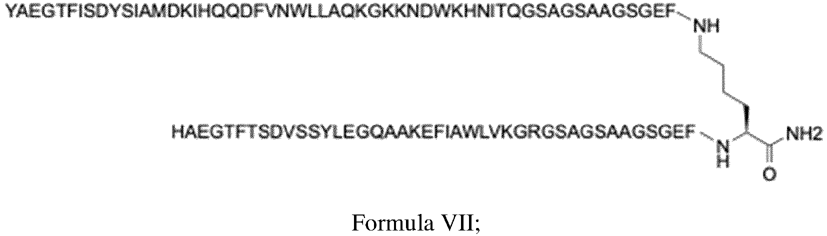

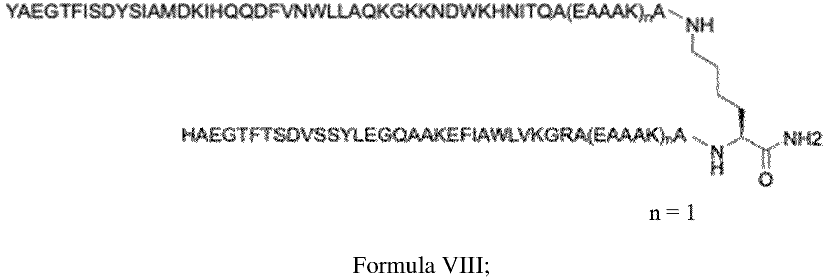

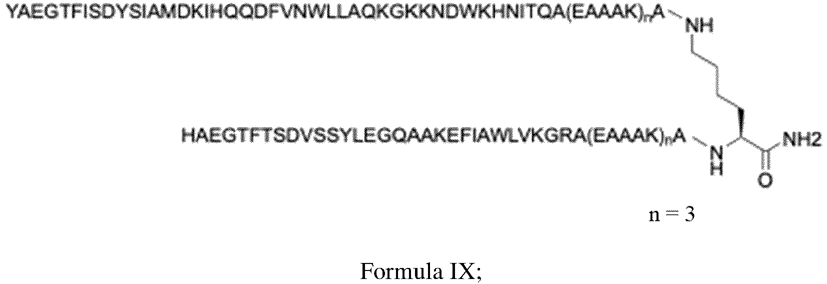

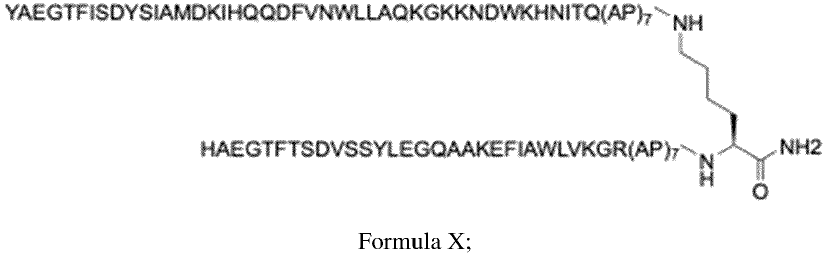

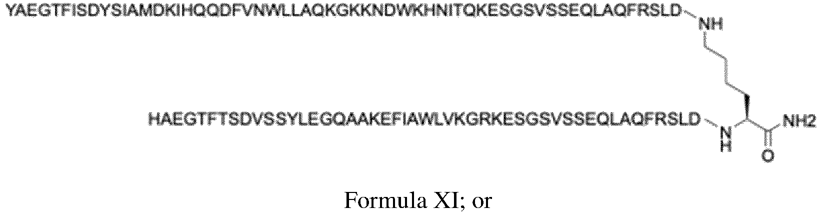

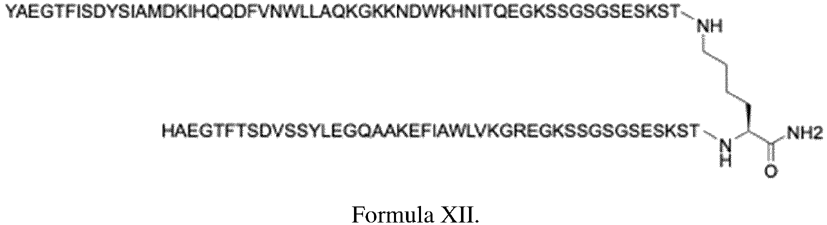

In one aspect, the GIP polypeptide unit is attached to the linker at the C-terminus of said GIP polypeptide.

In one aspect, the GLP-1 polypeptide unit is attached to the linker at the C-terminus of said GLP-1 polypeptide.

In some embodiments, the multimeric polypeptide comprises a GLP-1 polypeptide unit linked via a linker moiety to a GIP polypeptide unit, an amylin polypeptide unit, or the combination thereof. In some embodiments, the multimeric polypeptide further comprises a small-molecule SGLT2 inhibitor covalently linked to any one of the units.

In any of the foregoing aspects or embodiments, a linker moiety is bound to an amine of a C-terminal lysine appended to the C-terminus of each polypeptide unit.

In one aspect, a pharmaceutical composition is provided comprising a multimeric polypeptide as disclosed herein, and a pharmaceutically-acceptable excipient, diluent, vehicle or carrier.

In some embodiments, the pharmaceutical composition comprises 0.05% polysorbate-80. In some embodiments, the pharmaceutical composition comprises 50 mM sodium phosphate. In some embodiments, the pharmaceutical composition comprises 70 mM sodium chloride. In some embodiments, the pharmaceutical composition is at a pH of 7.4.

In one aspect, the pharmaceutical composition comprises 0.05% polysorbate-80, 50 mM sodium phosphate, and 70 mM sodium chloride at a of pH 7.4.

In one aspect, a method is provided for modulating at least one GPCR in a subject comprising administering to the subject a multimeric polypeptide as disclosed herein or a pharmaceutical composition comprising a multimeric polypeptide as disclosed herein. In some embodiments, GLP-1R and GIPR are modulated. In some embodiments, GLP-1R, GIPR and CRLR are modulated.

In one aspect, a method is provided for treating obesity or type 2 diabetes comprising administering to a subject in need an effective amount of a multimeric polypeptide as disclosed herein or a pharmaceutical composition comprising a multimeric polypeptide as disclosed herein.

In one aspect, a method is provided for treating obesity and type 2 diabetes comprising administering to a subject in need an effective amount of a multimeric polypeptide as disclosed herein or a pharmaceutical composition comprising a multimeric polypeptide as disclosed herein.

In some embodiments, the multimeric polypeptide in the methods provided is administered at between 1 nmoles/kg to 80 nmoles/kg of patient body weight. In some embodiments, the multimeric polypeptide in the methods provided is administered at 1 nmol/kg, 2 nmol/kg, 4 nmol/kg, 5 nmol/kg, 10 nmol/kg, and 20 nmol/kg, or 40 nmol/kg of patient body weight.

In some embodiments, the administration in the methods provided reduces fat without adversely affecting muscle mass.

In some embodiments, the pharmaceutical composition in the methods provided is administered subcutaneously.

These and other aspects of the invention will be appreciated from the ensuing detailed description.

### BRIEF DESCRIPTION OF THE DRAWINGS

The patent or patent application file contains at least one drawing executed in color. Copies of this patent or patent application publication with color drawing(s) will be provided by the Office upon request and payment of the necessary fee.

The present disclosure of multimeric polypeptides, both as to their generation and method of use, together with objects, features, and advantages thereof, may best be understood by reference to the following detailed description when read with the accompanying drawings in which:
**FIGS. 1A** and **1B** show a plotted graph of the cAMP assay results for GLP1 and GIP receptor activation assays. **FIG 1A** shows a plotted graph of the cAMP assay results for GLP1 receptor activation for Construct 01 through 12, Tirzepatide, and Retatrutide. **FIG. 1B** shows a plotted graph of the cAMP assay results for GIP receptor activation for Construct 01 through 12, Tirzepatide, and Retatrutide.
**FIG. 2A** shows UV-Vis Spectrum (λ = 210 nm) for Construct 01, and **FIG. 2B** shows the mass spectrum of Construct 01.
**FIG. 3A** shows UV-Vis Spectrum (λ = 210 nm) for Construct 02, and **FIG. 3B** shows the mass spectrum of Construct 02.
**FIG. 4A** shows UV-Vis Spectrum (λ = 210 nm) for Construct 03, and **FIG. 4B** shows the mass spectrum of Construct 03.
**FIG. 5A** shows UV-Vis Spectrum (λ = 210 nm) for Construct 04, and **FIG. 5B** shows the mass spectrum of Construct 04.
**FIG. 6A** shows UV-Vis Spectrum (λ = 210 nm) for Construct 05, and **FIG. 6B** shows the mass spectrum of Construct 05.
**FIG. 7A** shows UV-Vis Spectrum (λ = 210 nm) for Construct 06, and **FIG. 7B** shows the mass spectrum of Construct 06.
**FIG. 8A** shows UV-Vis Spectrum (λ = 210 nm) for Construct 07, and **FIG. 8B** shows the mass spectrum of Construct 07.
**FIG. 9A** shows UV-Vis Spectrum (λ = 210 nm) for Construct 08, and **FIG. 9B** shows the mass spectrum of Construct 08.
**FIG. 10A** shows UV-Vis Spectrum (λ = 210 nm) for Construct 09, and **FIG. 10B** shows the mass spectrum of Construct 09.
**FIG. 11A** shows UV-Vis Spectrum (λ = 210 nm) for Construct 10, and **FIG. 11B** shows the mass spectrum of Construct 10.
**FIG. 12A** shows UV-Vis Spectrum (λ = 210 nm) for Construct 11, and **FIG. 12B** shows the mass spectrum of Construct 11.
**FIG. 13A** shows UV-Vis Spectrum (λ = 210 nm) for Construct 12, and **FIG. 13B** shows the mass spectrum of Construct 12.
**FIG. 14A** shows UV-Vis Spectrum (λ = 210 nm) for Tirzepatide, and **FIG. 14B** shows the mass spectrum of Tirzepatide.
**FIG. 15A** shows UV-Vis Spectrum (λ = 210 nm) for Retatrutide, and **FIG. 15B** shows the mass spectrum of Retatrutide.
**FIG. 16** shows the circular dichroism (CD) spectrum of Construct 01 in water.
**FIG. 17** shows the circular dichroism (CD) spectrum of Construct 02 in water.
**FIG. 18** shows the circular dichroism (CD) spectrum of Construct 03 in water.
**FIG. 19** shows the circular dichroism (CD) spectrum of Construct 04 in water.
**FIG. 20** shows the circular dichroism (CD) spectrum of Construct 05 in water.
**FIG. 21** shows the circular dichroism (CD) spectrum of Construct 06 in water.
**FIG. 22** shows the circular dichroism (CD) spectrum of Construct 07 in water.
**FIG. 23** shows the circular dichroism (CD) spectrum of Construct 08 in water.
**FIG. 24** shows the circular dichroism (CD) spectrum of Construct 09 in water.
**FIG. 25** shows the circular dichroism (CD) spectrum of Construct 10 in water.
**FIG. 26** shows the circular dichroism (CD) spectrum of Construct 11 in water.
**FIG. 27** shows the circular dichroism (CD) spectrum of Construct 12 in water.
**FIG. 28** shows the average body weight changes in diet-induced obese (DIO) mice over 30 days of BXL-801 treatment. Four groups were compared: vehicle control and three BXL-801 dose ranges (1-4 nmol/kg, 5-20 nmol/kg, and 10-40 nmol/kg).
**FIG. 29** shows the correlation of cumulative food intake to weight loss for each treatment group.
**FIG. 30** shows the weight Loss versus food intake with processing efficiency during the study for each dose group and illustrates the slopes of weight loss vs. food intake regression lines for each dose group.
**FIGS. 31A** through **31C** show the changes in soft weight **(****FIG. 31A****),** lean weight **(****FIG. 31B****),** and fat weight **(****FIG. 31C****)** across four groups, Vehicle, 1-4 nmol/kg of BXL-801, 5-20 nmol/kg of BXL-801, and 10-40 nmol/kg of BXL-801.
**FIG. 32** shows the oral glucose tolerance (OGTT) results for diet-induced obese (DIO) mice across four groups: Vehicle (control), 1-4 nmol/kg of BXL-801, 5-20 nmol/kg of BXL-801, and 10-40 nmol/kg of BXL-801.
**FIG. 33A** shows the bone mineral content (BMC).
**FIG. 33B** shows bone mineral density (BMD) results from the study for all dosage groups.

### DETAILED DESCRIPTION

The present disclosure is directed to multimeric polypeptide that comprise at least two polypeptide units, wherein each polypeptide unit is covalently linked to at least one other polypeptide unit via a linker moiety, and wherein at least one polypeptide is a G-coupled polypeptide receptor (GPCR) ligand. Such multimeric polypeptides, and pharmaceutical compositions thereof, are useful in treating a number of conditions and diseases such as obesity and type 2 diabetes (T2D).

In some embodiments, multimeric polypeptides comprising two or more GPCR ligands may be considered polyagonists: single molecules that act on multiple receptors involved for example in metabolic homeostasis. As will be described in further detail below, in one embodiment, a GIPR/GLP1R dual agonist is provided.

### G-PROTEIN COUPLED RECEPTORS AND LIGANDS THEREOF

G-protein coupled receptors (GPCRs) are transmembrane proteins with seven alpha-helical segments separated by alternating intracellular and extracellular loop regions. The primary function of GPCRs is the transduction of extracellular stimuli into intracellular signals. GPCRs undertake a plethora of essential physiological functions and are targets for numerous approved drugs and those under development; the present disclosure provides multimeric polypeptides comprising two or more GPCR ligands, which may be the same or different, for improved activity, specificity, therapeutic index, and/or other features compared to approved drugs. Currently, approximately thirty to forty percent of marketed pharmaceuticals target GPCRs. Hence, there is enormous potential for the development of new drugs targeting these receptors.

The G-protein coupled receptor superfamily consists of structurally similar proteins arranged into families (classes). The human genome contains approximately 800 GPCRs making it the largest family of membrane proteins. The diversity of GPCRs has resulted in a perceived difficulty in developing a comprehensive classification system. In one classification system, the A-F system, the GPCRs are separated into six families on the basis of sequence homology and functional similarity. Family B (secretin receptor family) of the A-F system of classification contains 15 members including glucagon-like peptide receptors (GLPRs) such as GLP-1R, calcitonin receptors, calcitonin receptor-like receptors, and gastric inhibitory polypeptide receptors (GIPRs). These 15 receptors share between 21 and 67% sequence identity. The majority of the receptors within this family contain conserved cysteine residues that make up a cluster of cysteine bridges in the N-terminus. The binding profile of the secretin receptors is outlined by three binding domains comprising the proximal region and the juxtamembrane region of the N-terminus, as well as the extracellular loops, together with TM6 (*see e.g.* Odoemelam et al., G-Protein Coupled Receptors: Structure and Function in Drug Discovery. RSC Adv., 10:36337-36348 (2020)). The ligand is thought to activate the receptor by spanning the N terminal and the TMD extracellular loops. The secretin receptors have immense potential in drug discovery due to their importance in fundamental homeostatic functions. For example, glucagon-like peptide receptors (such as GLP-1R and GLP-2R) are particularly relevant targets due to the role they play in appetite control and the treatment of type 2 diabetes.

Upon stimulation by an extracellular stimuli (ligand), GPCRs undergo conformational changes, and triggers downstream intracellular signals by coupling with G proteins (or other intracellular proteins such as arrestins). A ligand can activate or block an agonist-induced GPCR resulting in agonism or antagonism, respectively, of the receptor signaling. A ligand can also be biased towards one signaling pathway over another relative to a reference ligand. This means that receptor activation induced by a biased ligand can result in selective G protein recruitment and reduced arrestin recruitment compared to the endogenous ligand, thereby causing a diverse cellular response. This concept has been shown for several agonists targeting the GLP-1R, such as the Val8-GLP-1 and several variants of exendin 4.

Agents targeting GPCRs for medicinal uses continue to expand in the past decades. Examples of these agents include exogenous small molecules such as traditionally developed synthetic organics, natural products, and inorganics. Additionally, these targeting agents can be biologicals such as peptides, antibodies, and metabolites. A number of peptide drugs have been approved recently, with many more in different stages of clinical development (*see e.g.* Yang et al., G-Protein Coupled Receptors: Structure- and Function-Based Drug Discovery. Signal Transduct. Target Ther., 6:7 (2021)). Naturally occurring peptides have been continually discovered from plants, animals, fungi, and bacteria. Although they act as efficient chemical messengers to modulate cellular functions, these peptides suffer from unfavorable pharmacokinetic and pharmacodynamics properties, such as very short plasma half-lives and low plasma protein binding. Therefore, chemical modifications are required to promote the membrane permeability, brain penetration, and oral bioavailability. Available strategies include peptide cyclization, N-methylation, palmitoylation, unnatural amino acid insertion, peptide-small molecule conjugation, and peptide self-assembly.

The present disclosure provides two or more same or different polypeptide GPCR ligands on a single molecule by use of one or more linkers, providing one or more improved features. The two or more same or different polypeptide GPCR ligands are among those described below, but the disclosure is not so limiting and embraces any other GPCR ligands.

### GLUCAGON-LIKE PEPTIDE-1 RECEPTOR (GLP-1R)

Multimeric polypeptides disclosed herein may comprise one or more ligands of GLP-1R.

The glucagon-like peptide-1 receptor (GLP-1R) was first identified by radioligand binding experiments and measurements of cyclic AMP accumulation using a rat insulinoma cell line. The human GLP-1R is located on chromosome 6 (6p21). The GLP-1R sequence contains a large hydrophilic extracellular domain and seven hydrophobic transmembrane domains. The GLP-1R protein has three potential N-linked glycosylation sites, and glycosylation may modulate receptor function.

The GLP-1R is expressed in lung; kidney; stomach; heart; intestine; α, β, and d cells of the pancreatic islets; and multiple regions of the central nervous system. It consists of three domains: the extracellular N-terminus, the transmembrane core domain, and an intracellular C-terminal domain. The large N-terminus binds the C-terminus of the GLP-1 peptide, while the transmembrane domain binds the N-terminus of the GLP-1 peptide. The GLP-1R is activated by a two-step binding mechanism upon binding of their endogenous ligands. According to this model, the ligand binding to the receptor is initiated by an interaction of the extra-cellular domain of the receptor with the C-terminal alpha helical part of the ligand. Then the ligand N-terminus is docked into the transmembrane domain of the receptor. This results in receptor activation and downstream signaling e.g. through G proteins.

To stimulate insulin secretion, and in the presence of elevated blood glucose concentrations, GLP-1R activation in pancreatic beta cells promote recruitment and activation of Gαs protein leading to adenylate cyclase-mediated cAMP production, elevation of Ca²⁺, and ERK1/2 phosphorylation. cAMP production lead to activation of protein kinase A (PKA) as well as exchange protein directly activated by cAMP (EPAC) and is directly involved in increasing proinsulin gene transcription and subsequent insulin secretion. Activated PKA and EPAC enhance insulin granular exocytosis by insulin granular priming and phosphorylates sulfonylurea receptor (SUR1) K_{ATP} channel subunit and thereby closes K_{ATP}-channels in the plasma membrane, leading to membrane depolarization, and opening of the voltage gated Ca²⁺-channels. This leads to an increased influx of extracellular Ca²⁺, which triggers fusion of intracellular insulin-containing granules with the plasma membrane and thereby insulin secretion. Additionally, PKA activates transducer of regulated CREB (TORC2) and cAMP response element-binding protein (CREB), which leads to beta cell proliferation through CREB- and TORC2-mediated IRS2 gene expression. In both rodent and human beta cells, EPAC2 also inhibits the function of the K_{ATP}-channels through activation of SUR1. Furthermore, CRE activation leads to stimulation of beta cell proliferation through CREB-mediated IRS-2 gene expression. The activated CREB also results in higher Bcl-2 activity, and inhibition of the pro-apoptotic bax, which is involved in beta cell survival processes and thereby maintain and promote beta cell functions. GLP-1 also stimulates expression of GLUT2 transporters and glucokinase leading to an increased intracellular glucose concentration and increased glycolysis.

For the non-G protein-dependent pathways, GLP-1R activation leads to c-SRC kinase-mediated transactivation of the epidermal growth factor tyrosine kinase receptor (EGFR). This leads to activation of PI3K, which activates AKT/PK, leading to phosphorylation of the nuclear transcription factor Foxo1. Phosphorylated Foxo1 can inactivate PDX-1, which in murine beta cells, has been demonstrated to lead to anti-apoptotic effects. Moreover, beta arrestin 1 binds to the GLP-1R upon activation by GLP-1 where the conformational receptor changes initiate phosphorylation of transmembrane helix 7 by G protein-coupled receptor kinases (GRKs) resulting in recruitment of beta arrestins. The beta arrestin recruitment results in ERK1/2 activation, beta cell proliferative and insulin secretion. Additionally, beta arrestin 1 also plays a role in receptor desensitization by at least two mechanisms: binding to the receptor and, thus, preventing G protein recruitment and by promoting receptor trafficking by internalization and recycling, although arrestin-independent internalization of the GLP-1R has been reported. Furthermore, GLP-1R internalization is mediated by agonist-induced stimulation of the G_{αq} pathway, which leads to receptor recycling to the plasma membrane or transport to lysosomes resulting in proteolysis. Beside this, G_{αq} coupling leads to hydrolysis of PIP₂ via phospholipase C activation, an increased Ca²⁺ accumulation, and phosphorylation of ERK 1/2 and beta cell proliferative processes. Thus, multiple pathways are activated in the beta cell downstream of the GLP-1R.

### Ligands of GLP-1R

Glucagon-like peptide 1 (GLP-1) binds to GLP-1R. GLP-1 has attracted much interest as a treatment for type 2 diabetes because it has multiple antidiabetic actions and, at the same time, lowers body weight. GLP-1 is a gastrointestinal peptide that is released in response to food intake. GLP-1 plays an important role in glucose homeostasis and augments glucose-induced insulin secretion and inhibits glucagon secretion. GLP-1 is also proposed to act as a satiety factor. Thus, peripheral administration of GLP-1 inhibits food intake. In addition to being produced in the intestine, GLP-1 is expressed at hypothalamic sites, the caudal portion of the nucleus of the solitary tract, and the forebrain. Central administration of GLP-1 inhibits food intake through actions in the hypothalamus.

GLP-1 was first identified in the early 1980s, following the cloning of the proglucagon gene. GLP-1 is a 30- or 31-amino-acid-long peptide hormone deriving from the tissue-specific posttranslational processing of the proglucagon peptide. The initial product GLP-1 (1-37) is susceptible to amidation and proteolytic cleavage, which gives rise to the two truncated and equipotent biologically active forms, GLP-1 (7-36) amide and GLP-1 (7-37). Active GLP-1 protein secondary structure includes two α-helices from amino acid position 13-20 and 24-35 separated by a linker region. Such peptides or variants thereof independently may be a component or components of a multimeric polypeptide disclosed herein.

Endogenous GLP-1 is rapidly degraded primarily by dipeptidyl peptidase-4 (DPP-4), as well as neutral endopeptidase 24.11 (NEP 24.11) and renal clearance, resulting in a half-life of approximately 2 minutes. Consequently, only 10-15 % of GLP-1 reaches circulation intact, leading to fasting plasma levels of only 0-15 pmol/L. To overcome this, GLP-1 receptor agonists and DPP-4 inhibitors have been developed to increase GLP-1 activity. As opposed to common treatment agents such as insulin and sulphonylurea, GLP-1-based treatment has been associated with weight loss and a lower risk of hypoglycemia, two important considerations for patients with type 2 diabetes.

GLP-1 possesses several physiological properties making it (and its functional analogs) a subject of intensive investigation as a potential treatment of diabetes mellitus, as these actions induce long-term improvements along with the immediate effects. The most noteworthy effect of GLP-1 is its ability to promote insulin secretion in a glucose-dependent manner. Additionally, GLP-1 ensures the β cell insulin stores are replenished to prevent exhaustion during secretion by promoting insulin gene transcription, mRNA stability and biosynthesis. In addition to enhancing insulin secretion, GLP-1 has been shown to inhibit glucagon secretion at glucose levels above fasting levels.

In the brain, GLP-1 receptor activation has been linked with neurotrophic effects including neurogenesis and neuroprotective effects including reduced necrotic and apoptotic signaling, cell death, and dysfunctions. In the diseased brain, GLP-1 receptor agonist treatment is associated with protection against a range of experimental disease models such as Parkinson's disease, Alzheimer's disease, stroke, traumatic brain injury, and multiple sclerosis.

### Glucagon-Like Peptide-1 Receptor Agonists (GLP-1R Agonists)

To date, multiple GLP-1 receptor (GLP-1R) agonists have been developed by a combination of selective amino acid substitutions, enzymatic cleavage blockade, and conjugation to entities that increase binding to plasma proteins. These methods not only slow down fast renal clearance of the peptides but also extend their half-lives. Any such agonists or variants thereof may be one or independently more than one component of a multimeric polypeptide disclosed here.

Examples of GLP-1R agonists include, but are not limited to, dulaglutide, exenatide, liraglutide, fixisenatide, and semaglutide. Dulaglutide is a GLP-1(7-37) polypeptide covalently linked to an Fc fragment of human IgG4, approved by FDA in 2014. Exenatide is a 39-amino-acid synthetic version of exendin-4, approved for use in U.S. in 2005. Liraglutide is an acylated GLP-1R agonist derived from human GLP-1(7-37), approved for use in U.S. in 2010. Lixisenatide is a 44-amino-acid polypeptide derived from exendin-4, approved by FDA in 2016. Lixisenatide has been described as "des-38-proline-exendin-4 (Heloderma suspectum)-(1-39)-peptidylpenta-L-lysyl-L-lysinamide", meaning it is derived from the first 39 amino acids in the sequence of the peptide exendin-4, omitting proline at position 38 and adding six lysine residues. Semaglutide, first approved for use in U.S. in 2017, is a human GLP-1 with amino acid substitutions at positions 8 and 34, where alanine and lysine are replaced by 2-aminoisobutyric acid and arginine, respectively. Amino-acid substitution at position 8 prevents chemical breakdown by dipeptidyl peptidase-4. In addition, the lysine at position 26 is in its derivative form (acylated with stearic diacid). Acylation with a spacer and C-18 fatty diacid chain increases the drug's binding to blood protein (albumin), resulting in longer presence in the blood circulation. Any one or more of such agonists, or a variant thereof, may be a component of a multimeric polypeptide disclosed herein.

These GLP-1R agonists mimic the action of GLP-1. When blood sugar levels start to rise after someone eats, these drugs stimulate the body to produce more insulin. The extra insulin helps lower blood sugar levels. These type 2 diabetes drugs not only improve blood sugar control but may also lead to weight loss.

### Small Molecule GLP-1R Agonists

There are challenges in the development of small molecule GLP-1R agonists owing to the complexity of ligand recognition and signal induction mechanisms. In 2007, a series of small molecules known as ago-allosteric modulators selective for the human GLP-1 receptor was discovered (Knudsen et al., Small-Molecule Agonists For The Glucagon-Like Peptide 1 Receptor. PNAS U.S.A., 104:937-942 (2007)). These compounds act as both allosteric activators of the receptor and independent agonists. Potency of GLP-1 was not changed by the allosteric agonists, but affinity of GLP-1 for the receptor was increased. The most potent compound identified stimulates glucose-dependent insulin release from normal mouse islets. In another study, a novel small molecule GLP-1R agonist, S6, was identified by virtual screening and fluorescent imaging plate reader (FLIPR)-based calcium assays. (Yang et al., Novel Small Molecule Glucagon-Like Peptide-1 Receptor Agonist S6 Stimulates Insulin Secretion From Rat Islets. Front Pharmacol., 12:664802 (2021)). It was found that S6 could bind potently with GLP-1R, and potentiated insulin secretion in a glucose-dependent manner. The results indicate that S6 stimulates glucose-dependent insulin secretion mainly by acting on GLP-1R, inhibiting voltage-dependent K⁺ channels, and increasing intracellular calcium concentration.

Thus, in some embodiments, the GLP-1R agonist is a peptide having the sequence
H**X**EGT FTSDV SSYLE GQAA**K** EFIAW LVKGR G;
wherein **X** refers to 2-aminoisobutyric acid or alanine, and **K** refers to an optionally lipidated lysine residue.

In some embodiments, the GLP-1R agonist is a peptide having the sequence
**Ac**-H**X**EGT FTSDV SSYLE GQAA**K** EFIAW LVKGR G;
Wherein **Ac** refers to an acetyl group, **X** refers to 2-aminoisobutyric acid or alanine, and **K** refers to an optionally lipidated lysine residue.

In some embodiments, the GLP-1R agonist is a peptide having the sequence
HAEGTFTSDVSSYLEGQAAKEFIAWLVKGR.

In some embodiments, the GLP-1R agonist is a peptide having the sequence
Ac-HAEGTFTSDVSSYLEGQAAKEFIAWLVKGR.

In some embodiments, the foregoing peptides may have a C-terminal amide.

In any of the foregoing embodiments, the polypeptide sequence in the multimeric polypeptide has a greater than about 70% sequence homology to the respective native GPCR ligand. In some embodiments, the sequence homology is greater than about 75%, 80%, 85%, 90%, 95% or 99%.

Other examples of GLP-1R agonists include, but are not limited to, dulaglutide, exenatide, liraglutide, lixisenatide, and semaglutide, analogues or variants thereof.

Dulaglutide is a GLP-1(7-37) polypeptide covalently linked to an Fc fragment of human IgG4, approved by FDA in 2014. The sequence is HGEGTFTSDV SSYLEEQAAK EFIAWLVKGG GGGGGSGGGG SGGGGSAESK YGPPCPPCPA.

Exenatide is a 39-amino-acid synthetic version of exendin-4, approved for use in U.S. in 2005. The sequence is HGEGTFTSDLSKQMEEEAVRLFIEWLKNGGPSSGAPPPS-amide.

Liraglutide is an acylated GLP-1R agonist derived from human GLP-1(7-37), approved for use in U.S. in 2010. The sequence is H-His-Ala-Glu-Gly-Thr-Phe-Thr-Ser-Asp-Val-Ser-Ser-Tyr-Leu-Glu-Gly-Gln-Ala-Ala-Lys(palmitoyl-Glu(1)-OH)-Glu-Phe-Ile-Ala-Trp-Leu-Val-Arg-Gly-Arg-Gly-OH. The epsilon amino group of the Lys at position 20 is bound to a palmitoyl-Glu-OH moiety.

Lixisenatide is a 44-amino-acid polypeptide derived from exendin-4, and has been described as "des-38-proline-exendin-4 (Heloderma suspectum)-(1-39)-peptidylpenta-L-lysyl-L-lysinamide", meaning it is derived from the first 39 amino acids in the sequence of the peptide exendin-4, omitting proline at position 38 and adding six lysine residues. The sequence is HGEGTFTSDLSKQMEEEAVRLFIEWLKNGGPSSGAPPSKKKKKK-NH2 .

Semaglutide, first approved for use in U.S. in 2017, is a human GLP-1 with amino acid substitutions at positions 8 and 34, where alanine and lysine are replaced by 2-aminoisobutyric acid and arginine, respectively. In addition, the lysine at position 26 is in its derivative form (acylated with stearic diacid). The sequence is H-His-Aib-Glu-Gly-Thr-Phe-Thr-Ser-Asp-Val-Ser-Ser-Tyr-Leu-Glu-Gly-Gln-Ala-Ala-Lys(AEEAc-AEEAc-γ-Glu-carboxyheptadecanoyl)-Glu-Phe-Ile-Ala-Trp-Leu-Val-Arg-Gly-Arg-Gly-OH .

The present disclosure encompasses modifications and variants of the foregoing peptides, as described elsewhere herein. Non-limiting examples of modifications include wherein the polypeptide comprises at least one modified amino acid, wherein the modification is any of acetylation, amidation, C-linked glycosylation, citrullination, crotonylation, formylation, glutarylation, glutathionylation, hydroxylation, lipidation, malonylation, methylation, myristoylation, N-linked glycosylation, O-linked glycosylation, oxidation, palmitoylation, phosphorylation, pyrrolidone carboxylic acid, S-nitrosylation, sulfation, or sumoylation. In some embodiments, the polypeptide units is N-terminus acetylated. In some embodiments, the polypeptide comprises one or more non-canonical amino acid, such as 2-aminoisobutyric acid. In another embodiment, any of the polypeptide units comprises one or more lipidated lysine residues.

### GASTRIC INHIBITORY POLYPEPTIDE/GLUCOSE-DEPENDENT INSULINOTROPIC POLYPEPTIDE RECEPTORS (GIPRs)

Multimeric polypeptides disclosed herein may comprise one or more ligands of GIPR.

Gastric inhibitory polypeptide receptor or glucose-dependent insulinotropic polypeptide receptor (GIPR) has several isoforms and is a G-protein coupled receptor for gastric inhibitory polypeptide (GIP), which was originally identified as a hormone that acts in gut extracts and inhibits the releasing of gastrin and subsequently gastric acid.

The GIPR is expressed at the pancreatic beta cells and other overlapping tissues with the GLP-1R (the nervous system, the gastrointestinal tract and the cardiovascular system), but is also distinctly expressed in adipocytes and bone cells. As for the interaction of GLP-1 with its receptor, GIP also use its C-terminal alpha-helix (position 6-30) to interact the N-terminal extracellular domain of the GIPR. Such interaction leads to engagement of the N-terminus of GIP with the receptor core. The GIPR then undergoes conformational changes and activation of downstream intracellular signaling.

To stimulate insulin secretion after nutrient ingestion, GIP binds to and activates the GIPR on the beta cell surface. Activation of GIPR promotes recruitment and activation of the Gαs protein, which in turn activates the adenylate cyclase to stimulate cAMP production. Again, the increased cAMP activates PKA and EPAC. PKA and cAMP activate a series of proteins including the mitogen-activated protein kinase (MAPK) cascades, and phosphorylates ERK1/2, which regulates genes involved in proliferative and anti-apoptotic processes. The GIPR activation of PKA leads to insulin secretion by the same mechanisms as described for the GLP-1R. GIPR activation can also promote non-insulinotropic actions, such as controlling pancreatic beta cell proliferation and survival. Furthermore, PKA inhibits AMPK, which results in dephosphorylation and nuclear import of TORC2. CREB and TORC2 form a complex promoting the transcription of the anti-apoptotic gene *bcl*2. Activation of Akt/PKB/PI3K promotes phosphorylation of the nuclear transcription factor Foxo1, which in turn inactivates *bax* and other apoptosis-related factors, resulting in downregulation of the pro-apoptotic signaling pathway. Additionally, activation of Akt/PKB will result in suppressing the anti-proliferative and anti-survival mechanism of p38 MAPK and c-Jun N-terminal kinase (JNK), leading to suppressed mitochondrial translocation of BAD and BimEL and thereby the subsequent activation of caspase-3 promoting proliferation. An important difference between GIPR and GLP-1R is that the GLP-1R activates EGFR leading to proliferation and anti-apoptosis, which is not the case for GIPR.

When GIP is bound to its receptor GIPR, GIPR undergoes conformational changes that also initiate the phosphorylation of helix 7 by G protein-coupled receptor kinases (GRK). This leads to recruitment of beta-arrestin 1 and 2. The beta-arrestins play a key role in receptor desensitization by blocking Gαs proteins and in receptor trafficking by internalization and recycling. In contrast to the GLP-1R, where the internalization is both beta arrestin-dependent and independent (e.g. Gαq mediated pathway), the arrestins are necessary for GIPR internalization. The coated pits that contain GIPRs are separated from the plasma membrane by the membrane-remodeling GTPase dynamin leading to the construction of early endosomes, in which the GIPR is suggested to trigger production of cAMP and PKA activation before recycling back to the beta cell surface.

### Ligands of GIPR

Endogenous GIPR ligands include GIP(1-42), GIP(1-30)NH2, GIP(3-42), and GIP(3-30)NH2. GIP, gastric inhibitory polypeptide, is also called glucose-dependent insulinotropic polypeptide. GIP was initially discovered and named for its ability to inhibit gastric acid secretion. Later it was found that at physiological levels, GIP stimulates insulin synthesis and release in rodents and humans. The function of GIP as an insulinotropic agent stimulating insulin synthesis and release depends on an increase in blood glucose levels, resulting in the name change to glucose-dependent insulinotropic polypeptide to reflect these findings. GIP appears to compensate for lost GLP-1-regulated insulin release in studies where the GLP-1 receptor gene has been disrupted. GIP may have a more restricted role in glucose homeostasis than GLP-1, as GIP has been shown to regulate glucose absorption and glycemic excursions only following enteral glucose challenge.

GIP is a 42-amino acid peptide secreted by enteroendocrine K cells located in the duodenum and proximal jejunum. It is a member of the incretin family mediating increases of insulin secretion prior to the postabsorptive nutrient-induced increase in blood glucose. GIP levels rise immediately after nutrient ingestion, leading to modest inhibitory effects on gastric acid secretion and gastrointestinal motility. GIP is structurally related to the secretin family of gastrointestinal regulatory polypeptides, which includes secretin, glucagon, glucagon-like peptides (GLP-1 and GLP-2), vasoactive intestinal peptide (VIP), peptide histidine isoleucine, growth hormone-releasing factor, and pituitary adenylate cyclase-activating polypeptide. GIP is derived from post-translational processing of pre-pro-GIP, a protein consisting of 153 amino acids. GIP is inactivated by the enzyme dipeptidyl-peptidase IV (DPP-4). Such peptides or variants thereof may be a component or independently components of a multimeric polypeptide disclosed herein.

Effects of GIP on lipid metabolism include increasing plasma triglyceride clearance following meals, increasing lipoprotein lipase activity, and promoting fat storage by adipocytes. Interestingly, interrupting GIP signaling appears to be beneficial in resisting high-fat diet-induced obesity and insulin resistance in GIPR^{-/-} mice. Although fat ingestion is a potent stimulator of GIP secretion in humans and GIP plasma levels are increased in some obese individuals, it is unknown whether increased GIP signaling causally contributes to human obesity.

Given the broad tissue distribution of GIPR, it is not surprising that GIP actions have been reported on several tissues other than stomach, islets, and adipocytes. In the CNS, GIP may play a role in neural progenitor cell proliferation and behavior modification. Functional GIPR is present on bone cells, and GIP increases bone mineral density in ovariectomized rats. However, acute administration of GIP does not alter markers of bone turnover in humans, although it is unknown whether more long-term GIP treatment affects bone turnover. GIPR is present in the vascular endothelium and GIP increases intracellular Ca²⁺ levels in endothelial cell cultures. Although GIPR mRNA is also detected in the heart, testis, lung, and several other tissues in rodents, the physiological actions of GIP in these tissues are still elusive.

### GIPR Agonists

Currently, the only drugs marketed that affects the GIPR signaling are the dipeptidylpeptidase-4 (DPP-4) inhibitors (e.g. linagliptin, saxagliptin, and sitagliptin) which inhibit the degradation of DPP-4 substrates such as GIP and GLP-1, and thereby indirectly result in increased GIPR activation. Any such peptides or variants thereof may be a component or independently components of a multimeric polypeptide disclosed herein.

Long-acting GIPR monoagonists have been found to reduce body weight in obese mice (Mroz et al., Optimized GIP Analogs Promote Body Weight Lowering In Mice Through GIPR Agonism Not Antagonism. Mol. Metab. 20:51-62 (2019)). Non-acylated, 2-aminoisobutyric acid substituted analogs derived from human GIP sequence showed full *in vitro* potency at human GIPR without cross-reactivity at GLP-1R. These GIPR agonists lowered acute blood glucose in wild-type and *Glp1r-*/*-* mice, and this effect was absent in *Gipr-*/*-* mice, which confirmed selectivity towards GIPR.

Thus, in some embodiments, the GIP agonist is a peptide having the sequence
YXEGT FISDY SIAMD KIHQQ DFVNW LLAQK GKKND WKHNI TQ ;wherein X refers to 2-aminoisobutyric acid or alanine, and K refers to an optionally lipidated lysine residue.

In some embodiments, the GIP agonist is a peptide having the sequence
Ac-YXEGT FISDY SIAMD KIHQQ DFVNW LLAQK GKKND WKHNI TQ ; wherein Ac refers to an N-terminal acetyl, X refers to 2-aminoisobutyric acid or alanine, and K refers to an optionally lipidated lysine residue.

In some embodiments, the GIP agonist peptide is GIP(1-42) (YAEGTFISDYSIAMDKIHQQDFVNWLLAQKGKKNDWKHNITQ), GIP(1-30)NH2 (YAEGTFISDYSIAMDKIHQQDFVNWLLAQK-NH2), GIP(3-42) (EGTFISDYSIAMDKIHQQDFVNWLLAQKGKKNDWKHNITQ), and GIP(3-30)NH2 (EGTFISDYSIAMDKIHQQDFVNWLLAQK-NH2).

In some embodiments, the GIP agonist peptide is YAEGTFISDYSIAMDKIHQQDFVNWLLAWKGKKNDWKHNITQ, or Ac-YAEGTFISDYSIAMDKIHQQDFVNWLLAWKGKKNDWKHNITQ.

In some embodiments, the foregoing peptides may have a C-terminal amide.

In any of the foregoing embodiments, the polypeptide sequence in the multimeric polypeptide has a greater than about 70% sequence homology to the respective native GPCR ligand. In some embodiments, the sequence homology is greater than about 75%, 80%, 85%, 90%, 95% or 99%.

The present disclosure encompasses modifications and variants of the foregoing peptides, as described elsewhere herein. Non-limiting examples of modifications include wherein the polypeptide comprises at least one modified amino acid, wherein the modification is any of acetylation, amidation, C-linked glycosylation, citrullination, crotonylation, formylation, glutarylation, glutathionylation, hydroxylation, lipidation, malonylation, methylation, myristoylation, N-linked glycosylation, O-linked glycosylation, oxidation, palmitoylation, phosphorylation, pyrrolidone carboxylic acid, S-nitrosylation, sulfation, or sumoylation. In some embodiments, the polypeptide units is N-terminus acetylated. In some embodiments, the polypeptide comprises one or more non-canonical amino acid, such as 2-aminoisobutyric acid. In another embodiment, any of the polypeptide units comprises one or more lipidated lysine residues.

### CALCITONIN RECEPTOR-LIKE RECEPTOR (CLR or CRLR)

Multimeric polypeptides disclosed herein may comprise one or more ligands of CLR or CRLR.

The calcitonin (CT) family of peptides consists of six members: CT, amylin, adrenomedullin (AM), two distinct forms of calcitonin gene-related peptide (αCGRP and βCGRP), and AM2, also known as intermedin. These peptides range from 32 to 52 amino acids in length and are responsible for a diverse array of functions. Consequently, their receptors have therapeutic relevance for many conditions, which include osteoporosis, diabetes, obesity, lymphatic insufficiency, migraine and cardiovascular disease.

The two GPCRs that are receptors for these peptides are the calcitonin receptor (CTR) and the calcitonin receptor-like receptor (CLR, historically known as CRLR). These belong to the subfamily of GPCRs known as the secretin or 'B' family of GPCRs. CTR and CLR can form complexes with members of the membrane protein family called the receptor activity-modifying proteins (RAMPs), which consists of RAMP1, 2 and 3 in humans. RAMP association with the CTR or with CLR generates multiple distinct receptor phenotypes with different specificities for the CT peptide family. CLR together with RAMP1 forms the CGRP receptor. In contrast, two AM receptors are formed by CLR and RAMP2 or RAMP3 respectively. CTR forms amylin receptors with RAMPs, the AMY receptor.

Although CTR and CLR associate with RAMPs, they share common characteristics with other family B GPCRs. They possess a large N-terminal extracellular domain (ECD), three extracellular loops (ECL1, ECL2, ECL3), seven transmembrane (TM) domains, three intracellular loops (ICL1, ICL2, and ICL3) and an intracellular C-terminus.

Both CTR and CLR oligomerize with RAMPs and this is essential for the formation of CGRP, AM and AMY receptors. The role of RAMPs has been extensively studied and the contacts between the extracellular domains of CLR and RAMP1 have been reported. In the crystal structure of the CGRP receptor, CLR and RAMP1 form 1:1 heterodimers, consistent with an earlier crosslinking study of full-length receptors. However, a bimolecular fluorescence complementation with BRET approach suggested the CGRP receptor contained two CLRs with only one RAMP1. This does not rule out the possibility that two individual RAMP molecules could bind to a CLR dimer. Although the stoichiometry of CGRP receptor is still unclear, the possibility that it contains a CLR dimer is thought provoking and is consistent with the paradigm that family B GPCRs can form dimers. There also is evidence for intra-family hetero-oligomerization in family B GPCRs. Determining the pharmacological profile of the various combinations of monomeric units along with their physiological relevance could aid drug design strategies immensely.

### Ligands of CRLR

### Calcitonin Gene-Related Peptide

Calcitonin gene-related peptide (CGRP) is primarily localized to C and Aδ sensory fibers. These fibers display a wide innervation throughout the body, with extensive perivascular localization, and have a dual role in sensory (nociceptive) and efferent (effector) function. CGRP is also localized in non-neuronal tissues, of which less is known at present.

The mature form of CGRP is a 37-amino acid peptide with an N-terminal disulfide bond and amidated C terminus. Both regions are required for receptor activation. The peptide has an amphiphilic α-helical structure between residues 8-18, based on nuclear magnetic resonance spectra. This structure is conserved across the six recognized members of the CGRP gene family: calcitonin, α- and β-CGRP, amylin, adrenomedullin, and adrenomedullin 2 (also known as intermedin. The α- and β-isoforms of CGRP are expressed from two genes: *CALCA* encodes α-CGRP, which is the predominant form expressed in trigeminal ganglia; and *CALCB,* which encodes β-CGRP and differs from α-CGRP by only 1-3 amino acids in different species. The two peptides have nearly indistinguishable activities, yet they are differentially regulated and expressed in a distinct but overlapping pattern. Such peptides or variants thereof may be a component or independently components of a multimeric polypeptide disclosed herein.

The CGRP receptor is composed of three subunits: calcitonin-like receptor (CLR), receptor activity-modifying protein 1 (RAMP1), and receptor component protein (RCP). The seven-transmembrane CLR protein requires RAMP1 for both its trafficking to the plasma membrane and its binding to CGRP, and RCP facilitates coupling of Gαs. RAMP1 appears to be the rate-limiting subunit of the receptor. The CGRP receptor generally activates a cyclic adenosine monophosphate (cAMP)-signaling pathway (although other pathways can be recruited) to modulate gene expression and regulate receptor and ion channel activity. Some structural requirements for CGRP binding to its receptor have been identified. CGRP C-terminal residues are likely to bind a pocket formed by the N-terminal extracellular domain of CLR and RAMP1, followed by binding of N-terminal CGRP residues to the juxtamembrane domain to allow receptor activation.

Originally, CGRP was shown to mediate sympathetic outflow from the brain. However, it was soon established that the major cardiovascular activity of CGRP is its potent vasodilator activity that is obvious when exogenous CGRP is administered at femtomolar doses to the skin of human and animal species. It has been suggested that CGRP may have potential as a therapy for treating cardiovascular diseases, but progress has been limited. The sensory fibers that CGRP is contained in are also associated with pain processes, and the development of CGRP antagonists has revealed the pivotal role that CGRP plays in migraine, and with it the therapeutic potential of CGRP receptor antagonists, which has led to a vibrant drug discovery program.

### Adrenomedullin

Adrenomedullin (AM or ADM) is an endogenous vasodilatory peptide that has many diverse effects and functions, including organ protection, anti-inflammatory effects, and tissue repair. AM and AM receptors are ubiquitously present in various tissues and are highly expressed in blood vessels. Furthermore, constitutive expression of AM and AM receptors has been confirmed in the heart, kidneys, brain, lungs, and adrenal glands of humans and various animals.

Adrenomedullin (AM) is composed of 52 amino acids, has a ring structure containing a disulfide bond between Cys¹⁶ and Cys²¹ and is amidated at the C-terminal Tyr⁵². Both disulfide bonds and amidation are crucial for bioactivity and are highly conserved in calcitonin (CT) and the two forms of calcitonin gene-related peptide (CGRP), amylin and adrenomedullin2/intermedin. Therefore, AM is considered a member of the CT/CGRP superfamily. AM is synthesized as a large preprohormone and processed to proadrenomedullin (proAM), including amino acid residues 22-185, which is then fragmented into four segments, namely, proAM N-terminal 20 peptide (PAMP)-Gly, mid-regional pro-adrenomedullin (MR-proADM, 45-92), AM-Gly, and C-terminal proAM (adrenotensin). Both PAMP and AM are initially processed as intermediate forms (C-terminally glycine-extended forms), which are biologically inactive. The C-terminal glycine of these peptides is subsequently amidated by an amidation enzyme, and the peptides are then converted to their mature bioactive forms. MR-proADM is a nonbioactive fragment that is stable in the bloodstream and is therefore useful as a biomarker of AM synthesis. Such peptides or variants thereof may be a component or independently components of a multimeric polypeptide disclosed herein.

The receptors for adrenomedullin (AM) consist of two different types of molecules, CRLR and chaperone molecules called receptor activity-modifying proteins (RAMPs). CRLR + RAMP1 serves as the CGRP receptor, CRLR + RAMP2 serves as the AM1 receptor, and CRLR + RAMP3 serves as the AM2 receptor. AM has equal affinity for both AM1 and AM2 receptors. Models have shown that the knockout (KO) of AM is lethal at mid-gestation due to serious vascular abnormalities and causes extreme hydrops. CRLR KO in mice is also lethal, but interestingly, CGRP KO mice appear to be normal, and only the KO of RAMP2 causes lethal abnormalities, such as those observed upon the KO of AM. These findings suggest that AM and AM1 receptors are crucial for angiogenesis and vascular homeostasis, while the AM2 receptor has been shown to be important for lymph vessel function.

### Amylin

In some embodiments, a polypeptide is amylin or has greater than about 70% sequence homology to amylin (Ac-KCNTA TCATQ RLAEF LRHSS NNFGP ILPPT NVGSN TP; ). In some embodiments, the sequence homology is greater than about 75%, 80%, 85%, 90%, 95% or 99%. In some embodiments, a polypeptide is an amylin variant having greater than about 70% sequence homology to amylin (Ac-KCNTA TCATQ RLAEF LRHSS NNFGP ILPPT NVGSN TP; ). In some embodiments, the sequence homology of the amylin variant is greater than about 75%, 80%, 85%, 90%, 95% or 99%.

The present disclosure encompasses modifications and variants of the foregoing peptides, as described elsewhere herein. Non-limiting examples of modifications include wherein the polypeptide comprises at least one modified amino acid, wherein the modification is any of acetylation, amidation, C-linked glycosylation, citrullination, crotonylation, formylation, glutarylation, glutathionylation, hydroxylation, lipidation, malonylation, methylation, myristoylation, N-linked glycosylation, O-linked glycosylation, oxidation, palmitoylation, phosphorylation, pyrrolidone carboxylic acid, S-nitrosylation, sulfation, or sumoylation. In some embodiments, the polypeptide units is N-terminus acetylated. In some embodiments, the polypeptide comprises one or more non-canonical amino acid, such as 2-aminoisobutyric acid. In another embodiment, any of the polypeptide units comprises one or more lipidated lysine residues.

### CRLR Agonists

An analogue of the α-form of the CGRP (Serinyl-α-CGRP (2-37)-amide) with an albumin binding fatty acid moiety in the N-terminus (αAnalogue) was reported to have prolonged half-life (10.2±0.9 h) in rodents and induced elevated energy expenditure and reduced food intake after single dosing in normal rats (Nilsson et al., Long Acting Analogue of the Calcitonin Gene-Related Peptide Induces Positive Metabolic Effects And Secretion of the Glucagon-Like Peptide-1. Eur J Pharmacol. 773:24-31 (2016)). αAnalogue increased levels of circulating Glucagon-Like Peptide-1 (GLP-1) by >60%. Treatment of the type 2 diabetic ob/ob mice with the αAnalogue caused reduction in fasting insulin levels and a tendency to reduce fasting blood glucose, demonstrating a potential anti-diabetic effect. *Id.* These results demonstrate that long-acting CGRP analogues may have a therapeutic potential for the treatment of type 2 diabetes through positive metabolic effects and effect on GLP-1 secretion. Such peptides or variants thereof may be a component or independently components of a multimeric polypeptide disclosed herein.

A long-acting human adrenomedullin (AM) derivative (PEG-AM), which had an effect similar to that of native AM, has been prepared by conjugating with a 60 kDa polyethylene glycol (Nagata et al., Polyethylene Glycol-Conjugated Human Adrenomedullin As A Possible Treatment For Vascular Dementia. Peptides.121:170133 (2019)). The 60 kDa PEGylated AM has shown therapeutic usefulness in a rodent model of colitis, similar to that of native AM. Additionally, the 60 kDa PEGylated AM was effective in a rat model of vascular dementia. Other derivatives have also been developed, such as an IgG Fc region fused-AM and human albumin-modified AM.

In the case of IgG Fc-AM fusion protein, two kinds of human IgG1 Fc fusion proteins containing either full-length human AM (IgG1-AM) or human AM residues 6-52 (IgG1-AM (6-52)) have been constructed (Nagata et al., Developments of Human Adrenomedullin-IgG1 Fc Fusion Proteins. J. Biochem. 166:157-162 (2019)). Sufficient concentrations of both AM-Fc fusions were observed in blood 2 days after a single subcutaneous administration. IgG1-AM and IgG1-AM (6-52) stimulated cAMP production in human embryonic kidney-293 cells stably expressing the AM1 receptor. The activity of IgG1-AM (6-52) was higher than that of IgG1-AM. Treatment with IgG1-AM (6-52) inhibited blood pressure increase in spontaneously hypertensive rats. In addition, IgG1-AM (6-52) reduced total inflammation scores in the dextran sulfate sodium colitis model.

Regarding human albumin-modified AM, a human serum albumin-adrenomedullin (HSA-AM) conjugate was synthesized by covalent attachment of a maleimide derivative of adrenomedullin to the 34th cysteine residue of HSA via a linker (Kuroishi et al., Development of a Novel Human Adrenomedullin Derivative: Human Serum Albumin-Conjugated Adrenomedullin. J. Biochem. 170:445-451 (2021)). Both adrenomedullin and HSA-AM stimulated the intracellular accumulation of cyclic AMP (cAMP) in HEK-293 cells stably expressing the adrenomedullin 1 receptor. The bioavailability of HSA-AM compared with that of adrenomedullin was much improved after subcutaneous administration in the rat, which was probably due to the superior resistance of HSA-AM towards endogenous proteases and its reduced clearance from the blood.

### Small Molecule CRLR Agonists

Small-molecule positive modulators for the CLR:RAMP complexes have been reported recently (Hendrikse et al., Identification of Small-Molecule Positive Modulators of Calcitonin-like Receptor-Based Receptors. ACS Pharmacol. Transl. Sci. 3:305-320 (2020)). As described above, calcitonin receptor-like receptor (CRLR) is linked to conditions such as migraine, cardiovascular disease, and inflammatory bowel disease. The CRLR cannot act as a cell-surface receptor alone but rather must couple to one of three receptor activity-modifying proteins (RAMPs), forming heterodimeric receptors for the peptides adrenomedullin and calcitonin gene-related peptide. These peptides have extended binding sites across their receptors. Small molecules that are able to positively modulate the signaling of the CRLR with all three RAMPs but are not active at the related calcitonin receptor were selected from a β-arrestin recruitment screen, coupled with rounds of medicinal chemistry to improve their activity. Translational potential is shown as the compounds can positively modulate cAMP signaling in a vascular cell line model. Molecular modeling reveals potential allosteric binding sites in multiple receptor regions.

In some embodiments, the CRLR agonist peptide is
VTHRLAGLLSRSGGVVKNNFVPTNVGSKAF-NH₂

In some embodiments, the amylin variant has at least about 70% sequence homology to
Ac-KCNTA TCATQ RLAEF LRHSS NNFGP ILPPT NVGSN TP
wherein Ac refers to an N-terminal acetyl, and K refers to an optionally lipidated lysine residue.

In some embodiments, the foregoing peptides may have a C-terminal amide.

In any of the foregoing embodiments, the polypeptide sequence in the multimeric polypeptide has a greater than about 70% sequence homology to the respective native GPCR ligand. In some embodiments, the sequence homology is greater than about 75%, 80%, 85%, 90%, 95% or 99%.

The present disclosure encompasses modifications and variants of the foregoing peptides, as described elsewhere herein. Non-limiting examples of modifications include wherein the polypeptide comprises at least one modified amino acid, wherein the modification is any of acetylation, amidation, C-linked glycosylation, citrullination, crotonylation, formylation, glutarylation, glutathionylation, hydroxylation, lipidation, malonylation, methylation, myristoylation, N-linked glycosylation, O-linked glycosylation, oxidation, palmitoylation, phosphorylation, pyrrolidone carboxylic acid, S-nitrosylation, sulfation, or sumoylation. In some embodiments, the polypeptide units is N-terminus acetylated. In some embodiments, the polypeptide comprises one or more non-canonical amino acid, such as 2-aminoisobutyric acid. In another embodiment, any of the polypeptide units comprises one or more lipidated lysine residues.

### Selection of Two or More Polypeptides for a Multimeric Polypeptide

A multimeric polypeptide disclosed herein may comprise one or more GPCR peptide agonists, such as but not limited to GLP-1R agonist, GIPR agonist, CRL agonist or amylin , and variants or analogues thereof. As will be described below, any multimeric polypeptide embodied herein may have one or more non-peptidic components bound thereto, such as but not limited to a SGLT2 antagonist, a lipid, a protein such as albumin, or an immunoglobulin.

In one embodiment, the multimeric polypeptide comprises a GLP-1R agonist and a GIPR agonist.

In one embodiment, the multimeric polypeptide comprises a GLP-1R agonist and a CRL agonist.

In one embodiment, the multimeric polypeptide comprises a GIPR agonist and a CRL agonist.

In one embodiment, the multimeric polypeptide comprises two GLP-1R agonists. The structures of the two GLP-1R agonists may be the same or different.

In one embodiment, the multimeric polypeptide comprises two CRL agonist. The structures of the two CRL agonists may be the same or different.

In one embodiment, the multimeric polypeptide comprises two GIPR agonists. The structures of the two GIPR agonists may be the same or different.

In one embodiment, the multimeric polypeptide comprises two GLP-1R agonists and a GIPR agonist.

In one embodiment, the multimeric polypeptide comprises two GLP-1R agonists and a CRL agonist. The structures of the two GLP-1R agonists may be the same or different.

In one embodiment, the multimeric polypeptide comprises a GLP-1R agonist and two GIPR agonists. The structures of the two GIPR agonists may be the same or different.

In one embodiment, the multimeric polypeptide comprises a GLP-1R agonist and two CRL agonists. The structures of the two CLR agonists may be the same or different.

### NON-PEPTIDIC COMPONENTS

As noted herein, a multimeric polypeptide disclosed herein may further comprise a non-peptidic component covalently bound thereto, such as but not limited to a SGLT2 inhibitor, a lipid, a protein such as albumin, or an immunoglobulin. In one embodiment, the protein extends the half-life of the multimeric polypeptide in vivo.

In some embodiments, the non-peptidic components is covalently linked to the linker as described herein, or to an amino acid on one or more of the polypeptides. In some embodiments, the non-peptidic molecule is linked by an amido-PEG-acid or maleimide-PEG-acid.

### SGLT2 Inhibitors

In some embodiments, a multimeric polypeptide disclosed herein may further comprise a SGLT2 inhibitor bound thereto. The SGLT2 inhibitor may be bound to the linker, or bound to any amino acid in a polypeptide.

In one embodiment, the SGLT-2 inhibitor has a structure of Formula XIII 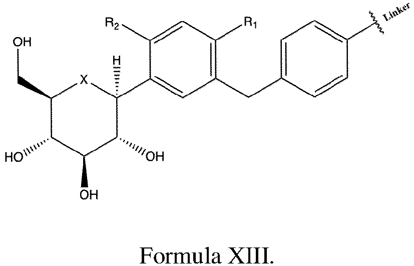 wherein
"Linker" refers to a linker covalently linking to the polypeptide unit or linker
R₁ = -H, -OH, or a halogen;
R₂ = -H, -OH, or a halogen; and
X = S or O.

In some embodiments, the SGLT2 inhibitor is canagliflozin, dapagliflozin, ipragliflozin, tofogliflozin, ertugliflozin, luseogliflozin or empagliflozin.

Non-limiting examples of SGLT2 inhibitors covalent bound to multivalent polypeptides disclosed herein include Compound B described herein.

### Lipids

In some embodiments, at least one non-peptidic molecule is a lipid.

### Proteins such as Albumin and Immunoglobulin.

Covalent attachment of a lipid to a polypeptide or linker of a multimeric polypeptide provides benefits such as increased circulating or biologic half-life, resistance to proteolysis and/or degradation, and enhanced biological activity. Albumin such as human serum albumin, or an immunoglobulin such as human IgG, may be bound to the linker, or bound to any amino acid in a polypeptide.

### LINKERS

As disclosed herein, a linker is provided to covalently attach the two or more GCPR ligands and the optional SGLT2 inhibitor, lipid and/or protein. In one embodiment, the linker comprises a linear or branched structure comprising a plurality of reactive sites that covalently link the at least two polypeptides. Such reactive sites may be, by non-limiting example, independently amine, thiol, or halogen reactive sites. As used herein, independently, when referring to multiple selections (e.g., polypeptides, reactive sites) means that each selection may be different or the same. In some embodiments, the plurality of reactive sites of the linker moiety independently comprise one or more azides or alkynes. In some embodiments, the plurality of reactive sites of the linker moiety comprise one or more orthogonal protecting groups that may be removed independently of other protecting groups.

In some embodiments, the linker comprises a polymer linker.

In some embodiments, the linker comprises an alpha amino acid, a beta-amino acid or a gamma-amino acid.

In some embodiments, the linker moiety comprises a D amino acid.

In some embodiments, the linker moiety comprises an ethylene glycol oligomer.

In some embodiments, the linker comprises a backbone that connects the two or more multimeric polypeptides together, optionally with a further SGLT2 inhibitor, lipid or protein, and wherein the moieties on the linker that link to each polypeptide may also comprise a linker. Thus, the polypeptides may be bound to the backbone by linkers, and the backbone itself may comprise linkers. Thus, polymeric, polyamino acid, or other typical linking components may be present both between each polypeptide (and optional SGLT2 inhibitor, lipid and/or protein) and the backbone, and between parts of the backbone.

In some embodiments, linker moiety comprises one or more independent PEG4, PEG8, PEG20, 2x(GGGGS), 4x(GGGGS), 8xG, GSAGSAAGSGEF, A-(EAAAK)-A, A-3x(EAAAK)-A, 7x(AP), KESGSVSSEQLAQFRSLD or EGKSSGSGSESKST.

| **Linker component abbreviation** | **Structure** |
|---|---|
| PEG4 | 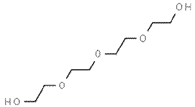 or HO-(CH₂O)₃CH₂CH₂OH |
| PEG8 | HO-(CH₂O)₇CH₂CH₂OH |
| PEG20 | HO-(CH₂O)₁₉CH₂CH₂OH |
| 2x(GGGGS) | GGGGSGGGGS |
| 4x(GGGGS), | GGGGSGGGGSGGGGSGGGGS |
| 8xG | GGGGGGGG |
| GSAGSAAGSGEF | GSAGSAAGSGEF |
| A-(EAAAK)-A | AEAAAKA |
| A-3x(EAAAK)-A | AEAAAKEAAAKEAAAKA |
| 7x(AP) | APAPAPAPAPAPAP |
| KESGSVSSEQLAQFRSLD | KESGSVSSEQLAQFRSLD |
| EGKSSGSGSESKST | EGKSSGSGSESKST |

As noted herein, a non-peptidic molecule (e.g., SGLT2 inhibitor, lipid, protein) may be covalently linked to any portion of any of the polypeptide units or the linker moiety.

In some embodiments, the non-peptidic molecule is linked by an amido-PEG-acid or maleimide-PEG-acid.

In any of the foregoing embodiments, the linker is covalently bound to each of the polypeptides and/or non-peptidic components of the multimeric polypeptide through known chemistry and coupling means. Variations in the linker reagents to facilitate reaction with and binding to the various components of a multimeric polypeptides are embraced herein, such as reactive groups, amines, carboxylic acids, maleimides, among other derivatives and modifications routinely used in linkers.

In some embodiments, the GIP polypeptide unit of the multimeric polypeptide is attached to the linker at the C-terminus of the GIP polypeptide. In some embodiments, the GLP-1 polypeptide unit of the multimeric polypeptide is attached to the linker at the C-terminus of the GLP-1 polypeptide. In some embodiments, the multimeric polypeptide contains a GIP polypeptide unit and a GLP-1 polypeptide unit which are attached to the linker at the C-terminus of the GIP polypeptide and at the -terminus of the GLP-1 polypeptide.

### Multimeric Polypeptides

In one embodiment disclosed herein are multimeric polypeptides represented by any of the following formulas:

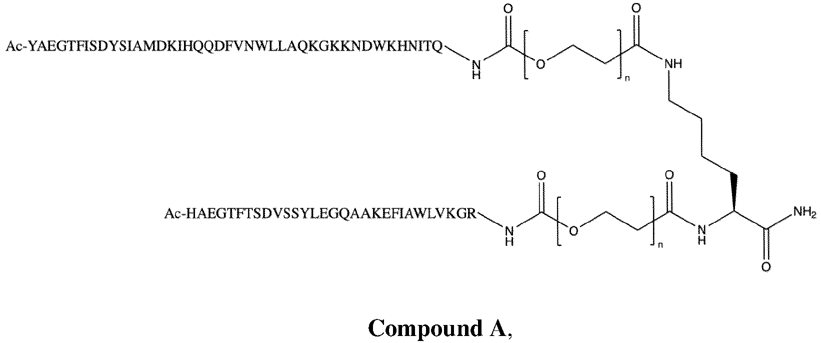

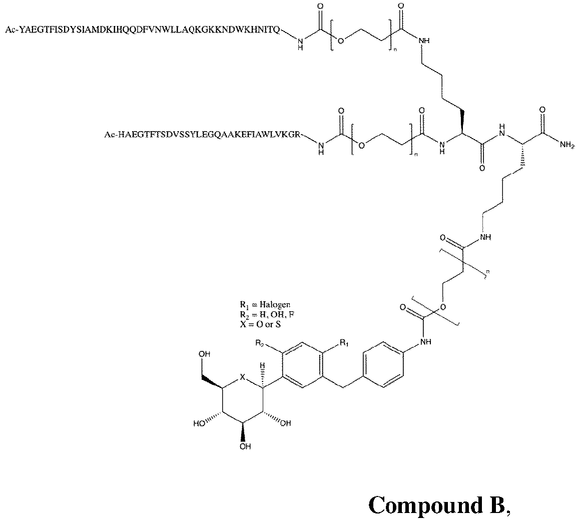

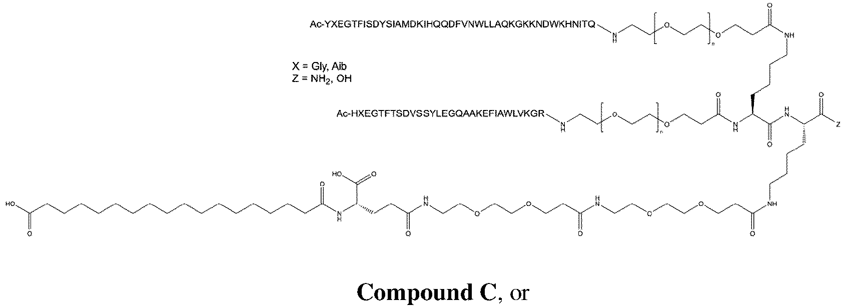

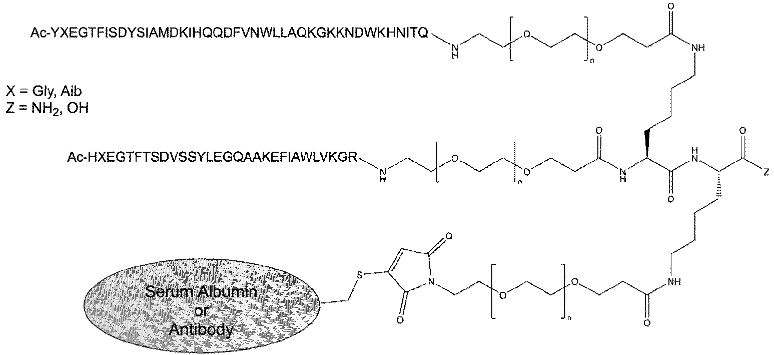

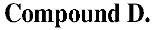

In one embodiment, in any of Compound A, Compound B, Compound C, or Compound D, n is equal to any integer between 1 and 20. In another embodiment, in any of Compound A, Compound B, Compound C, or Compound D, n is equal to 1. In another embodiment, in any of Compound A, Compound B, Compound C, or Compound D, n is equal to 2. In another embodiment, in any of Compound A, Compound B, Compound C, or Compound D, n is equal to 3. In another embodiment, in any of Compound A, Compound B, Compound C, or Compound D, n is equal to 4. In another embodiment, in any of Compound A, Compound B, Compound C, or Compound D, n is equal to 5. In another embodiment, in any of Compound A, Compound B, Compound C, or Compound D, n is equal to 6. In another embodiment, in any of Compound A, Compound B, Compound C, or Compound D, n is equal to 7. In another embodiment, in any of Compound A, Compound B, Compound C, or Compound D, n is equal to 8. In another embodiment, in any of Compound A, Compound B, Compound C, or Compound D, n is equal to 1. In another embodiment, in any of Compound A, Compound B, Compound C, or Compound D, n is equal to 9. In another embodiment, in any of Compound A, Compound B, Compound C, or Compound D, n is equal to 10. In another embodiment, in any of Compound A, Compound B, Compound C, or Compound D, n is equal to 11. In another embodiment, in any of Compound A, Compound B, Compound C, or Compound D, n is equal to 12. In another embodiment, in any of Compound A, Compound B, Compound C, or Compound D, n is equal to 13. In another embodiment, in any of Compound A, Compound B, Compound C, or Compound D, n is equal to 14. In another embodiment, in any of Compound A, Compound B, Compound C, or Compound D, n is equal to 15. In another embodiment, in any of Compound A, Compound B, Compound C, or Compound D, n is equal to 16. In another embodiment, in any of Compound A, Compound B, Compound C, or Compound D, n is equal to 17. In another embodiment, in any of Compound A, Compound B, Compound C, or Compound D, n is equal to 18. In another embodiment, in any of Compound A, Compound B, Compound C, or Compound D, n is equal to 19. In another embodiment, in any of Compound A, Compound B, Compound C, or Compound D, n is equal to 20.

In one embodiment disclosed is a multimeric polypeptide represented by the following formula:

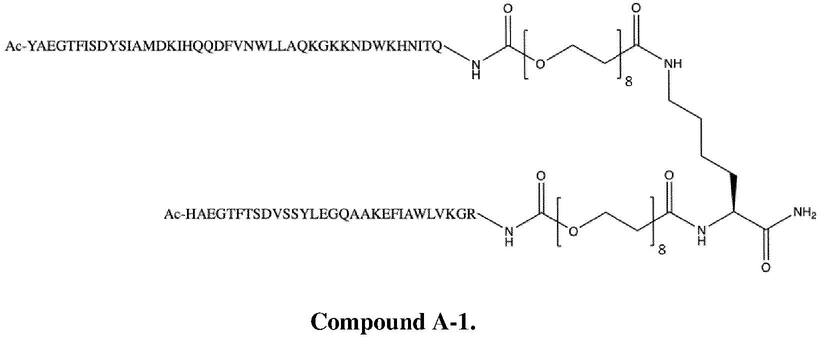

In one embodiment disclosed is a multimeric polypeptide represented by the following formula:

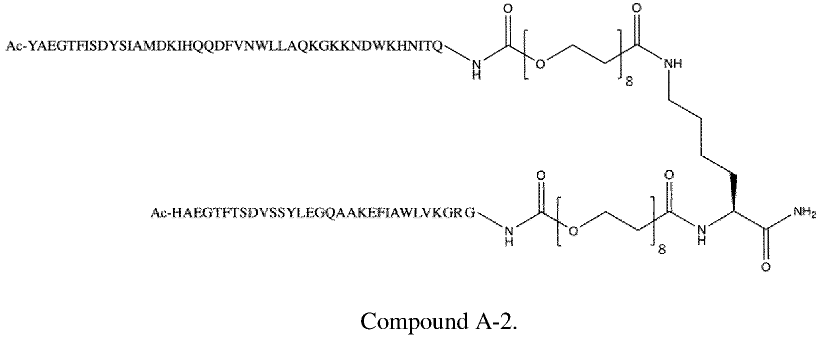

In one embodiment disclosed is a multimeric polypeptide represented by the following formula: 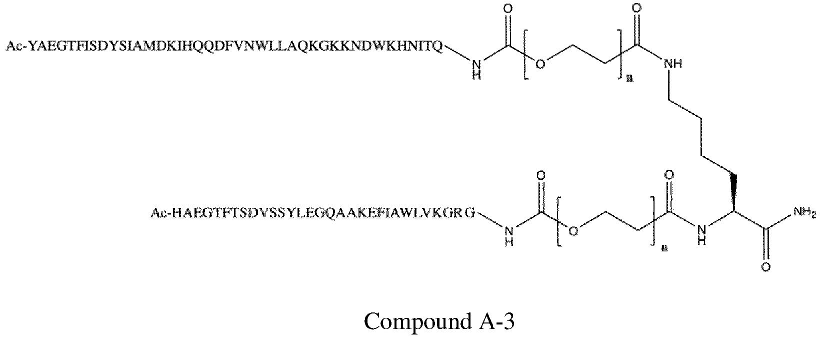 wherein is n is any integer between 1 and 20.

In one embodiment disclosed is a multimeric polypeptide represented by Compound A-3, wherein the n of Compound A-3 is 4, 8, or 20. In one embodiment disclosed is a multimeric polypeptide represented by Compound A-3, wherein the n of Compound A-3 is 3, 7, or 19.

In one embodiment disclosed is a multimeric polypeptide represented by the following Formula I:

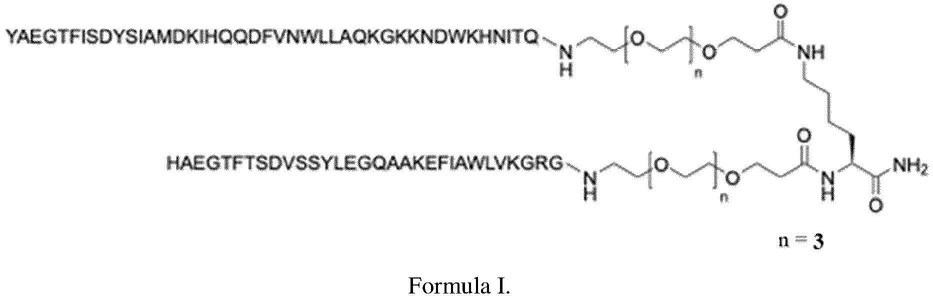

In one embodiment disclosed is a multimeric polypeptide represented by the following Formula II:

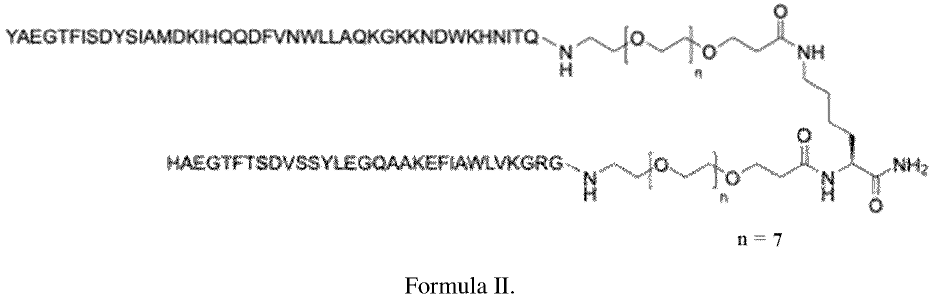

In one embodiment disclosed is a multimeric polypeptide represented by the following Formula III:

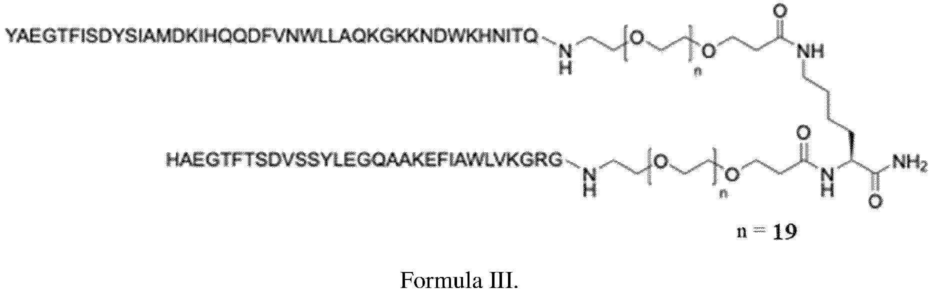

In one embodiment disclosed is a multimeric polypeptide represented by the following Formula IV:

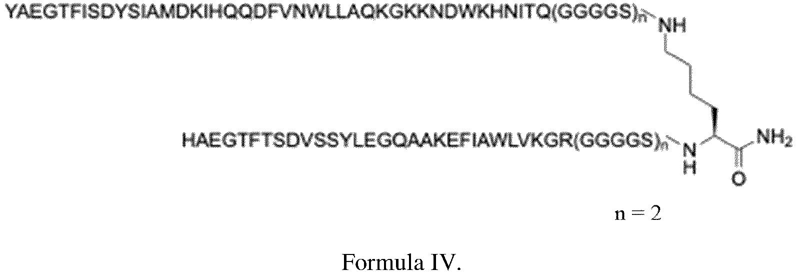

In one embodiment disclosed is a multimeric polypeptide represented by the following Formula V:

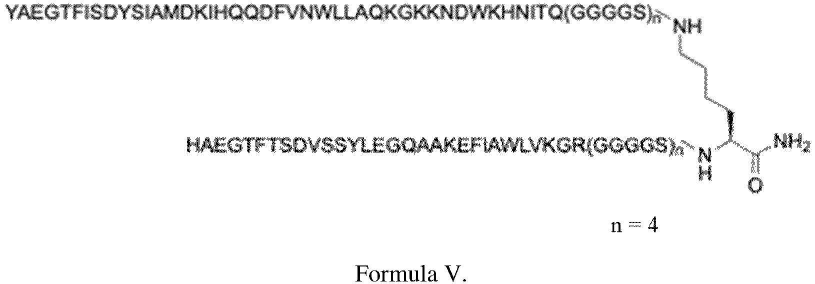

In one embodiment disclosed is a multimeric polypeptide represented by the following Formula VI:

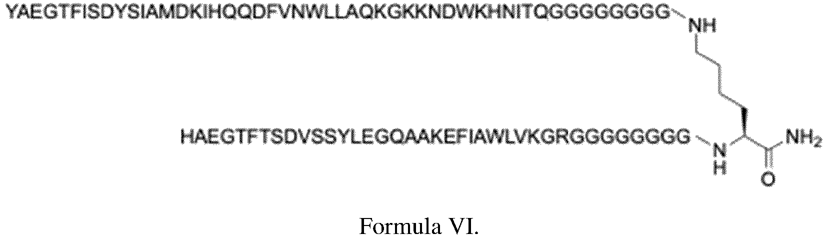

In one embodiment disclosed is a multimeric polypeptide represented by the following Formula VII:

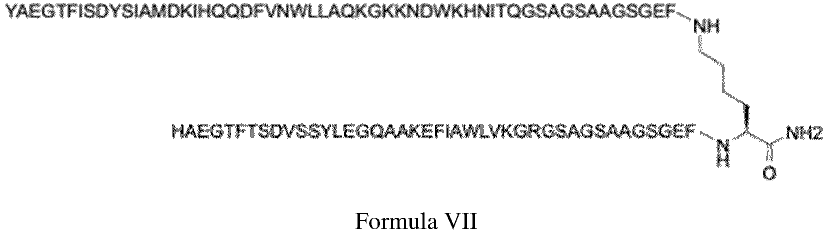

In one embodiment disclosed is a multimeric polypeptide represented by the following Formula VIII:

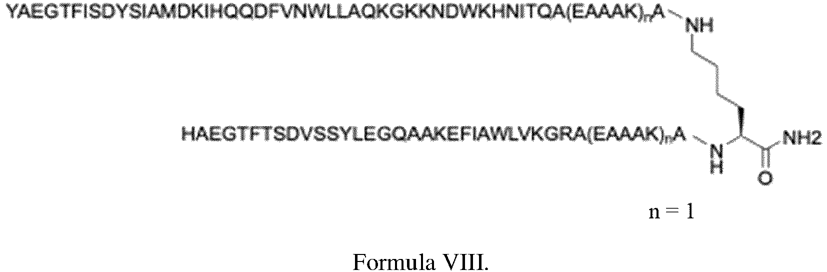

In one embodiment disclosed is a multimeric polypeptide represented by the following Formula IX:

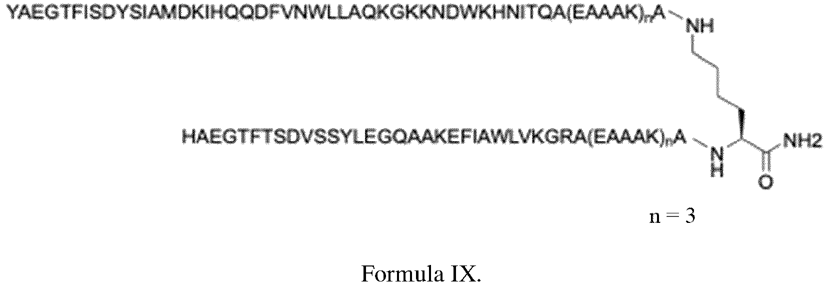

In one embodiment disclosed is a multimeric polypeptide represented by the following Formula X:

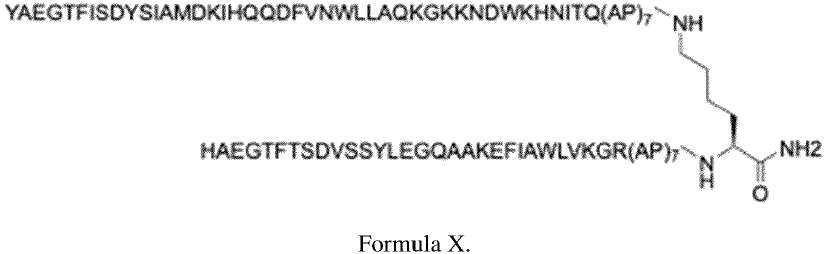

In one embodiment disclosed is a multimeric polypeptide represented by the following Formula XI:

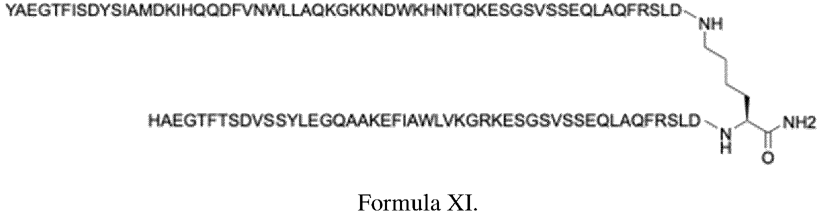

In one embodiment disclosed is a multimeric polypeptide represented by the following Formula XII:

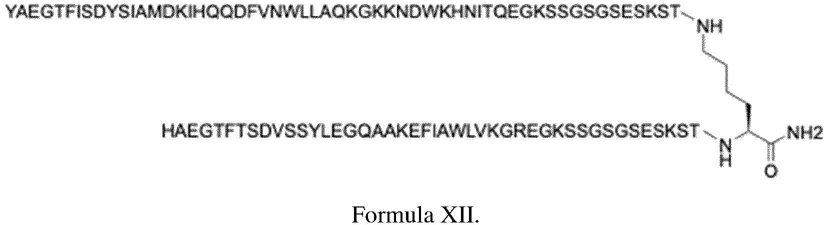

In one embodiment disclosed is a multimeric polypeptide represented by the following Formula XIV: 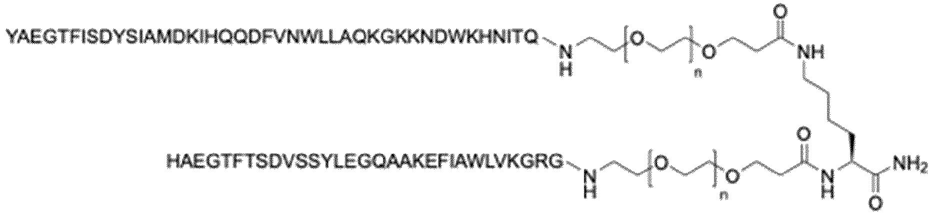 wherein n is any integer selected between 1 and 20.

In one embodiment disclosed is a multimeric polypeptide represented by Formula XIV, wherein the n of Formula XIV is 4, 8, or 20. In one embodiment disclosed is a multimeric polypeptide represented by Formula XIV, wherein the n of Formula XIV is 3, 7, or 19.

In one embodiment, the linker in any of Formula I through Formula XII is attached at any one or more lysine residues present in the GLP1 or GIP ligand or both the GLP1 and GIP ligand. In another embodiment, the linker in any of Formula I through Formula XII is attached at the lysine 27 position of the GLP-1 (7-37) amino acid sequence.

In one embodiment, the linker in any of Formula I through Formula XII is attached at the C-terminus of the GIP polypeptide unit of the multimeric polypeptide. In one embodiment, the linker in any of Formula I through Formula XII is attached at the C-terminus of the GLP-1 polypeptide unit of the multimeric polypeptide. In one embodiment, the linker in any of Formula I through Formula XII is attached at the C-terminus of both the GIP polypeptide unit and the GLP-1 polypeptide unit of the multimeric polypeptide.

In one embodiment, the GLP1 ligand corresponds to the GLP-1 (7-37) amino acid sequence. In another embodiment, the GLP1 ligand corresponds to the GLP-1 (7-37) amino acid sequence and is lipidated at lysine at amino acid position 27 of the GLP-1.

### Pharmaceutical Compositions

The multimeric polypeptides disclosed herein may be formulated for administration by combination with a pharmaceutically-acceptable excipient, diluent, vehicle or carrier. In one embodiment, such compositions are prepared for parenteral administration, such as intravenous, subcutaneous or intramuscular administration.

In some embodiments, the pharmaceutical composition can be suitable for intravenous, intraperitoneal, intramuscular, sublingual or oral administration. Pharmaceutically acceptable excipients, diluents, vehicles or carriers include sterile aqueous solutions or dispersions and sterile powders for the extemporaneous preparation of sterile injectable solutions or dispersion. The use of such media and agents for pharmaceutically active substances is well known in the art. Therapeutic pharmaceutical compositions typically are sterile and stable under the conditions of manufacture and storage. The composition can be formulated as a solution, microemulsion, liposome, or other ordered structure suitable in some embodiments to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols, such as mannitol, sorbitol, or sodium chloride in the composition. Prolonged absorption of injectable compositions can be brought about by including in the composition an agent which delays absorption, for example, monostearate salts and gelatin.

In one embodiment, the pharmaceutical formulation containing the multimeric polypeptides disclosed herein is 0.05% polysorbate-80, 50 mM sodium phosphate and 70 mM sodium chloride at a of pH 7.4.

### Methods of Administration

The multimeric polypeptides disclosed herein may be formulated for administration by combination with a pharmaceutically-acceptable excipient, diluent, vehicle or carrier. In one embodiment, such compositions are prepared for parenteral administration, such as intravenous, subcutaneous or intramuscular administration. In one embodiment, regular self-administration by use of a subcutaneous pen injection device is contemplated. In one embodiment, a liquid or dry powder aerosol for intranasal administration, including nose-to-brain administration, is provided. Formulations that allow for protection of peptides for oral administration are also embodied herein.

The dosing regimen, including but not limited to dose level, frequency of administration and duration of administration will be guided by the particular needs of the subject and prescribed by a health care professional. The dosing regimen will be readily established during clinical studies of the multimeric polypeptide for one or more diseases or conditions for which it is indicated.

### Methods of Use

Polypeptide GPCR receptor agonists in one embodiment have been found useful in the treatment of obesity, type 2 diabetes, osteoporosis, lymphatic insufficiency, migraine and cardiovascular disease, among others. The multimeric polypeptides disclosed herein are useful for treatment of such conditions and diseases.

In one embodiment, the present disclosure provides a method of treating obesity, type 2 diabetes, osteoporosis, lymphatic insufficiency, migraine and cardiovascular disease comprising administering to a subject in need of such treatment a therapeutically effective amount of any one of the multimeric polypeptides disclosed herein.

In one embodiment, the present disclosure provides a method of treating obesity and type 2 diabetes comprising administering to a subject in need of such treatment a therapeutically effective amount of any one of the multimeric polypeptides disclosed herein.

In one embodiment, the dose of the multimeric polypeptide disclosed herein, or pharmaceutically acceptable salt thereof, can range, for example, from 1 nmoles/kg to 80 nmoles/kg of patient body weight, or from 1 nmoles/kg to 40 nmoles/kg of patient body weight. In another embodiment, the dose of the multimeric polypeptide disclosed herein, or pharmaceutically acceptable salt thereof, is selected from any one of 1 nmol/kg, 2 nmol/kg, 4 nmol/kg, 5 nmol/kg, 10 nmol/kg, and 20 nmol/kg, or 40 nmol/kg of patient body weight.

In one embodiment, the administration of any of the multimeric polypeptides disclosed herein reduces fat without adversely affecting muscle mass.

### Definitions

The terms "comprise", "comprises", "comprising", "includes", "including", "having" and their conjugates mean "including but not limited to".

As used herein, the singular form "a", "an" and "the" include plural references unless the context clearly dictates otherwise. For example, the term "an enzyme" or "at least one enzyme" may include a plurality of enzymes, including mixtures thereof.

Throughout this application, various embodiments of the present disclosure may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting. Each literature reference or other citation referred to herein is incorporated herein by reference in its entirety.

In the description presented herein, each of the steps of the invention and variations thereof are described. This description is not intended to be limiting and changes in the components, sequence of steps, and other variations would be understood to be within the scope of the present invention.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

### EXAMPLES

### EXAMPLE 1

### DESIGN OF MULTIMERIC POLYPEPTIDES

The following multimeric polypeptides were synthesized and tested for GCPR agonist activity. Such activity was identified. 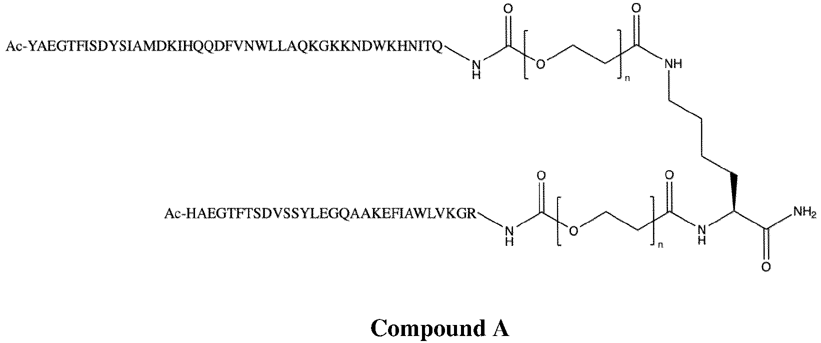 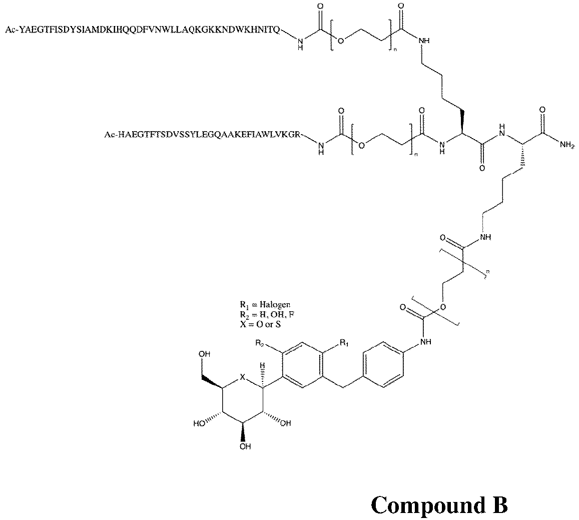 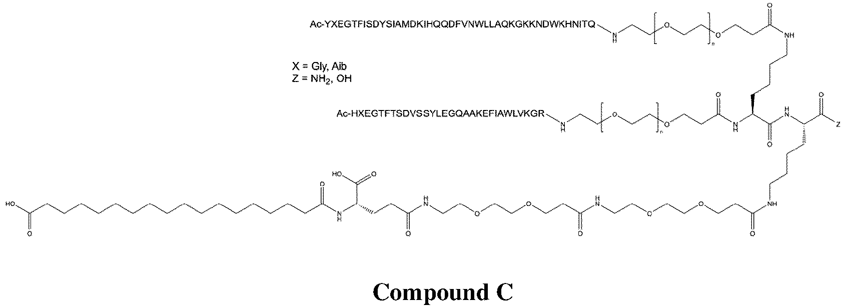 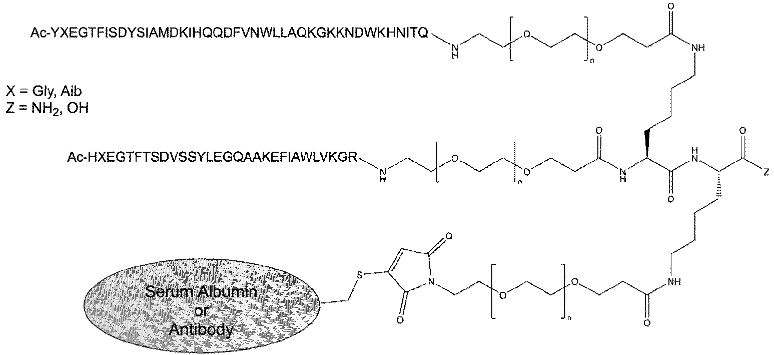 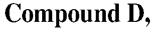 wherein n is any number selected from between 1 to 20 for any of Compound A, Compound B, Compound C, or Compound D.

### EXAMPLE 2

### IN VITRO ACTIVITY AND BINDING AFFINITIES OF MULTIMERIC POLYPEPTIDES

### I. Overview

The present study details the design of several polypeptides with multiple peptidic arms. The study demonstrates use of multimeric branched peptides to bind multiple G protein-coupled receptors (GPCRs).

One class of the newly designed multimeric polypeptides studied herein utilize a GLP-1 sequence and a GIP sequence connected to a lysine backbone via various linkers as shown in Compound A.

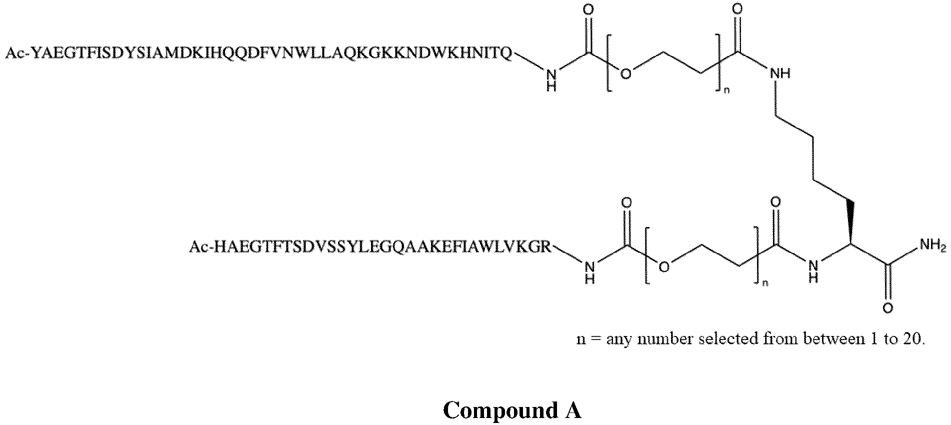

The multimeric polypeptide design has three parts: a backbone, linkers, and bioactive sequences. For the class of multimeric polypeptides shown in Compound A, the bioactive sequences are glucagon-like peptide-1 (GLP1) and gastric inhibitory polypeptide (GIP). The multimeric polypeptides shown in Compound A targets GLP and GIP receptors.

12 multimeric polypeptides were designed and synthesized, and biophysical evaluations were performed. It was observed that the linker composition and length variations were well tolerated for bioactivity using cAMP release for GLP-1 and GIP receptors. Circular dichroism studies concluded that these multimeric polypeptides adopt an alpha-helical secondary structure under aqueous conditions, consistent with the free GLP1 and GIP peptides.

### II. Results

12 multimeric polypeptides constructs (referred to as "Construct 1" through "Construct 12") were designed, synthesized, and tested for cAMP release using GLP1 and GIP pathways, and compared with the known positive controls Tirzepatide and Retatrutide.

Construct variants 01 through 12 contain the GLP1 and GIP sequences connected by various linkers to the alpha-amino and delta-amino, respectively, of the lysine used as a backbone.

**Table 1 shows the chemical structures for each of the multimeric polypeptides, Construct 01 through Construct 12.**

| **Table 1: Chemical Structures of Multimeric Polypeptides Constructs** | |
|---|---|
| Construct Name | Chemical Structure |
| Construct 01 | 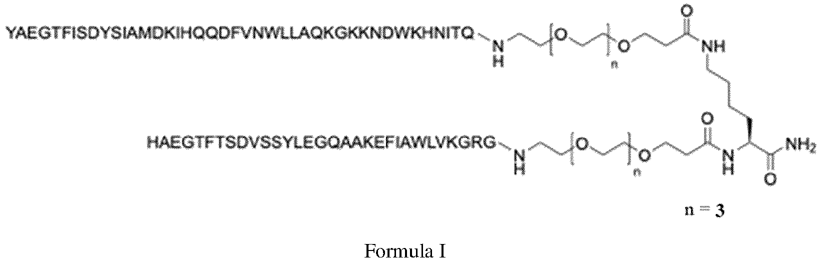 |
| Construct 02 | 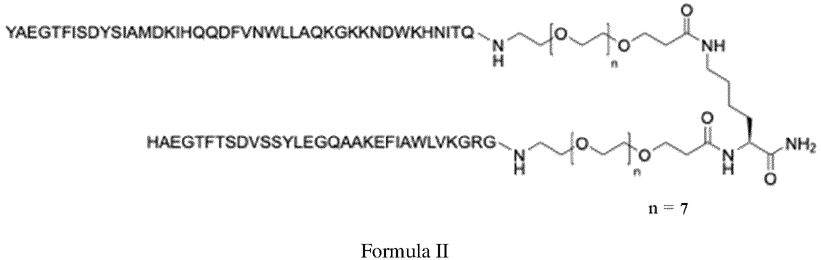 |
| Construct 03 | 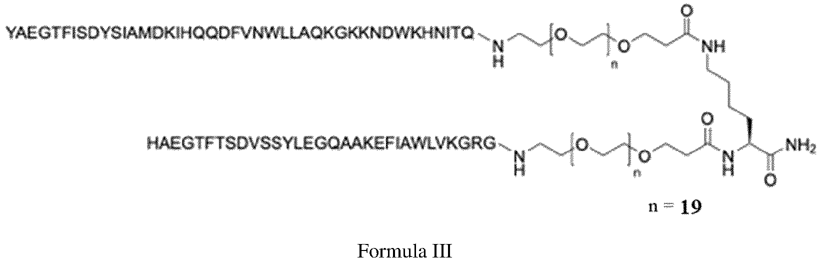 |
| Construct 04 | 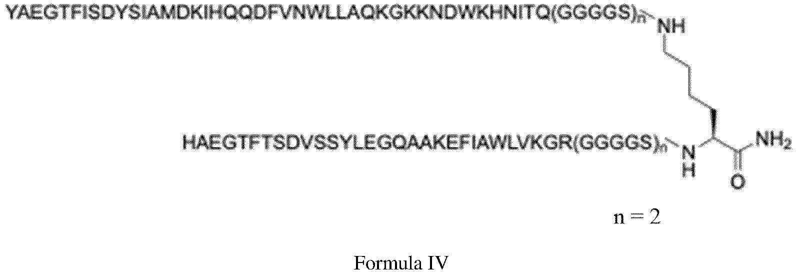 |
| Construct 05 | 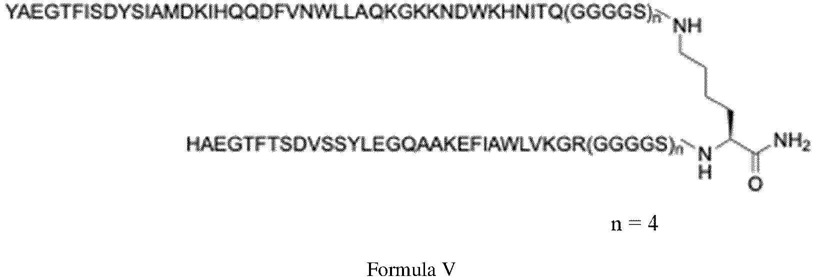 |
| Construct 06 | 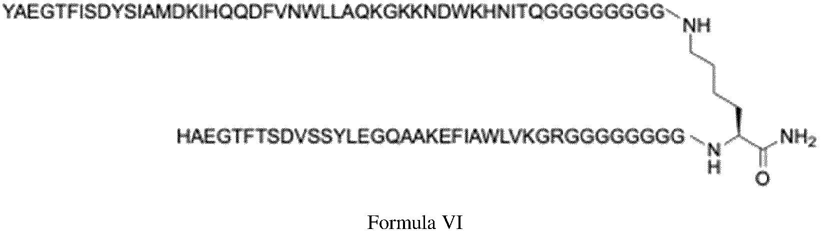 |
| Construct 07 | 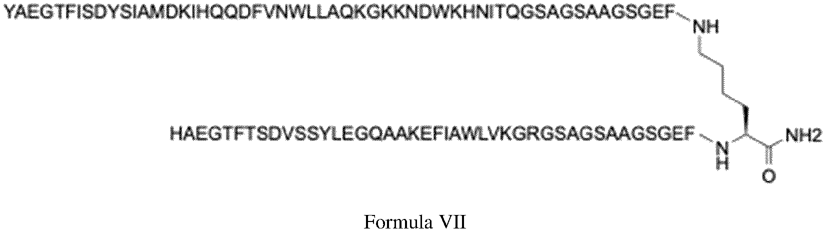 |
| Construct 08 | 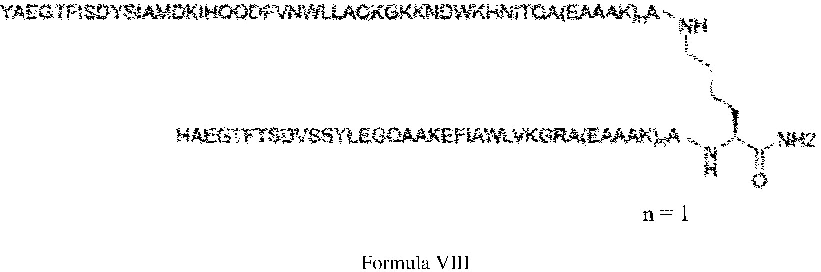 |
| Construct 09 | 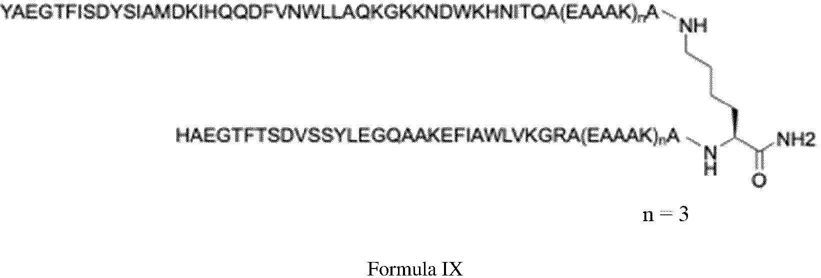 |
| Construct 10 | 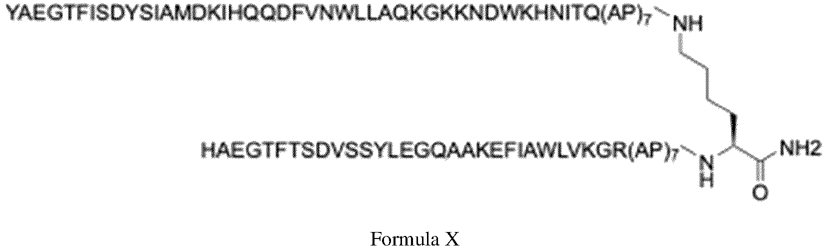 |
| Construct 11 | 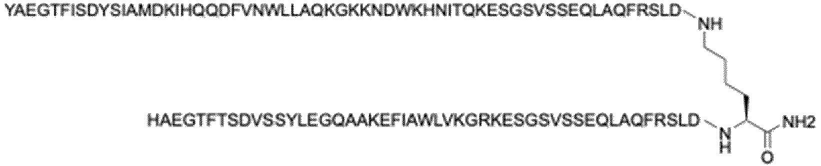 |
| | 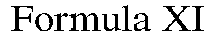 |
| Construct 12 | 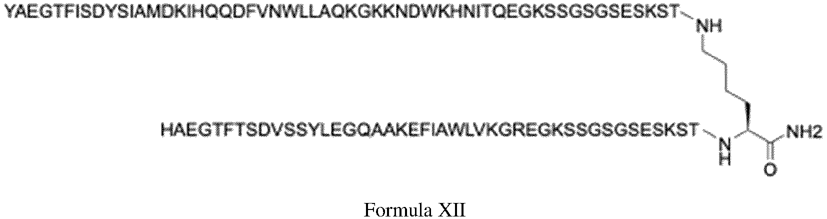 |

The positive control in the assay was the well-known drug Tirzepatide, marketed under the trade name Mounjaro by Eli Lily. Another potent positive control used is Retatrutide (LY3437943), under clinical development by Eli Lily. cAMP release was also compared to the native ligands GLP1 for the GLP1 receptor and GIP for the GIP receptor.

The Constructs were synthesized using an automated fast flow peptide synthesis (AFPS) approach. Fmoc-Lys(Mtt)-OH was coupled as the C-terminal residue, and chain extension from the alpha amino proceeded as usual, with a final acetyl cap on the N-terminus. Next, the resin was removed from the synthesizer, and Mtt protection on the c-terminal lysine's delta amino was removed by treatment with TFA:TIPS:DCM (1:5:94) at RT. Once Mtt deprotection was complete, as evidenced by failure to produce more yellow deprotection byproduct upon addition of fresh deprotection solution, the resin was returned to the AFPS instrument and chain extension from the delta amino proceeded as usual with an N-terminal acetyl cap. The peptide was cleaved from the resin and deprotected using TFA:Water:EDT:TIPS (94:2.5:2.5: 1) for 2 hours at room temperature, followed by filtration and precipitation with cold diethyl ether. The crude peptide was dissolved in the appropriate concentration of aqueous acetonitrile and purified using reverse-phase HPLC chromatography on a C18 Zorbax 300SB stationary phase.

Constructs 01-12 were subjected to circular dichroism spectroscopy from 200 nm to 280nm. Constructs 1-10 and 12 had a negative absorption band around 208 nm and 222 nm, suggesting alpha-helical secondary structure under aqueous conditions. This was consistent with expectations based on the structures of GLP1 and GIP.

**Table 2 provides the results of the cAMP release assay results for GLP1 and GIP for each of the multimeric polypeptides (Constructs 01 through 12) and describes the respective linkers, the linker length, and bioactive branches of each Construct variant 01 through 12.**

| **Table 2: Multimeric Polypeptides cAMP Release Assay Results for GLP1 and GIP** | | | | | | |
|---|---|---|---|---|---|---|
| **Name** | **Linker** | **Linker Length (gly. eqv.)** | **Branch 1** | **Branch 2** | **GLP1 EC50 (95% CI)** | **GIP EC50 (95% CI)** |
| Construct 01 | Amido-PEG4-Acid | 5 | GLP1 | GIP | 2.0e-012 (1.0e-012 to 4.0e-012) | 2.2e-011 (1.8e-011 to 2.6e-011) |
| Construct 02 | Amido-PEG8-Acid | 9 | GLP1 | GIP | 6.0e-012 (5.0e-012 to 7.2e-012) | 2.8e-011 (2.5e-011 to 3.3e-011) |
| Construct 03 | Amido-PEG20-Acid | 20 | GLP1 | GIP | 4.7e-012 (3.7e-012 to 5.9e-012) | 2.3e-011 (2.0e-011 to 2.7e-011) |
| Construct 04 | 2x(GGGGS) | 10 | GLP1 | GIP | 3.5e-012 (2.4e-012 to 5.1e-012) | 4.8e-011 (3.8e-011 to 6.2e-011) |
| Construct 05 | 4x(GGGGS) | 20 | GLP1 | GIP | 1.7e-012 (1.4e-012 to 2.2e-012) | 3.0e-011 (2.3e-011 to 4.0e-011) |
| Construct 06 | 8xG | 8 | GLP1 | GIP | 1.7e-012 (1.1e-012 to 2.6e-012) | 2.1e-011 (1.7e-011 to 2.6e-011) |
| Construct 07 | GSAGSAAGSGEF | 12 | GLP1 | GIP | 3.7e-012 (3.3e-012 to 4.3e-012) | 2.9e-011 (2.1e-011 to 3.9e-011) |
| Construct 08 | A-(EAAAK)-A | 7 | GLP1 | GIP | 9.7e-012 (7.3e-012 to 1.3e-011) | 5.1e-011 (4.3e-011 to 6.1e-011) |
| Construct 09 | A-3x(EAAAK)-A | 17 | GLP1 | GIP | 1.1e-011 (7.8e-012 to 1.5e-011) | 4.5e-011 (3.9e-011 to 5.3e-011) |
| Construct 10 | 7x(AP) | 14 | GLP1 | GIP | 3.8e-012 (2.6e-012 to 5.5e-012) | 2.9e-011 (2.5e-011 to 3.4e-011) |
| Construct 11 | KESGSVSSEQLAQ FRSLD | 18 | GLP1 | GIP | 1.5e-011 (1.3e-011 to 1.7e-011) | 9.7e-011 (8.3e-011 to 1.1e-010) |
| Construct 12 | EGKSSGSGSESKST | 14 | GLP1 | GIP | 5.4e-012 (4.9e-012 to 5.9e-012) | 4.6e-011 (4.0e-011 to 5.3e-011) |

**Table 3 provides the results of the cAMP release assay results for GLP1 and GIP for Tirzepatide, Retatrutide, native GLP1 ligand, and native GIP ligand.**

| **Table 3: cAMP release assay results for GLP1 and GIP** | | |
|---|---|---|
| **Name** | **GLP1 EC50 (95% CI)** | **GIP EC50 (95% CI)** |
| Tirzepatide | 2.0e-013 (1.7e-013 to 2.3e-013) | 1.6e-010 (1.2e-010 to 2.1e-010) |
| Retatrutide | 6.7e-015 (5.6e-015 to 7.9e-015) | 1.3e-014 (1.1e-014 to 1.4e-014) |
| GLP1 | 6.3e-012 (5.4e-012 to 7.2e-012) | |
| GIP | | 5.7e-011 (4.7e-011 to 6.9e-011) |

The amino acid sequence of Tirzepatide described in Table 3 is YGEGTFTSDYSIYLDKIAQKAFVQWLIAGGPSSGAPPPS.

The amino acid sequence of Retatrutide described in Table 3 is Y[Aib]QGTFTSDYSI[αMeL]LDKK[AEEA-γE-C20acid]AQ[Aib]AFIEYLLEGGPSSGAPPPS.

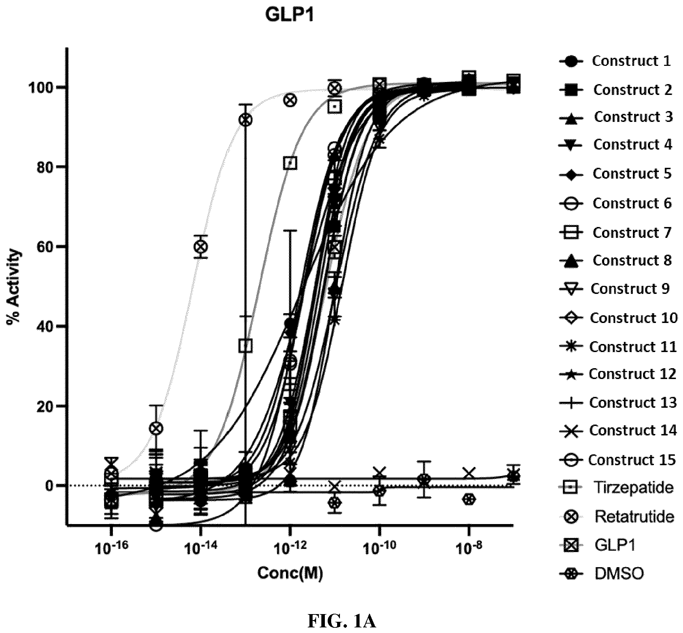

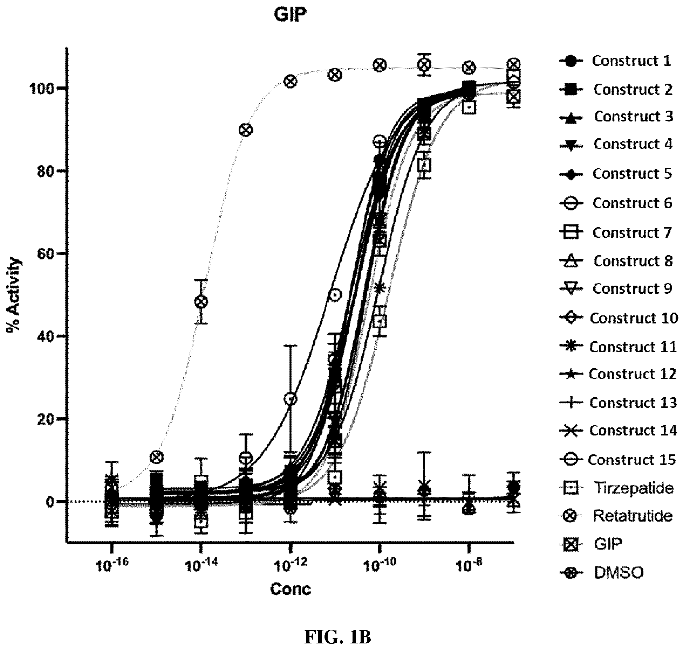

**FIGS. 1A** and **1B** show a plotted graph of the cAMP assay results for GLP1 and GIP receptor activation assays. **FIG 1A** shows a plotted graph of the cAMP assay results for GLP1 receptor activation for Construct 01 through 12, Tirzepatide, and Retatrutide. **FIG. 1B** shows a plotted graph of the cAMP assay results for GIP receptor activation for Construct 01 through 12, Tirzepatide, and Retatrutide.

LCMS analysis was performed for each of the Constructs 01 through 12.

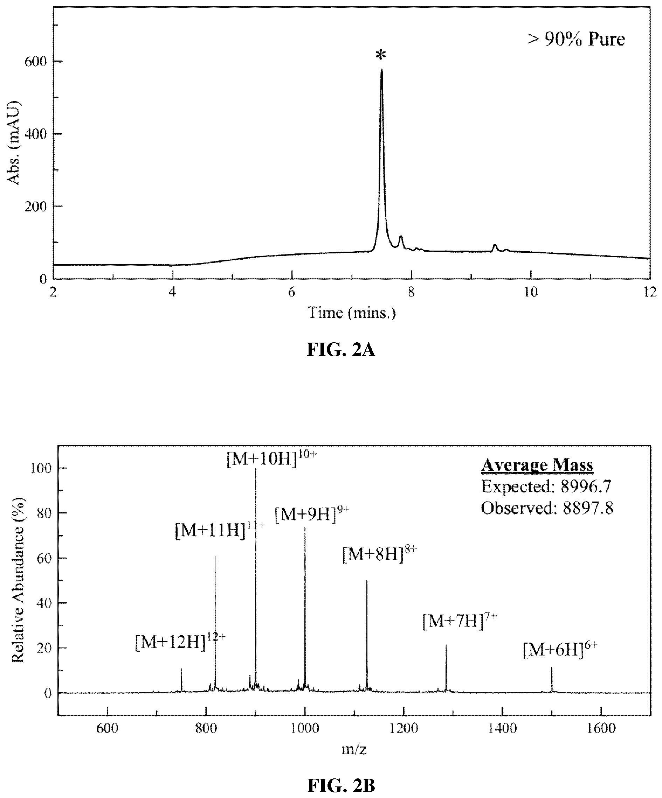

**FIG. 2A** shows UV-Vis Spectrum (λ = 210 nm) for Construct 01, and **FIG. 2B** shows the mass spectrum of Construct 01.

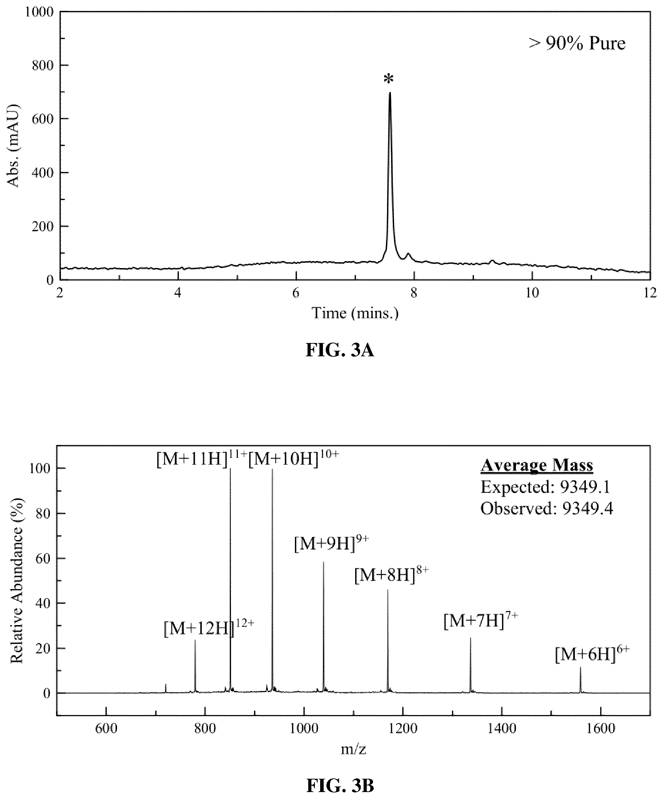

**FIG. 3A** shows UV-Vis Spectrum (λ = 210 nm) for Construct 02, and **FIG. 3B** shows the mass spectrum of Construct 02.

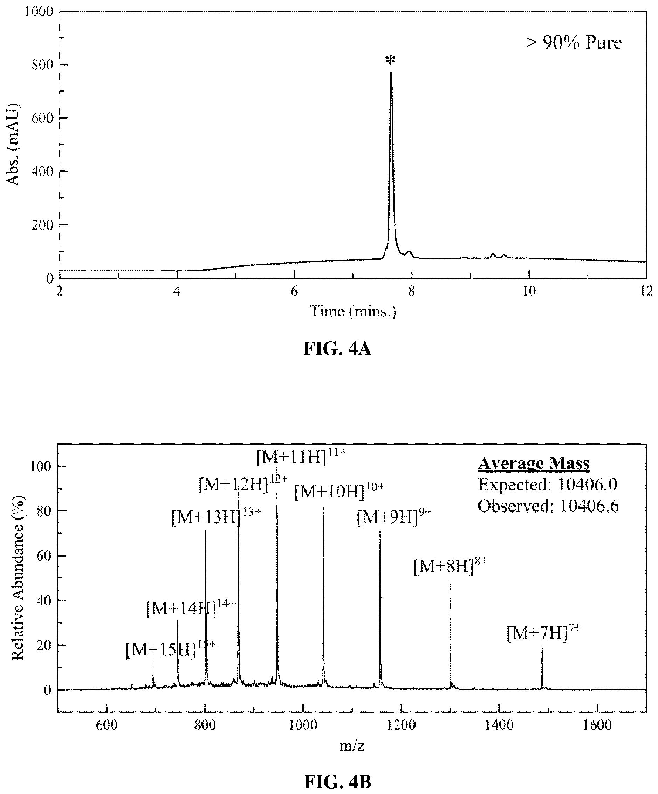

**FIG. 4A** shows UV-Vis Spectrum (λ = 210 nm) for Construct 03, and **FIG. 4B** shows the mass spectrum of Construct 03.

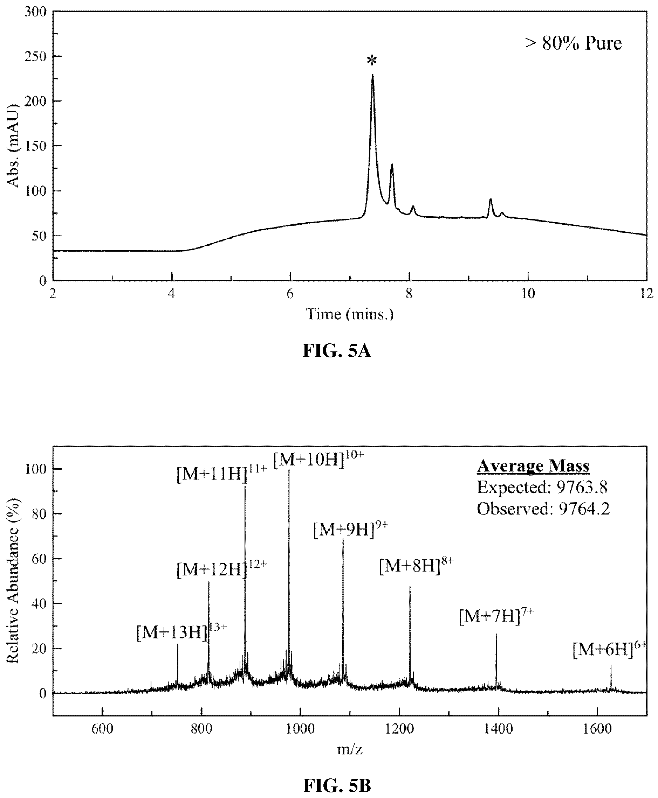

**FIG. 5A** shows UV-Vis Spectrum (λ = 210 nm) for Construct 04, and **FIG. 5B** shows the mass spectrum of Construct 04.

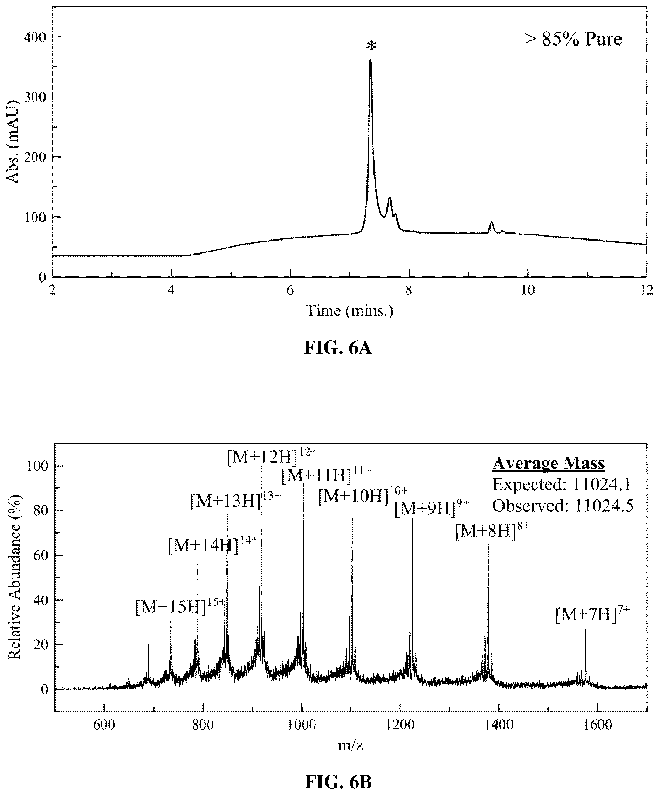

**FIG. 6A** shows UV-Vis Spectrum (λ = 210 nm) for Construct 05, and **FIG. 6B** shows the mass spectrum of Construct 05.

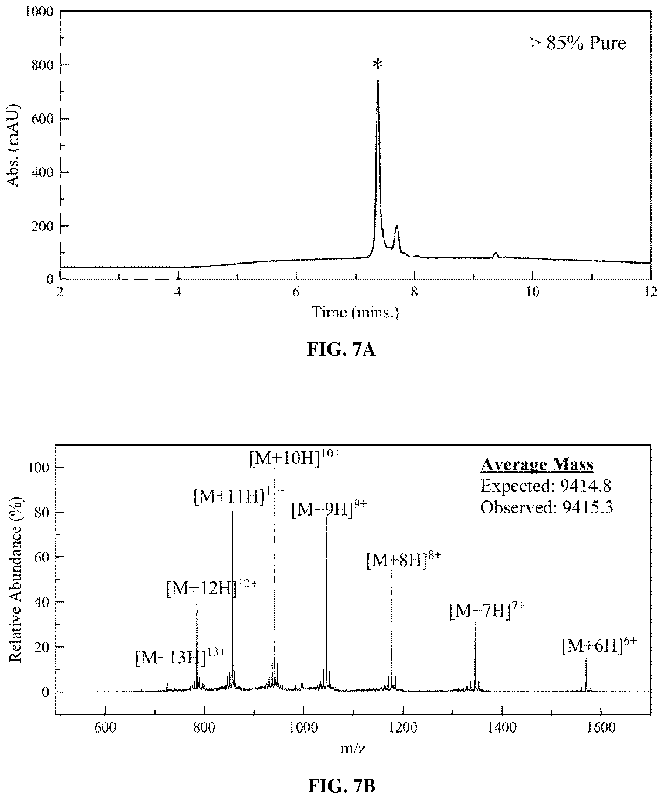

**FIG. 7A** shows UV-Vis Spectrum (λ = 210 nm) for Construct 06, and **FIG. 7B** shows the mass spectrum of Construct 06.

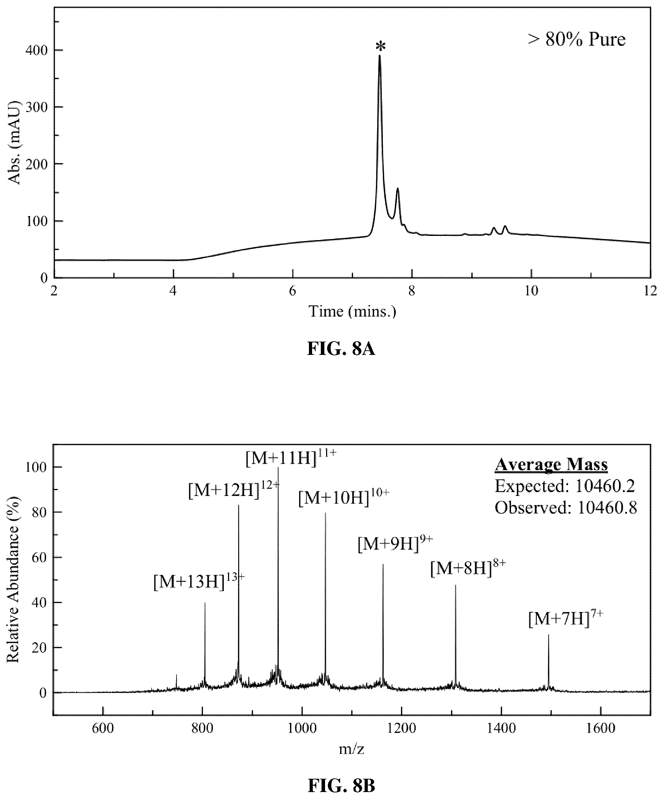

**FIG. 8A** shows UV-Vis Spectrum (λ = 210 nm) for Construct 07, and **FIG. 8B** shows the mass spectrum of Construct 07.

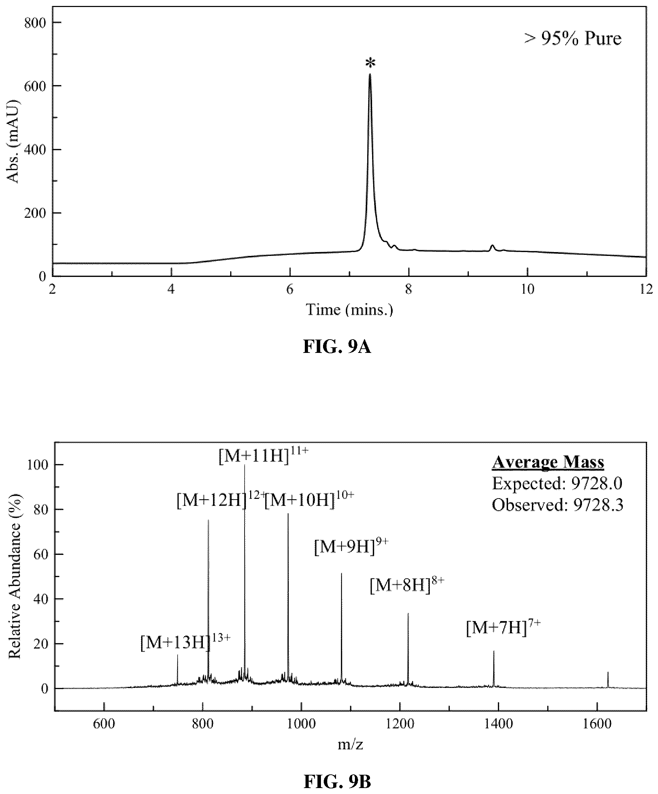

**FIG. 9A** shows UV-Vis Spectrum (λ = 210 nm) for Construct 08, and **FIG. 9B** shows the mass spectrum of Construct 08.

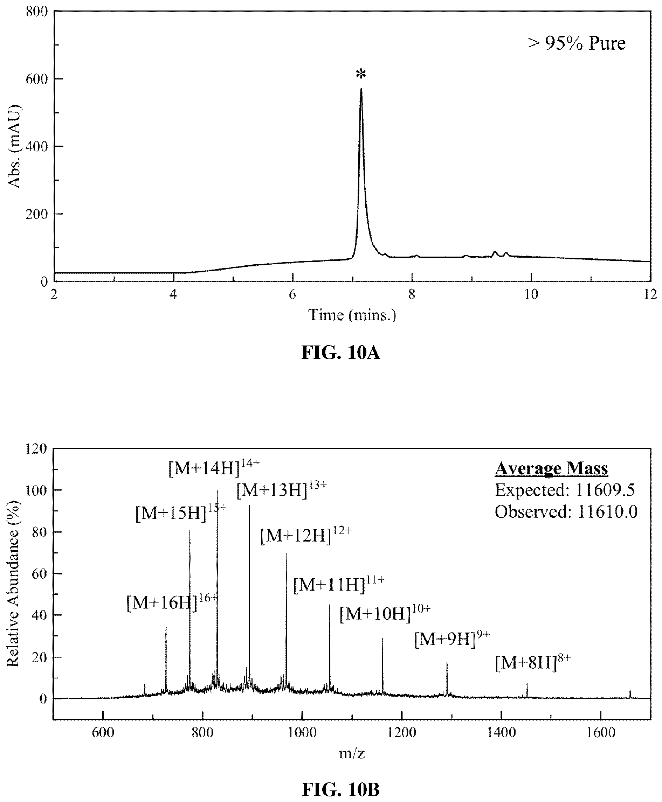

**FIG. 10A** shows UV-Vis Spectrum (λ = 210 nm) for Construct 09, and **FIG. 10B** shows the mass spectrum of Construct 09.

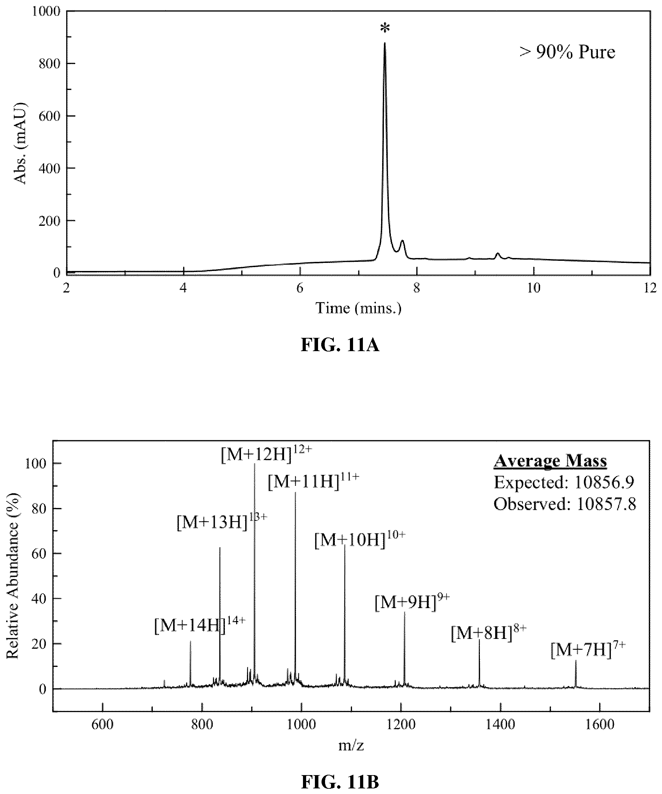

**FIG. 11A** shows UV-Vis Spectrum (λ = 210 nm) for Construct 10, and **FIG. 11B** shows the mass spectrum of Construct 10.

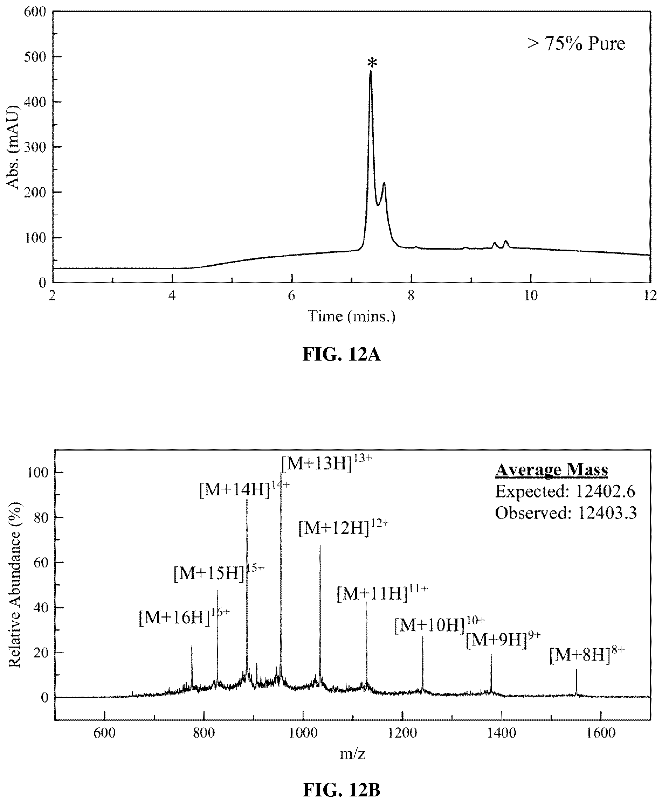

**FIG. 12A** shows UV-Vis Spectrum (λ = 210 nm) for Construct 11, and **FIG. 12B** shows the mass spectrum of Construct 11.

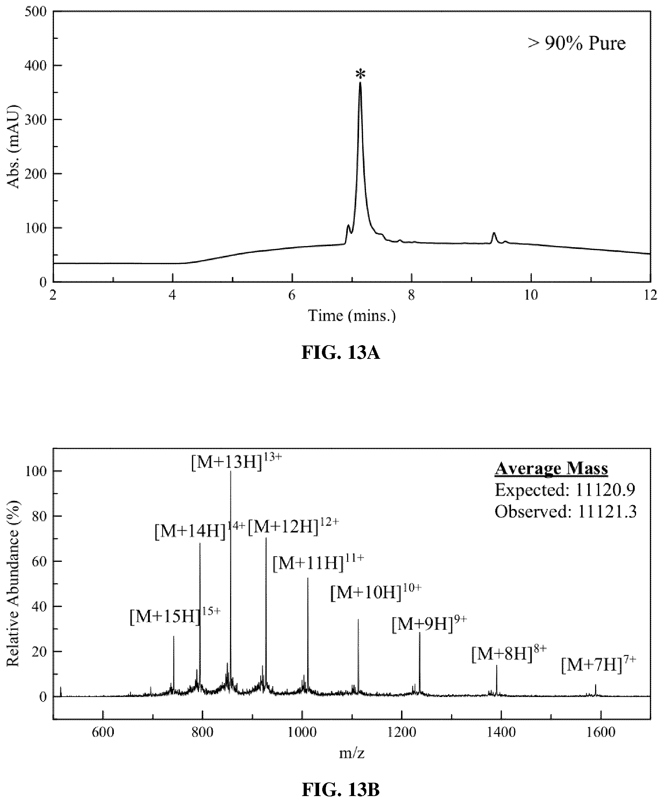

**FIG. 13A** shows UV-Vis Spectrum (λ = 210 nm) for Construct 12, and **FIG. 13B** shows the mass spectrum of Construct 12.

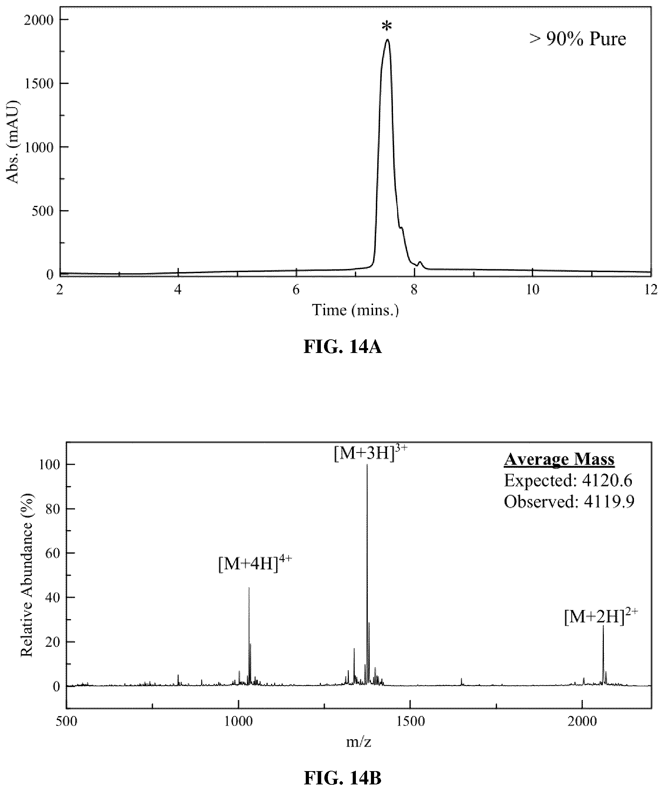

**FIG. 14A** shows UV-Vis Spectrum (λ = 210 nm) for Tirzepatide, and **FIG. 14B** shows the mass spectrum of Tirzepatide.

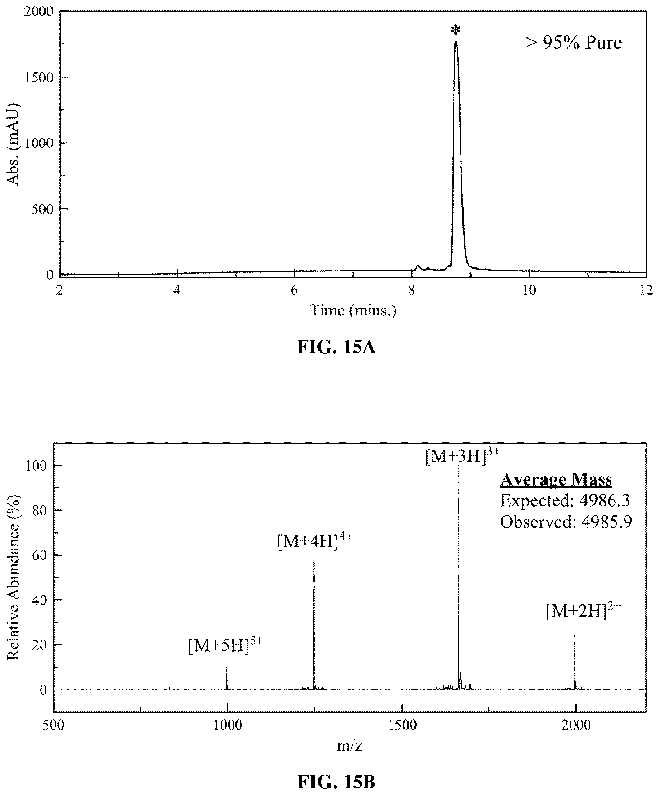

**FIG. 15A** shows UV-Vis Spectrum (λ = 210 nm) for Retatrutide, and **FIG. 15B** shows the mass spectrum of Retatrutide.

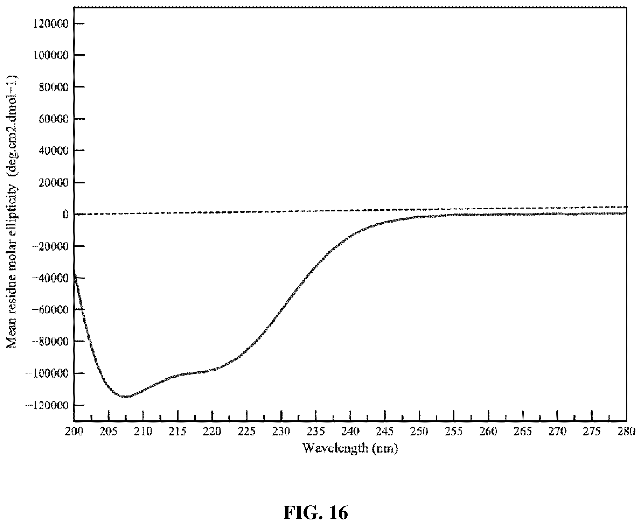

**FIG. 16** shows the circular dichroism (CD) spectrum of Construct 01 in water.

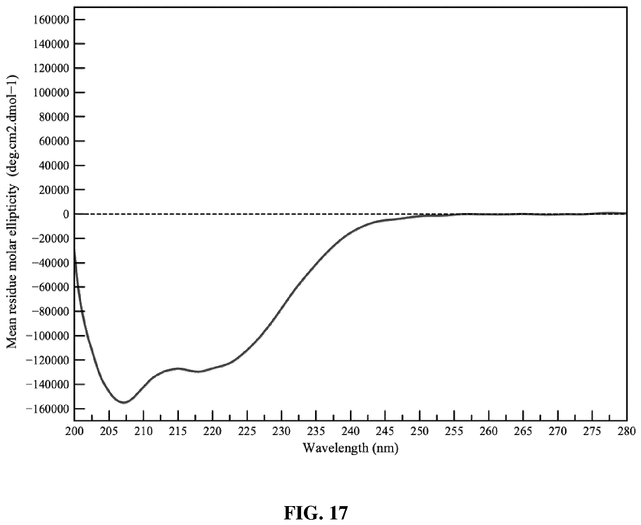

**FIG. 17** shows the circular dichroism (CD) spectrum of Construct 02 in water.

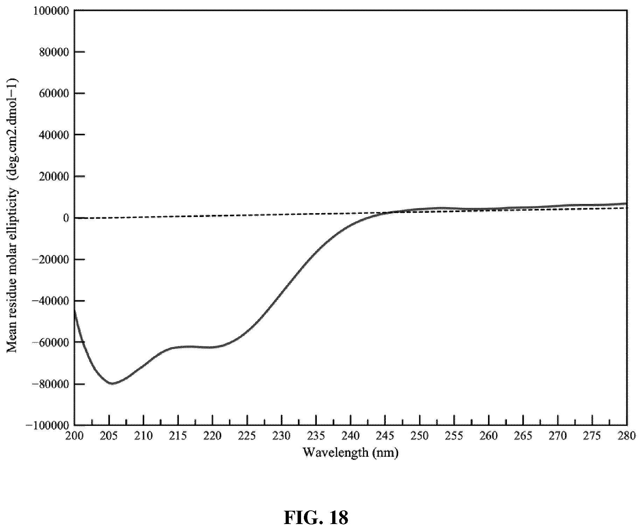

**FIG. 18** shows the circular dichroism (CD) spectrum of Construct 03 in water.

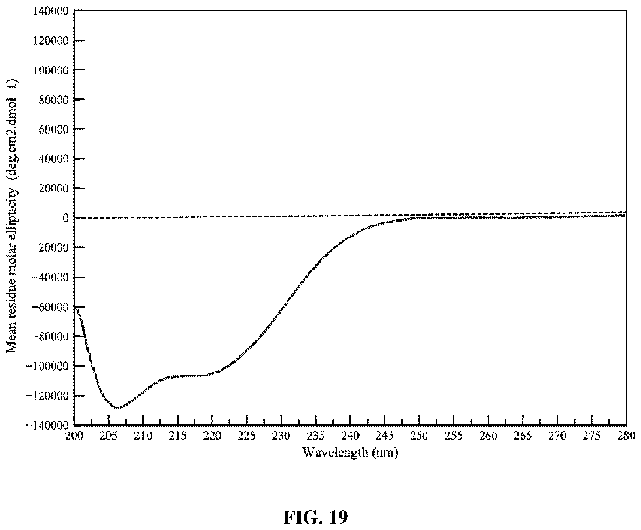

**FIG. 19** shows the circular dichroism (CD) spectrum of Construct 04 in water.

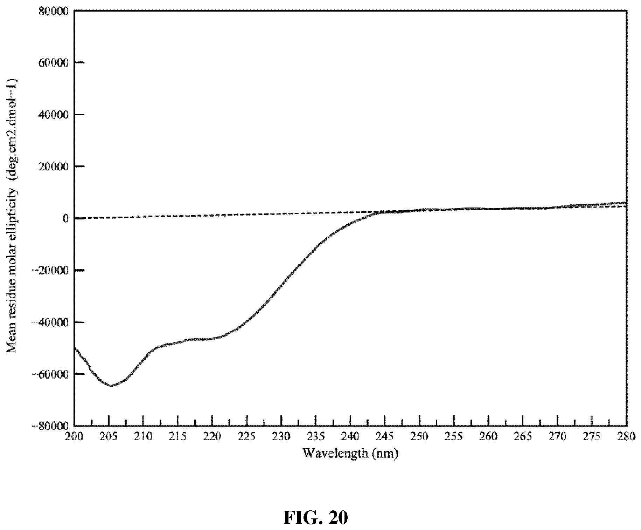

**FIG. 20** shows the circular dichroism (CD) spectrum of Construct 05 in water.

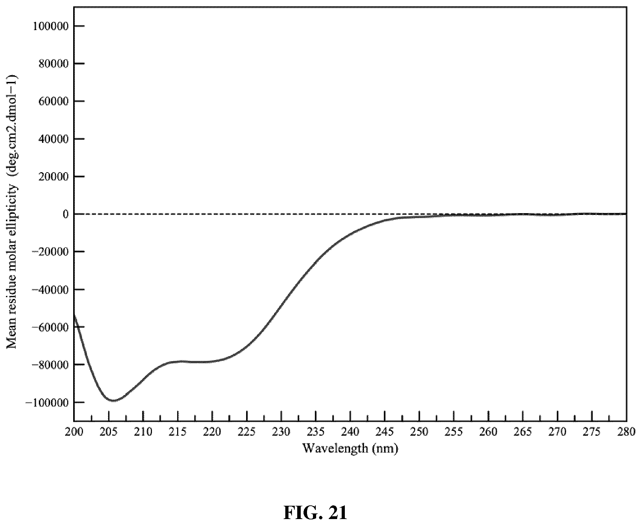

**FIG. 21** shows the circular dichroism (CD) spectrum of Construct 06 in water.

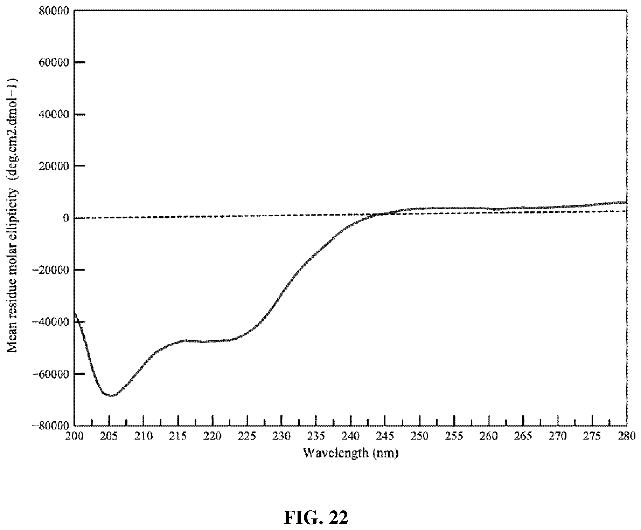

**FIG. 22** shows the circular dichroism (CD) spectrum of Construct 07 in water.

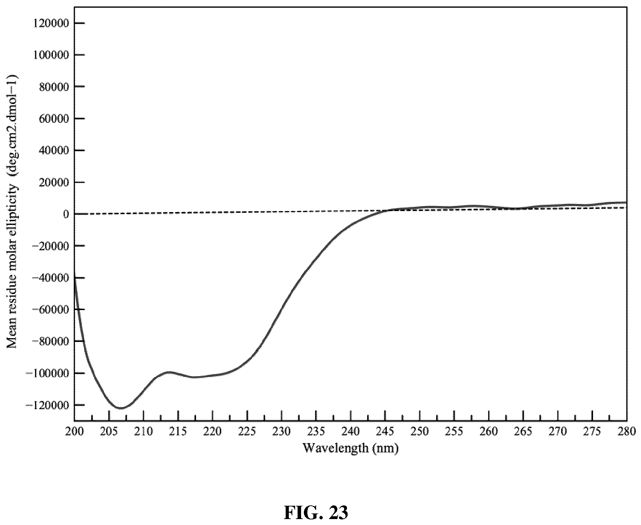

**FIG. 23** shows the circular dichroism (CD) spectrum of Construct 08 in water.

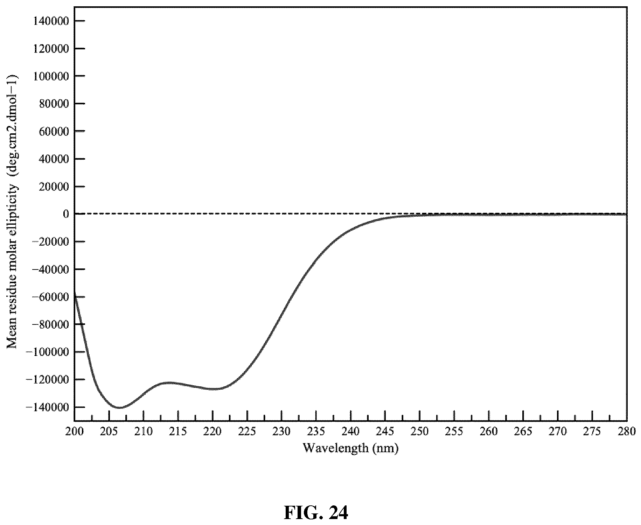

**FIG. 24** shows the circular dichroism (CD) spectrum of Construct 09 in water.

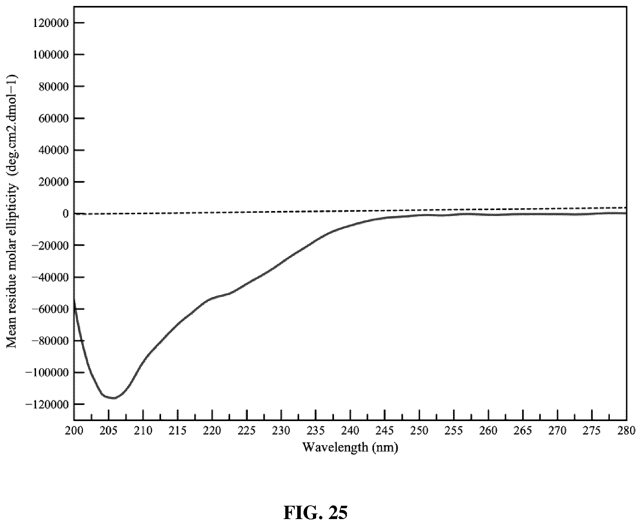

**FIG. 25** shows the circular dichroism (CD) spectrum of Construct 10 in water.

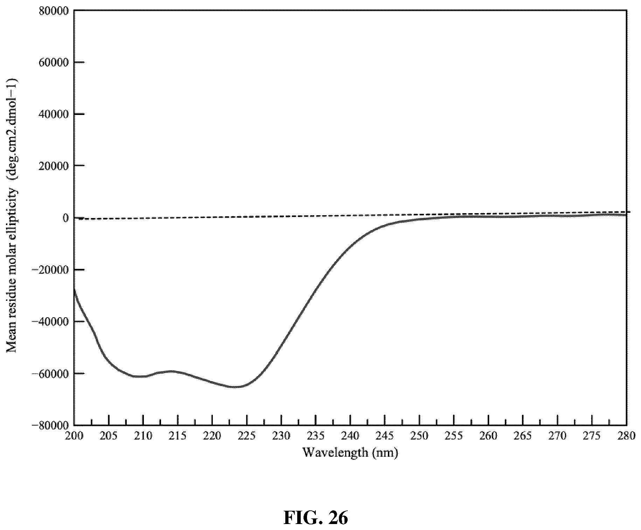

**FIG. 26** shows the circular dichroism (CD) spectrum of Construct 11 in water.

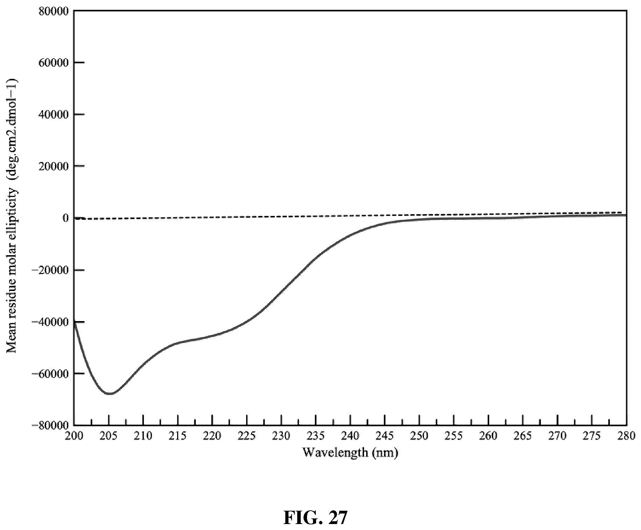

**FIG. 27** shows the circular dichroism (CD) spectrum of Construct 12 in water.

**Table 4 provides the molelcular weight and chemical purity of each of the Constructs 01 through 12 and Tirzepatide.**

| **Table 4: M.W. and Chemical Purity** | | |
|---|---|---|
| **Name** | **M.W.** | **Chemical Purity** |
| Construct 01 | 8996 | > 90% |
| Construct 02 | 9349 | > 90% |
| Construct 03 | 10406 | > 90% |
| Construct 04 | 9763 | > 80% |
| Construct 05 | 11024 | > 85% |
| Construct 06 | 9414 | > 85% |
| Construct 07 | 10460 | > 80% |
| Construct 08 | 9728 | > 95% |
| Construct 09 | 11609 | > 95% |
| Construct 10 | 10856 | > 75% |
| Construct 11 | 12402 | > 90% |
| Construct 12 | 11120 | > 90% |
| Tirzepatide | 4121 | > 90% |

### III. Conclusion

In the GLP1R activation assay, Constructs 01-12 demonstrated cAMP release comparable to the GLP1 control. Similarly, in the GIPR activation assay, Constructs 01-12 demonstrated cAMP release similar to the GIP control. The EC50 for cAMP release for Tirzepatide was in the low picomolar range for the GLP1 receptor and the low nanomolar range for the GIP receptor, respectively. The EC50 for cAMP release for Retatrutide was in the femtomolar range for the GLP1 receptor and the high femtomolar range for the GIP receptor, respectively, suggesting positive controls for this experiment worked as expected.

Taken together, these results demonstrated high performance of multimeric peptides designed to activate both GLP1 and GIP receptors. In-vitro activity in line with positive controls and marketed drugs showed the efficacy of the Construct designs. Using a very naive backbone (lysine), virtually no sensitivity to the linker structure was found. This multimeric polypeptide architecture offers a highly advantaged approach to multi-target drug design. This multimeric polypeptide architecture provides advantages for therapeutic drugs with multiple cell surface targets.

### EXAMPLE 3

### EFFECT OF TREATMENT WITH MULTIMERIC POLYPEPTIDES ON WEIGHT LOSS, GLUCOSE TOLERANCE, AND BODY COMPOSITION IN DIO MICE MODEL

### I. Overview

The present study shows the dose-dependent effects on weight loss, glucose tolerance, and body composition of the branched peptide, hereinafter referred to as BXL-801, in a DIO mouse model. The formula of BXL-801 is represented below as Compound A-1. BXL-801 is also referred to as Construct 02 in Example 2.

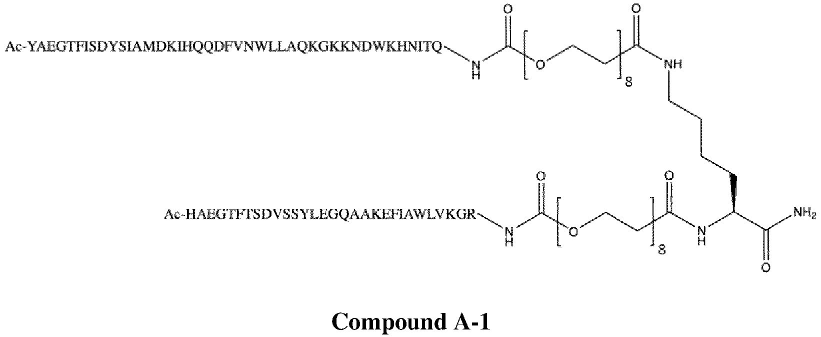

### II. Methods

Four groups of diet-induced obesity (DIO) mice were dosed every day subcutaneously for 36 days. There were 8 animals in each group and 4 groups for total of 32 mice. The mice were acclimated for two weeks with 5 mL/kg of vehicle each day. The mice were fed a high fat diet for 2 weeks prior to and throughout the entirety of the study.

The average starting body weights for each group were: Group 1: 42.7 g +/- 1.5 g; Group 2: 42.6 g +/- 1.5 g; Group 3: 42.5 g +/- 1.2 g; and Group 4: 42.7 g +/- 1.7 g. The dosing for each group was: Group 1: vehicle; Group 2: 1-4 nmol/kg of BXL-801; Group 3: 5-20 nmol/kg of BXL-801; and Group 4: 10-40 nmol/kg of BXL-801.

The dosing schedule was 1 nmol/kg, 5 nmol/kg, and 10 nmol/kg of BXL-801 for the first 17 days. At day 17, the doses were doubled to 2 nmol/kg, 10 nmol/kg, and 20 nmol/kg of BXL-801, respectively. On day 24 the doses were doubled again to 4 nmol/kg, 20 nmol/kg, and 40 nmol/kg of BXL-801, respectively. The volume of each daily dose administered subcutaneously was 5 mL/kg.

The vehicle/buffer was 0.05% polysorbate-80, 50 mM sodium phosphate and 70 mM sodium chloride at a pH 7.4. BXL-801 was administered in a formulation of .05% polysorbate-80, 50 mM sodium phosphate and 70 mM sodium chloride at a pH 7.4.

Body weight and food intake were measured every 3 days.

Oral glucose tolerance tests were performed and compared between groups. The glucose tolerance test was performed 30 minutes post dosing after 16 hours fasting. The pre-glucose was measured via tail vein by using Accu-Chek Guide and then 2 g/kg glucose was administered via PO. Glucose was evaluated at all time points including t=-30min(pre-dose), 0(pre glucose administration), 15 minutes, 30 minutes, 60 minutes, and 120 minutes following the glucose challenge and reported in mmol/L.

The body mass composition was taken at the end of the study via Dual-Energy X-ray Absorptiometry (DEXA).

The data was analyzed with a simple one-way ANOVA followed by Tukey's HSD to identify pairwise differences.

All tests and analyses were conducted using routine methods known in the art.

### III. Results

### III(A). Weight Loss

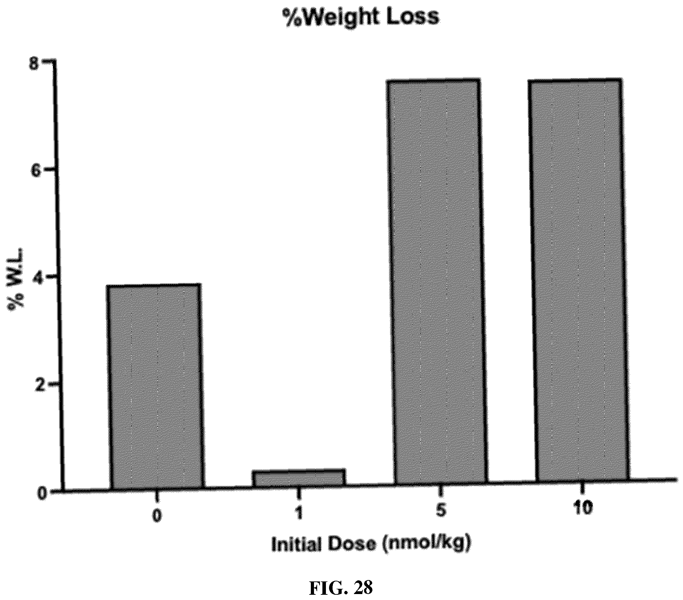

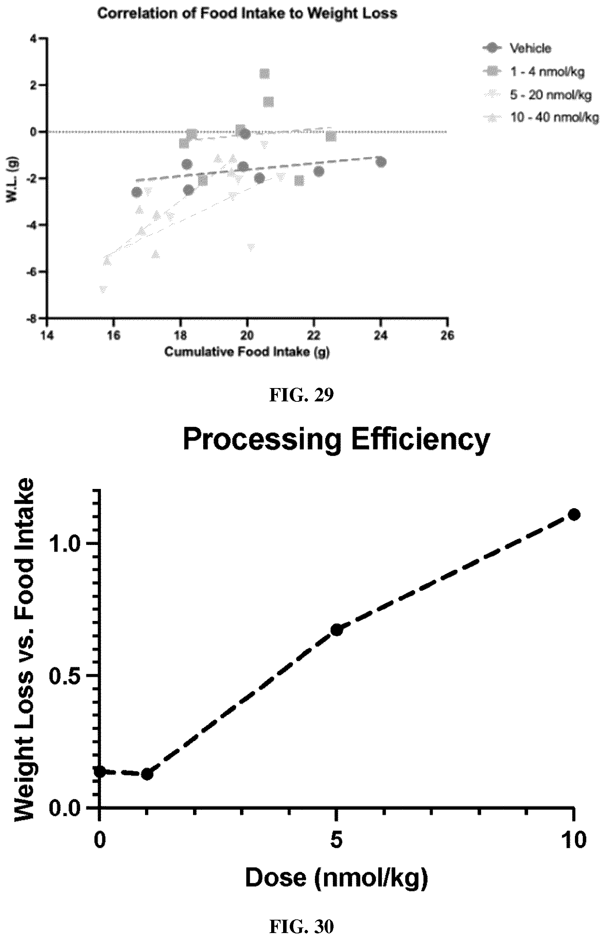

**FIG. 28** shows the average body weight changes in diet-induced obese (DIO) mice over 30 days of BXL-801 treatment. Four groups were compared: vehicle control and three BXL-801 dose ranges (1-4 nmol/kg, 5-20 nmol/kg, and 10-40 nmol/kg). Higher doses demonstrate greater weight loss. **FIG. 29** shows the correlation of cumulative food intake to weight loss for each treatment group. Higher doses show a greater correlation between food intake and weight loss.

A significant dose-dependent increase in weight loss was observed across the different dosing groups (1-4 nmol/kg, 5-20 nmol/kg, and 10-40 nmol/kg). Higher doses resulted in greater weight loss. Statistical analysis was performed once administration of all dose groups was completed. A one-way ANOVA was performed and revealed a significant difference in weight loss among the groups (P = 0.00118). BXL-801 effectively engaged its biological targets, producing measurable weight reduction, particularly at higher doses.

### III(B). Metabolic Processing Efficiency

**FIG. 30** shows the weight Loss versus food intake with processing efficiency during the study for each dose group. **FIG. 30** illustrates the slopes of weight loss vs. food intake regression lines for each dose group. The increasing slope at higher doses suggests improved metabolic efficiency and provides evidence of BXL-801's broader metabolic impact in vivo.

The weight loss observed was not solely attributable to reduced food intake. At higher doses, greater weight loss was achieved than would be predicted by food intake alone, indicating enhanced metabolic processing.

One-way ANOVA was performed and did not show a statistically significant difference in food intake across groups (P = 0.0712). The results shown in FIG. 3 suggest that higher doses of BXL-801 may improve metabolic efficiency and suggest that BXL-801 has broader impact beyond appetite suppression.

### III(C). Body Composition

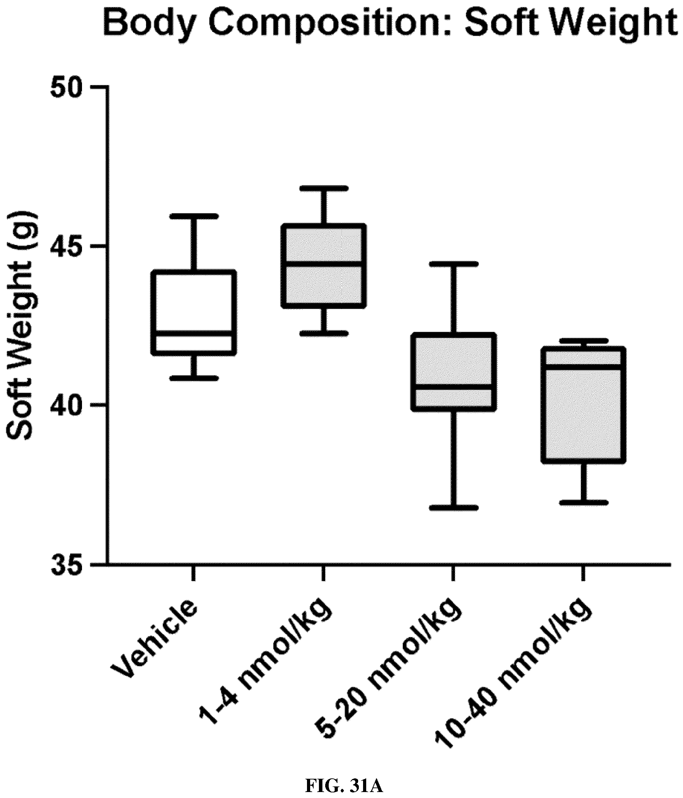

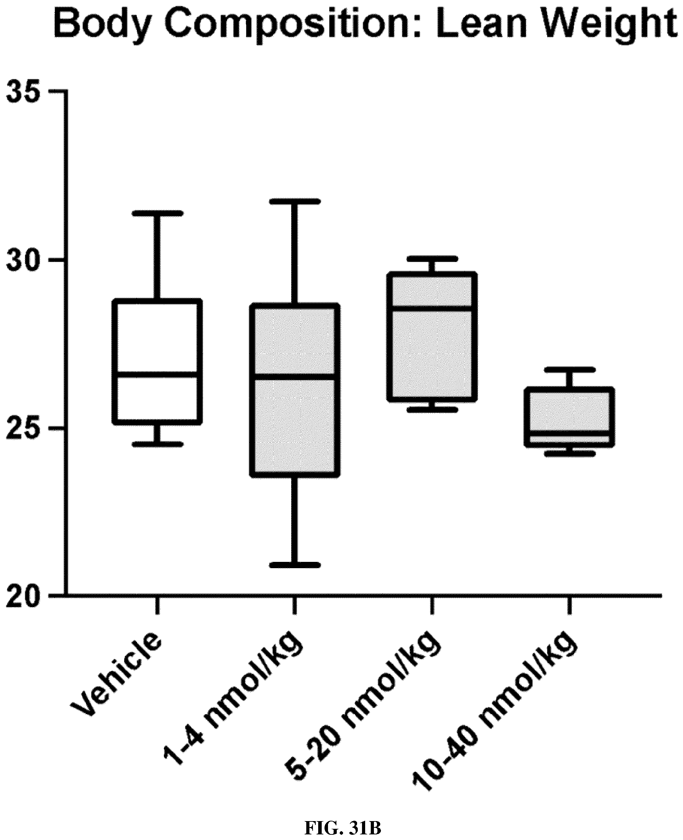

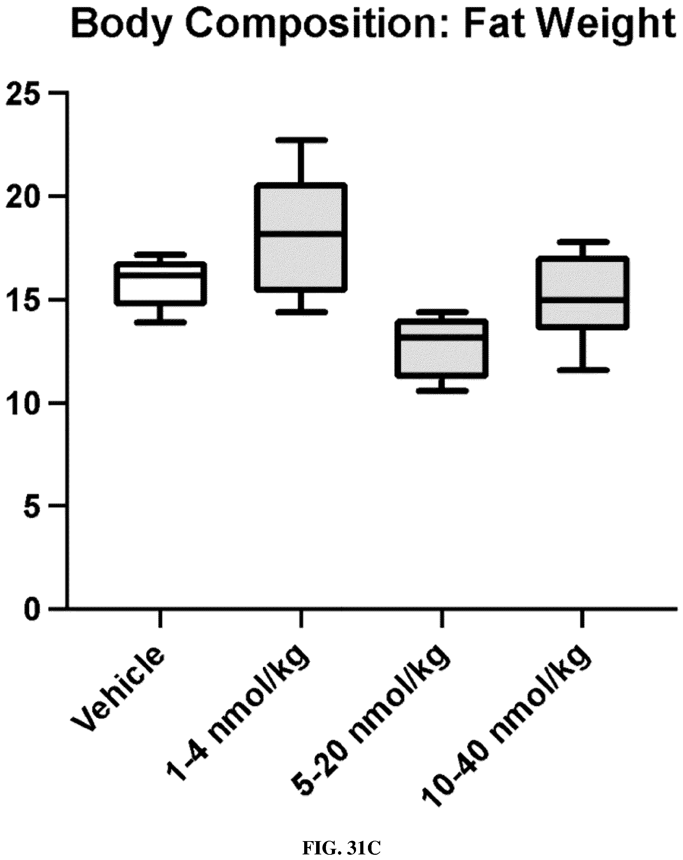

**FIGS. 31A** through **31C** show the changes in soft weight **(****FIG. 31A****),** lean weight **(****FIG. 31B****),** and fat weight **(****FIG. 31C****)** across four groups, Vehicle, 1-4 nmol/kg of BXL-801, 5-20 nmol/kg of BXL-801, and 10-40 nmol/kg of BXL-801, during the study.

The body composition data indicated that higher doses of BXL-801 resulted in significant fat loss in the 5-20 nmol/kg and 10-40 nmol/kg groups, while lean mass was preserved or slightly increased.

One-way ANOVA was performed and confirmed a significant difference in soft weight (P = 0.000486), with the higher doses resulting in reduced fat weight while sparing lean mass. BXL-801 demonstrated its therapeutic potential in promoting a healthier body composition, making it a more favorable option compared to treatments that result in muscle loss.

The results demonstrated a significant reduction in fat weight in the higher dose groups, while lean mass is preserved. The data highlighted BXL-801's ability to promote a healthier body composition by reducing fat without adversely affecting muscle mass.

### III(D). Glucose Tolerance and Metabolic Control

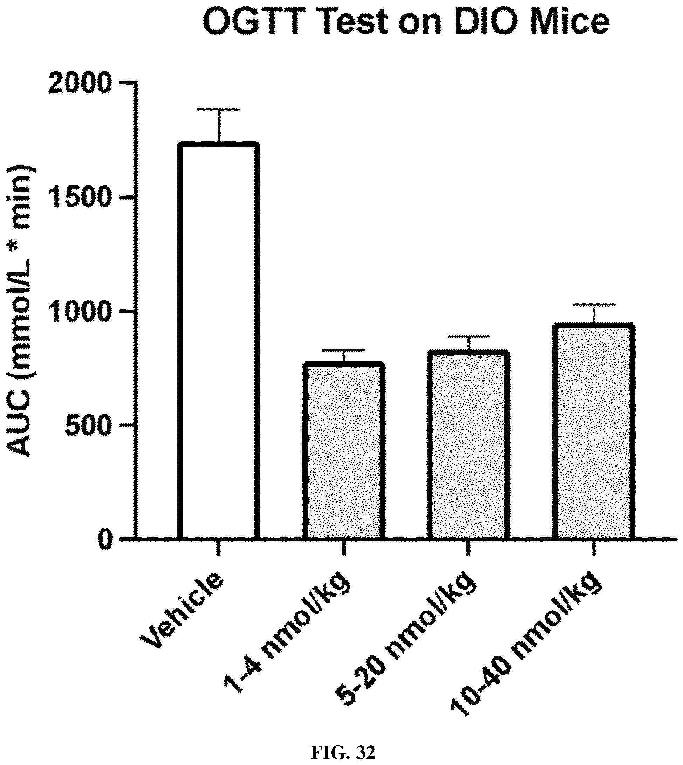

**FIG. 32** shows the oral glucose tolerance (OGTT) results for diet-induced obese (DIO) mice across four groups: Vehicle (control), 1-4 nmol/kg of BXL-801, 5-20 nmol/kg of BXL-801, and 10-40 nmol/kg of BXL-801. Glucose levels (measured as area under the curve, AUC) were significantly reduced in the treated groups compared to the vehicle groups indicating improved glucose tolerance of BXL-801. The reduction in glucose levels suggests that BXL-801 enhances insulin sensitivity and glucose regulation, providing dual benefits in both weight management and glycemic control.

Oral glucose tolerance test (OGTT) data showed that glucose handling improved significantly in the 1-4 nmol/kg, 5-20 nmol/kg, and 10-40 nmol/kg groups of BXL-801compared to the vehicle group.

ANOVA analysis was performed and confirmed a highly significant difference in OGTT results (P = 6.31e-19) among the groups. BXL-801 greatly enhanced insulin sensitivity and glucose regulation, providing a dual benefit in weight management and glycemic control.

### III(E). Glucose Tolerance and Metabolic Control

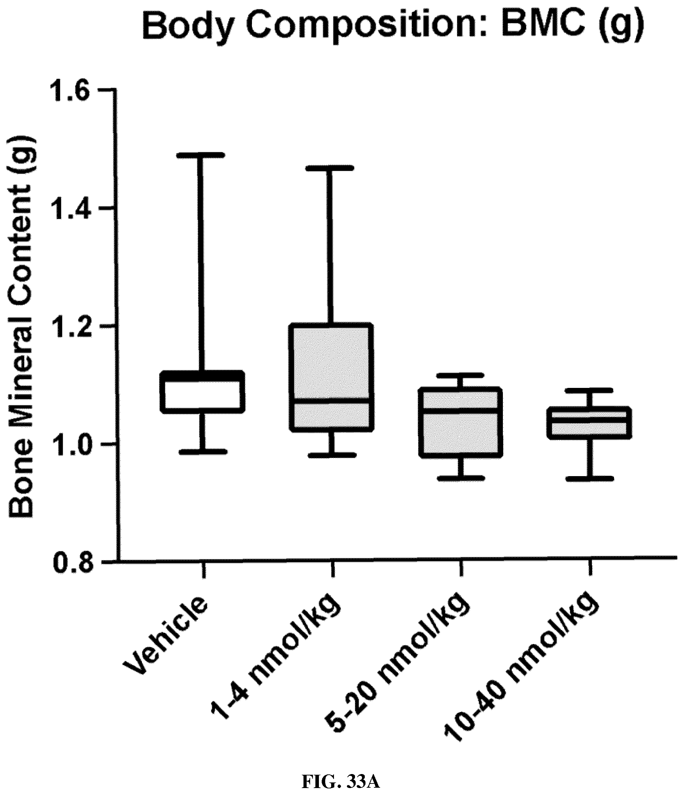

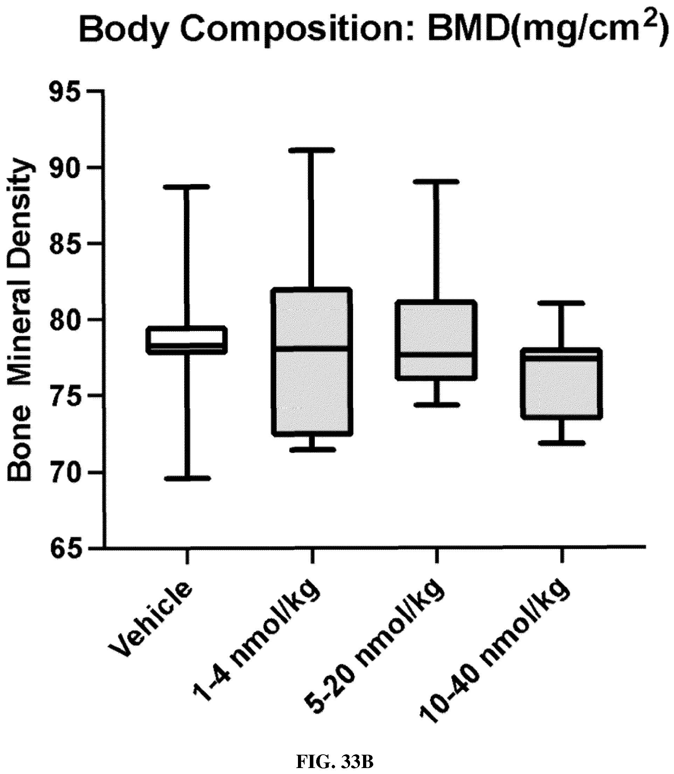

**FIG. 33A** shows the bone mineral content (BMC) and **FIG. 33B** shows bone mineral density (BMD) results from the study for all dosage groups. Even at higher doses, BXL_801 did not negatively impact bone health, as evidenced by stable BMC and BMD values across all groups. These findings suggest that BXL-801 provides effective weight loss while preserving bone integrity, making it a favorable candidate for long-term treatment of obesity and type 2 diabetes.

Bone mineral content (BMC) and bone mineral density (BMD) were not negatively affected by BXL-801 treatment, even in the higher dose groups.

ANOVA was performed and did not indicate statistically significant differences in lean weight (P = 0.1416). The preservation of bone health alongside weight loss is an encouraging sign that BXL-801 may be a safe long-term option for treating obesity without compromising skeletal integrity.

### IV. Conclusion

The BXL-801 branched peptide (Compound A-1), targeting both GLP-1R and GIPR, demonstrated dose-dependent effects on weight loss, glucose tolerance, and body composition in a DIO mouse model. These results highlighted BXL-801's ability to reduce fat mass while preserving lean mass and bone health. Additionally, BXL-801 significantly improved glucose tolerance, indicating its dual potential as a therapy for both obesity and type 2 diabetes and providing a comprehensive approach to treating metabolic disorders.

While certain features of the invention have been illustrated and described herein, many modifications, substitutions, changes, and equivalents will now occur to those of ordinary skill in the art. It is, therefore, to be understood that the appended claims are intended to cover all such modifications and changes as fall within the true spirit of the invention.

Embodiments of the invention may further be described using the following clauses:
1. A multimeric polypeptide comprising:
   at least two polypeptide units, wherein each polypeptide unit is covalently linked to at least one other polypeptide unit via a linker moiety;
   wherein at least one polypeptide is a G-coupled polypeptide receptor (GPCR) ligand.
2. The multimeric polypeptide of clause 1 comprising two, three, four, five, or at least six polypeptide units.
3. The multimeric polypeptide of any of clauses 1-2 wherein each of the polypeptide units comprises a different amino acid sequence from each of the other polypeptide units.
4. The multimeric polypeptide of any of clauses 1-2 wherein at least one of the polypeptide units comprises the same amino acid sequence as another polypeptide unit.
5. The multimeric polypeptide of any of clauses 1-4 wherein each of the polypeptide units is bound to a linker moiety.
6. The composition of any of clauses 1-5 wherein the linker moiety comprises a linear or branched structure comprising a plurality of reactive sites that covalently link the at least two polypeptides.
7. The composition of clause 6 wherein the plurality of reactive sites of the linker moiety comprise a combination of one or more independent amine, thiol, or halogen reactive sites.
8. The composition of any of clauses 6-7 wherein the plurality of reactive sites of the linker moiety comprise one or more azides or alkynes.
9. The composition of any of clauses 6-8 wherein the plurality of reactive sites of the linker moiety comprise one or more orthogonal protecting groups that may be removed independently of other protecting groups.
10. The multimeric polypeptide of any of clauses 1-9, wherein the linker moiety comprises a polymer linker.
11. The multimeric polypolypeptide of any of clauses 1-10, wherein the linker moiety comprises an alpha amino acid, a beta-amino acid or a gamma-amino acid.
12. The multimeric polypolypeptide of any of clauses 1-11, wherein the linker moiety comprises a d-amino acid.
13. The multimeric polypolypeptide of any of clauses 1-12, wherein the linker moiety comprises an ethylene glycol oligomer.
14. The multimeric polypeptide of any of clauses 1-13, wherein the linker moiety comprises one or more independent PEG4, PEG8, PEG20, 2x(GGGGS), 4x(GGGGS), 8xG, GSAGSAAGSGEF, A-(EAAAK)-A, A-3x(EAAAK)-A, 7x(AP), KESGSVSSEQLAQFRSLD or EGKSSGSGSESKST.
15. The multimeric polypeptide of any of clauses 1-14, comprising at least one non-peptidic molecule covalently linked to any portion of any of the polypeptide units or the linker moiety.
16. The multimeric polypeptide of clause 15 wherein the non-peptidic molecule is linked by an amido-PEG-acid or maleimide-PEG-acid.
17. The multimeric polypeptide of clause 15 wherein the at least one non-peptidic molecule is a sodium-glucose cotransporter-2 (SGLT-2) inhibitor.
18. The multimeric polypeptide of clause 15 wherein the at least one non-peptidic molecule is a lipid, albumin or antibody.
19. The multimeric polypeptide of clause 15 wherein the at least one non-peptidic molecule is a lipid.
20. The multimeric polypeptide of clause 19 wherein the lipid is bound to a lysine.
21. The multimeric polypeptide of any off clauses 1-20, wherein at least one polypeptide comprises at least one modified amino acid, wherein the modification is any of acetylation, amidation, C-linked glycosylation, citrullination, crotonylation, formylation, glutarylation, glutathionylation, hydroxylation, lipidation, malonylation, methylation, myristoylation, N-linked glycosylation, O-linked glycosylation, oxidation, palmitoylation, phosphorylation, pyrrolidone carboxylic acid, S-nitrosylation, sulfation, or sumoylation.
22. The multimeric polypeptide of any of clauses 1-21, wherein at least one of the polypeptide units is N-terminus acetylated.
23. The multimeric polypeptide of any of clauses 1-22, wherein at least one of the polypeptide units comprises one or more non-canonical amino acid.
24. The multimeric polypeptide of clause 23 wherein the non-canonical amino acid is 2-aminoisobutyric acid.
25. The multimeric polypeptide of clause 17 wherein the SGLT-2 inhibitor is represented by Formula I 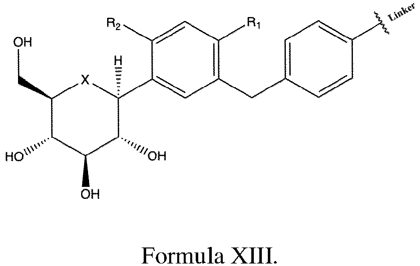 wherein
   "Linker" refers to a linker covalently linking to the polypeptide unit
   R₁ = -H, -OH, or a halogen;
   R₂ = -H, -OH, or a halogen; and
   X = S or O.
26. The multimeric polypeptide of any of clauses 1-25, wherein the GPCR ligand is to GLP-1R, GIPR, or CRLR.
27. The multimeric polypeptide of any of clauses 1-26, wherein the multimeric polypeptide comprises polypeptide sequences with greater than about 70% sequence homology to the respective native GPCR ligand.
28. The multimeric polypeptide of any of clauses 1-27, wherein the multimeric polypeptide comprises polypeptide sequences of:
   a. GLP-1 and GIP;
   b. GLP-1 and an amylin variant; or
   c. GLP-1, GIP, and an amylin variant;

   optionally wherein at least one lysine of one or more polypeptides is lipidated, and
   optionally wherein a conjugate molecule is bound thereto, and wherein each GPCR ligand has greater than about 70% homology with the respective native GPCR ligand.
29. The multimeric polypeptide of any of clauses 1-28, wherein at least one or all of the polypeptide units comprise native GPCR ligands.
30. The multimeric polypeptide of any of clauses 1-29, wherein the GLP-1 polypeptide unit comprises a peptide with at least about 70% sequence homology to Ac-HXEGT FTSDV SSYLE GQAAK EFIAW LVKGR G;
   wherein Ac refers to an N-terminal acetyl, X refers to 2-aminoisobutyric acid or alanine, and K refers to an optionally lipidated lysine residue.
31. The multimeric polypeptide of any of clauses 1-30, wherein the GIP polypeptide unit comprises a peptide with at least about 70% sequence homology to
   Ac-YXEGT FISDY SIAMD KIHQQ DFVNW LLAQK GKKND WKHNI TQ;
   wherein Ac refers to an N-terminal acetyl, X refers to 2-aminoisobutyric acid or alanine, and K refers to an optionally lipidated lysine residue.
32. The multimeric polypeptide of any of clauses 1-31, wherein the amylin variant polypeptide unit comprises a peptide with at least about 70% sequence homology to Ac-KCNTA TCATQ RLAEF LRHSS NNFGP ILPPT NVGSN TP
   wherein Ac refers to an N-terminal acetyl, and K refers to an optionally lipidated lysine residue.
33. The multimeric polypeptide of any of clauses 1-32 selected from among: 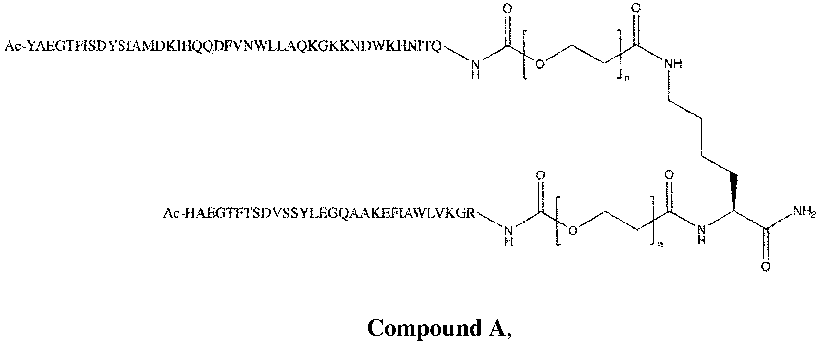 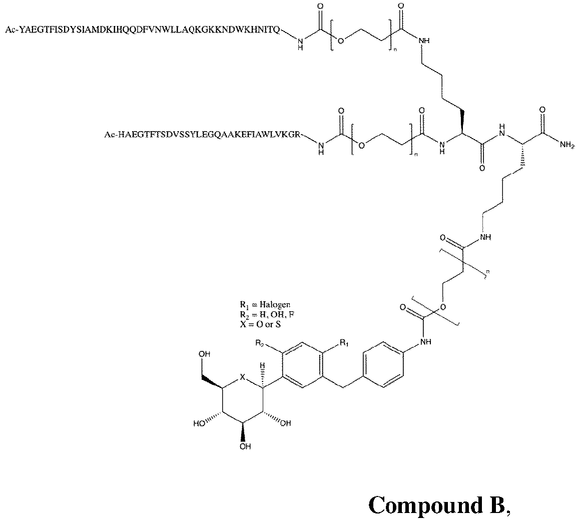 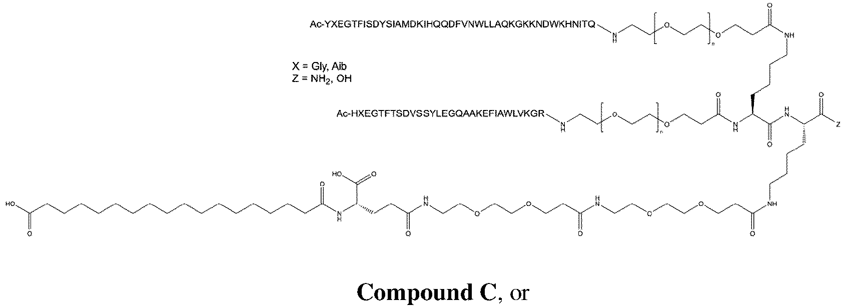 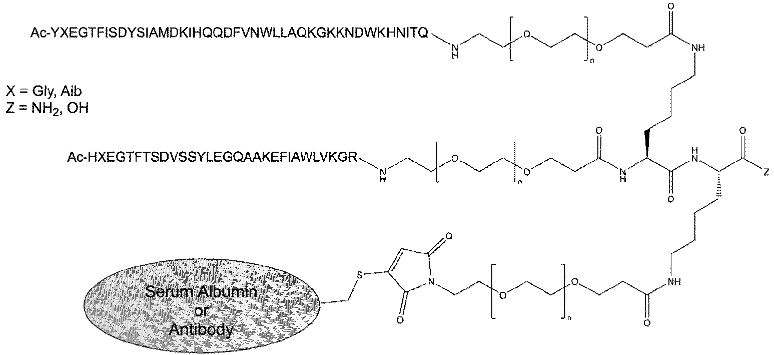 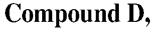 wherein n is any number selected from between 1 to 20.
34. The multimeric polypeptide of clause 33, wherein said multimeric polypeptide is represented by the formula of Compound A-1 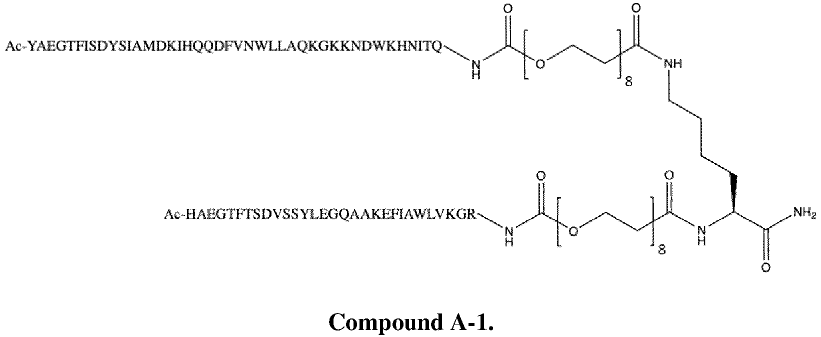
35. The multimeric polypeptide of clause 33, wherein said multimeric polypeptide is represented by any one of Formulas I through XII: 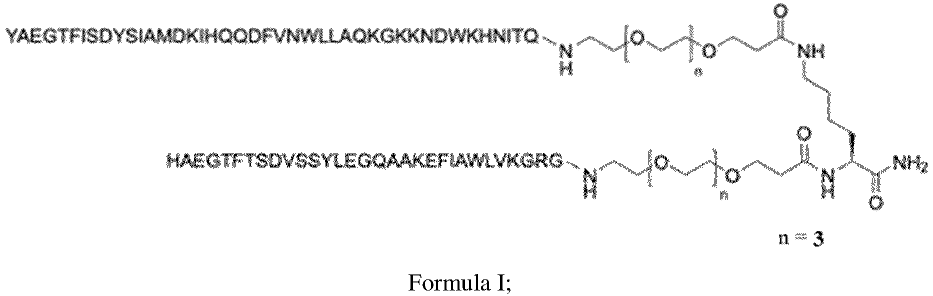 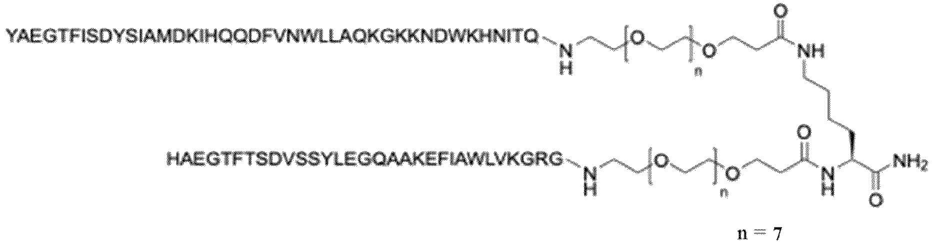 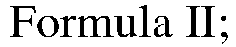 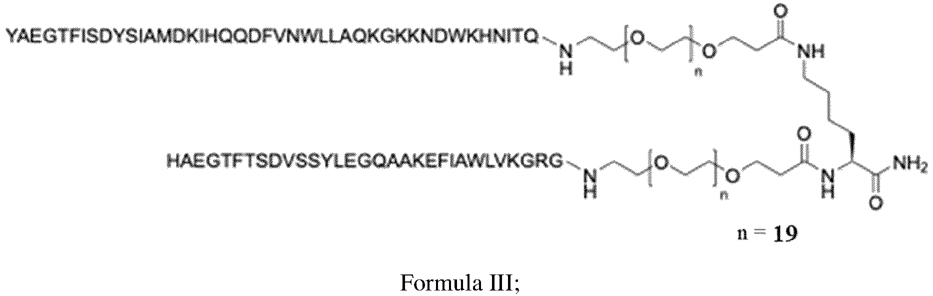 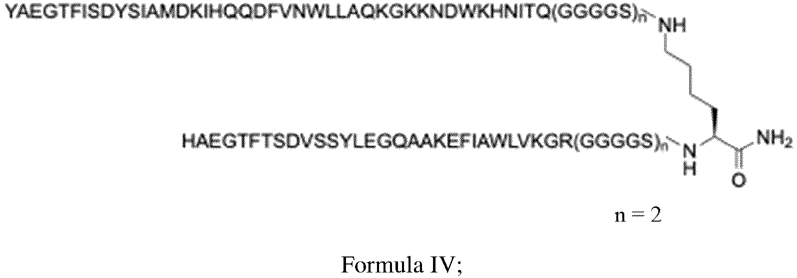 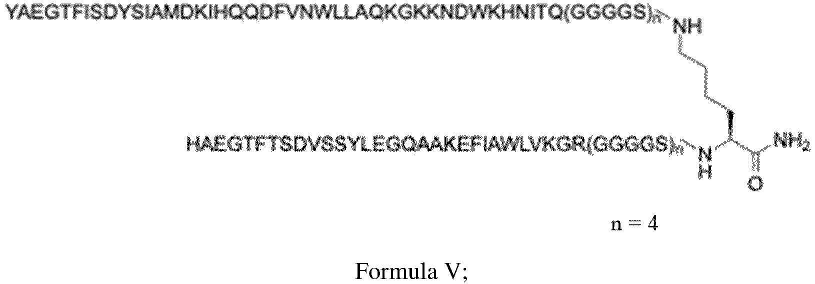 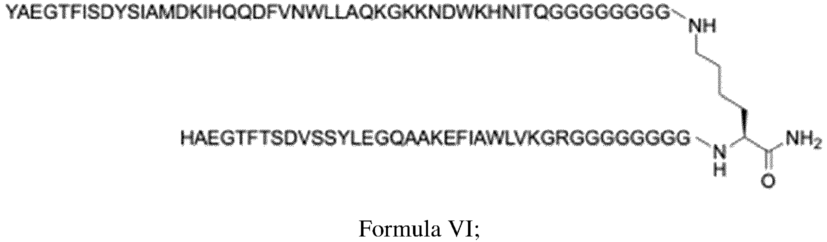 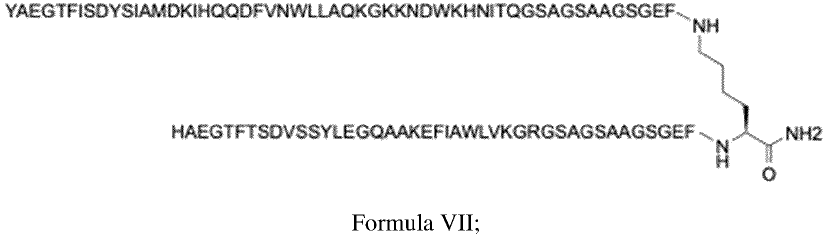 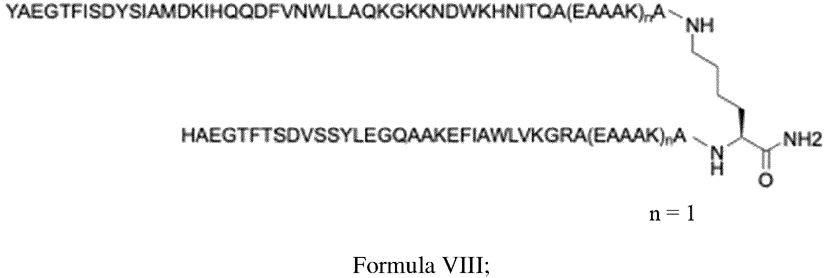 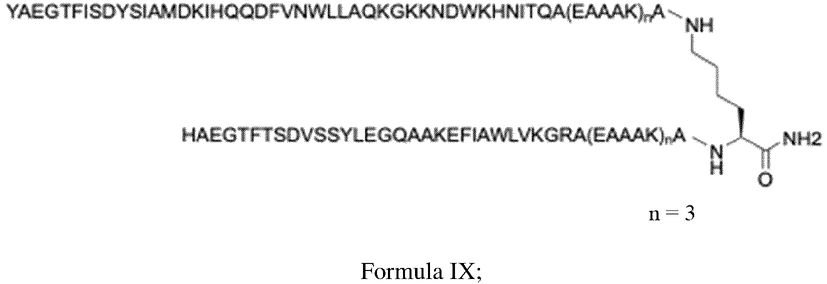 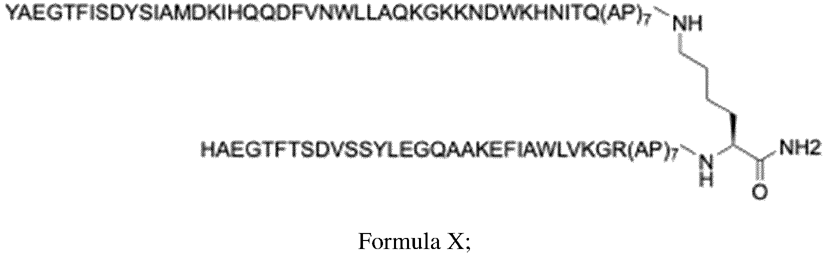 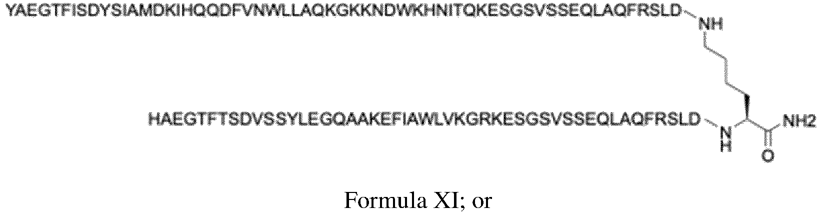 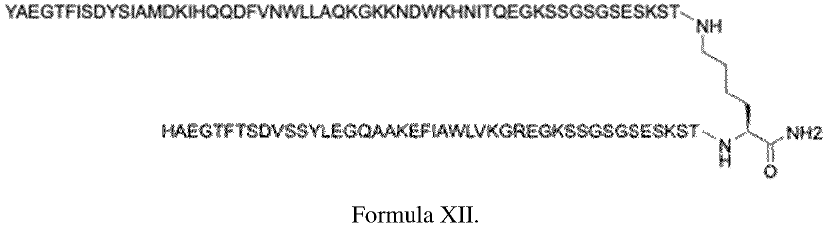
36. The multimeric polypeptide of clause 35, wherein the GIP polypeptide unit is attached to the linker at the C-terminus of said GIP polypeptide.
37. The multimeric polypeptide of any one of clauses 35-36, wherein the GLP-1 polypeptide unit is attached to the linker at the C-terminus of said GLP-1 polypeptide.
38. A multimeric polypeptide of clause 1 comprising a GLP-1 polypeptide unit linked via a linker moiety to a GIP polypeptide unit, an amylin polypeptide unit, or the combination thereof.
39. The multimeric polypeptide molecule of clause 38 further comprising a small-molecule SGLT2 inhibitor covalently linked to any one of the units.
40. The multimeric polypeptide of any of clauses 1-39, wherein the linker moiety is bound to an amine of a C-terminal lysine appended to the C-terminus of each polypeptide unit.
41. A pharmaceutical composition comprising a multimeric polypeptide of any one of clauses 1-40 and a pharmaceutically-acceptable excipient, diluent, vehicle or carrier.
42. The pharmaceutical composition of clause 41, comprising 0.05% polysorbate-80.
43. The pharmaceutical composition of any one of clauses 42-42, comprising 50 mM sodium phosphate.
44. The pharmaceutical composition of any one of clauses 42-43, comprising 70 mM sodium chloride.
45. The pharmaceutical composition of any one of clauses 42-44, wherein said pharmaceutical composition is at a pH of 7.4.
46. The pharmaceutical composition of clause 42, comprising 0.05% polysorbate-80, 50 mM sodium phosphate, and 70 mM sodium chloride at a of pH 7.4.
47. A method for modulating at least one GPCR in a subject comprising administering to the subject a multimeric polypeptide of any one of clauses 1-40 or the pharmaceutical composition of any one of clauses 41-46.
48. The method of clause 47 wherein GLP-1R and GIPR are modulated.
49. The method of clause 47 wherein GLP-1R, GIPR and CRLR are modulated.
50. A method for treating obesity or type 2 diabetes comprising administering to a subject in need an effective amount of a composition of any one of clauses 1-40 or a pharmaceutical composition of any one of clauses 41-46.
51. A method for treating obesity and type 2 diabetes comprising administering to a subject in need an effective amount of a composition of any one of clauses 1-40 or a pharmaceutical composition of any one of clauses 41-46.
52. The method of any one of clauses 50-51, wherein said multimeric polypeptide is administered at between 1 nmoles/kg to 80 nmoles/kg of patient body weight.
53. The method of clause 52, wherein said multimeric polypeptide is administered at 1 nmol/kg, 2 nmol/kg, 4 nmol/kg, 5 nmol/kg, 10 nmol/kg, and 20 nmol/kg, or 40 nmol/kg of patient body weight.
54. The method of any one of clauses 50-53, wherein said administration reduces fat without adversely affecting muscle mass.
55. The method of any one of clauses 50-54, wherein said pharmaceutical composition is administered subcutaneously.
56. A multimeric polypeptide of any one of clauses 1-40 or the pharmaceutical composition of any one of clauses 41-46 for use as a medicament.
57. The multimeric polypeptide or the pharmaceutical composition for use as a medicament according to clause 56, by modulating at least one GPCR in a subject.
58. The multimeric polypeptide or the pharmaceutical composition for use according to clause 57 wherein GLP-1R and GIPR are modulated, or wherein GLP-1R, GIPR and CRLR are modulated.
59. A multimeric polypeptide of any one of clauses 1-40 or the pharmaceutical composition of any one of clauses 41-46 for use in treating obesity or type 2 diabetes, or both obesity and type 2 diabetes, in a subject.
60. The multimeric polypeptide or the pharmaceutical composition for use according to clause 59, wherein said multimeric polypeptide is administered at between 1 nmoles/kg to 80 nmoles/kg of patient body weight.
61. The multimeric polypeptide or the pharmaceutical composition for use according to clause 59 or 60, wherein said multimeric polypeptide is administered at 1 nmol/kg, 2 nmol/kg, 4 nmol/kg, 5 nmol/kg, 10 nmol/kg, and 20 nmol/kg, or 40 nmol/kg of patient body weight.
62. The multimeric polypeptide or the pharmaceutical composition for use according to any of clauses 59-61, wherein said multimeric polypeptide or pharmaceutical composition reduces fat without adversely affecting muscle mass.
63. The multimeric polypeptide or the pharmaceutical composition for use according to any of clauses 59-62, wherein said multimeric polypeptide or pharmaceutical composition is administered subcutaneously.

## Claims

1. A multimeric polypeptide comprising:
at least two polypeptide units, wherein each polypeptide unit is covalently linked to at least one other polypeptide unit via a linker moiety;
wherein at least one polypeptide is a G-coupled polypeptide receptor (GPCR) ligand.

2. The multimeric polypeptide of claim 1:
a. comprising two, three, four, five, or at least six polypeptide units;
b. wherein each of the polypeptide units comprises a different amino acid sequence from each of the other polypeptide units or wherein at least one of the polypeptide units comprises the same amino acid sequence as another polypeptide unit;
c. wherein each of the polypeptide units is bound to a linker moiety;
d. wherein the linker moiety comprises a linear or branched structure comprising a plurality of reactive sites that covalently link the at least two polypeptides;
e. wherein the linker moiety comprises a linear or branched structure comprising a plurality of reactive sites that covalently link the at least two polypeptides and wherein the plurality of reactive sites of the linker moiety comprise: a combination of one or more independent amine, thiol, or halogen reactive sites; one or more azides or alkynes; and/or one or more orthogonal protecting groups that may be removed independently of other protecting groups;
f. wherein the linker moiety comprises a polymer linker;
g. wherein the linker moiety comprises an alpha amino acid, a beta-amino acid, a gamma-amino acid, a d-amino acid, or an ethylene glycol oligomer; and/or
h. wherein the linker moiety comprises one or more independent PEG4, PEG8, PEG20, 2x(GGGGS), 4x(GGGGS), 8xG, GSAGSAAGSGEF, A-(EAAAK)-A, A-3x(EAAAK)-A, 7x(AP), KESGSVSSEQLAQFRSLD or EGKSSGSGSESKST.

3. The multimeric polypeptide of any of claims 1-2, comprising at least one non-peptidic molecule covalently linked to any portion of any of the polypeptide units or the linker moiety and wherein:
a. the non-peptidic molecule is linked by an amido-PEG-acid or maleimide-PEG-acid;
b. the at least one non-peptidic molecule is a sodium-glucose cotransporter-2 (SGLT-2) inhibitor;
c. the at least one non-peptidic molecule is a lipid, albumin or antibody;
d. the at least one non-peptidic molecule is a lipid and is bound to a lysine; and/ or
e. the at least one non-peptidic molecule is a sodium-glucose cotransporter-2 (SGLT-2) inhibitor and is represented by Formula I 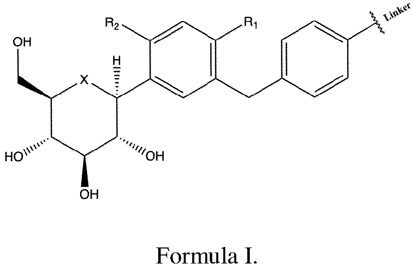 wherein
"Linker" refers to a linker covalently linking to the polypeptide unit R₁ = -H, -OH, or a halogen;
R₂ = -H, -OH, or a halogen; and
X = S or O.

4. The multimeric polypeptide of any of claims 1-3:
a. wherein at least one polypeptide comprises at least one modified amino acid, wherein the modification is any of acetylation, amidation, C-linked glycosylation, citrullination, crotonylation, formylation, glutarylation, glutathionylation, hydroxylation, lipidation, malonylation, methylation, myristoylation, N-linked glycosylation, O-linked glycosylation, oxidation, palmitoylation, phosphorylation, pyrrolidone carboxylic acid, S-nitrosylation, sulfation, or sumoylation;
b. wherein the linker moiety is bound to an amine of a C-terminal lysine appended to the C-terminus of each polypeptide unit;
c. wherein at least one of the polypeptide units is N-terminus acetylated;
d. wherein at least one of the polypeptide units comprises one or more non-canonical amino acid;
e. wherein at least one of the polypeptide units comprises one or more non-canonical amino acid and said non-canonical amino acid is 2-aminoisobutyric acid;
f. wherein the GPCR ligand is to GLP-1R, GIPR, or CRLR; and/or
g. wherein the multimeric polypeptide comprises polypeptide sequences with greater than about 70% sequence homology to the respective native GPCR ligand.

5. The multimeric polypeptide of any of claims 1-4, wherein the multimeric polypeptide comprises polypeptide sequences of:
a. GLP-1 and GIP;
b. GLP-1 and an amylin variant; or
c. GLP-1, GIP, and an amylin variant;
optionally wherein at least one lysine of one or more polypeptides is lipidated, and
optionally wherein a conjugate molecule is bound thereto, and wherein each GPCR ligand has greater than about 70% homology with the respective native GPCR ligand.

6. The multimeric polypeptide of any of claims 1-5:
a. wherein at least one or all of the polypeptide units comprise native GPCR ligands;
b. wherein the GIP polypeptide unit is attached to the linker at the C-terminus of said GIP polypeptide and/or wherein the GLP-1 polypeptide unit is attached to the linker at the C-terminus of said GLP-1 polypeptide;
c. wherein the GLP-1 polypeptide unit comprises a peptide with at least about 70% sequence homology to Ac-HXEGT FTSDV SSYLE GQAAK EFIAW LVKGR G;
wherein Ac refers to an N-terminal acetyl, X refers to 2-aminoisobutyric acid or alanine, and K refers to an optionally lipidated lysine residue;
d. wherein the GIP polypeptide unit comprises a peptide with at least about 70% sequence homology to
Ac-YXEGT FISDY SIAMD KIHQQ DFVNW LLAQK GKKND WKHNI TQ;
wherein Ac refers to an N-terminal acetyl, X refers to 2-aminoisobutyric acid or alanine, and K refers to an optionally lipidated lysine residue; and/or
e. wherein the amylin variant polypeptide unit comprises a peptide with at least about 70% sequence homology to
Ac-KCNTA TCATQ RLAEF LRHSS NNFGP ILPPT NVGSN TP;
wherein Ac refers to an N-terminal acetyl, and K refers to an optionally lipidated lysine residue.

7. The multimeric polypeptide of any of claims 1-6 selected from among: 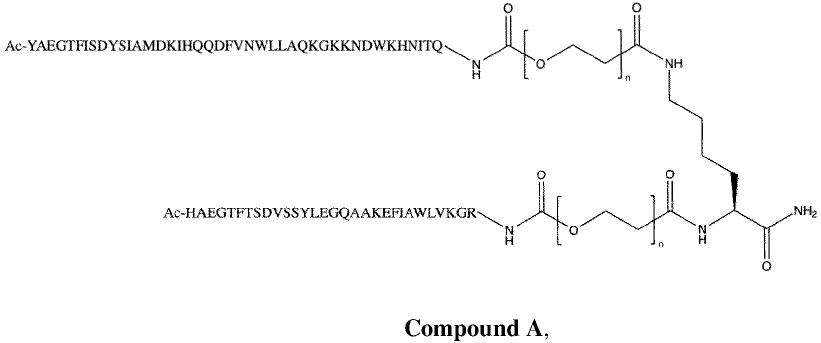 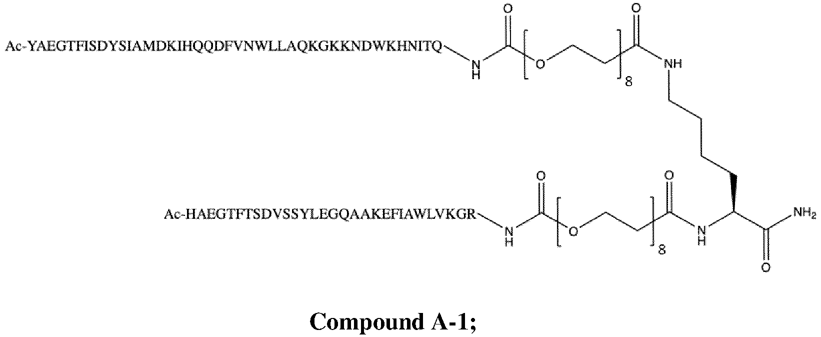 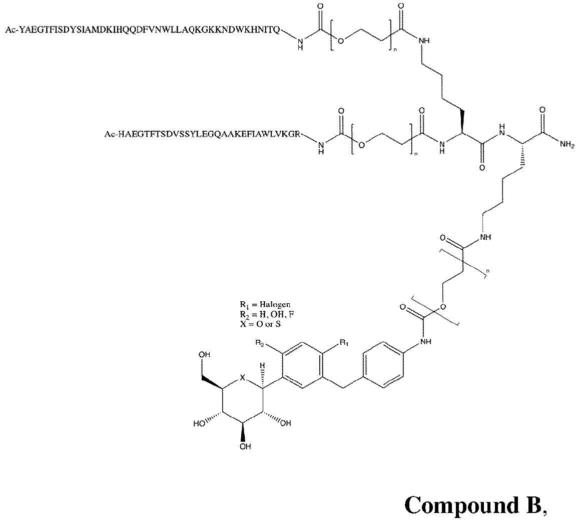 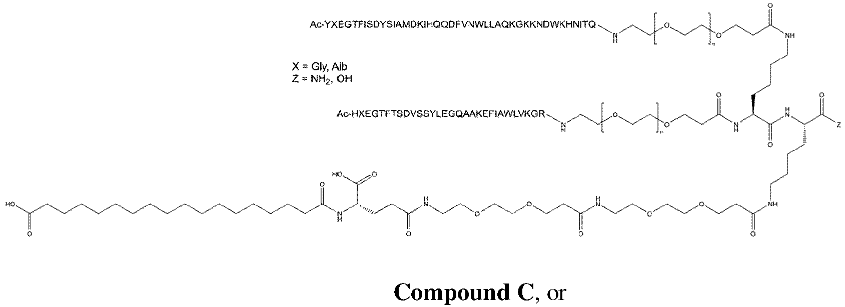 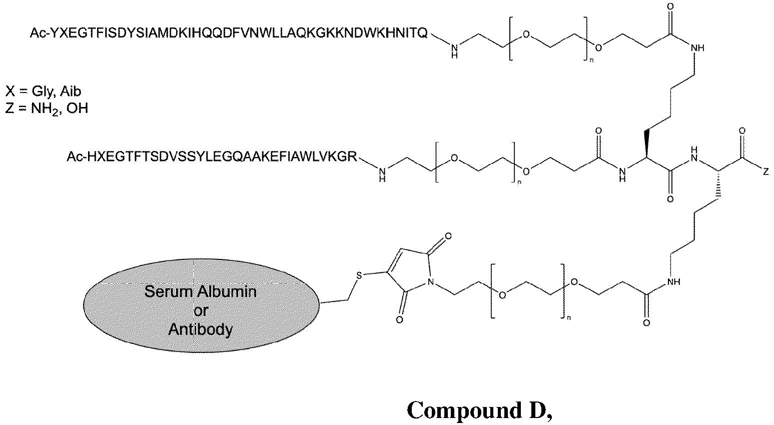 wherein n is any number selected from between 1 to 20.

8. The multimeric polypeptide of any one of claims 1-7, wherein said multimeric polypeptide is represented by any one of Formulas I through XII:

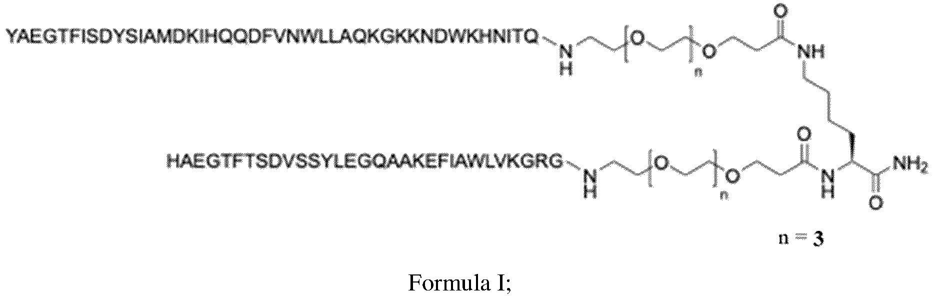

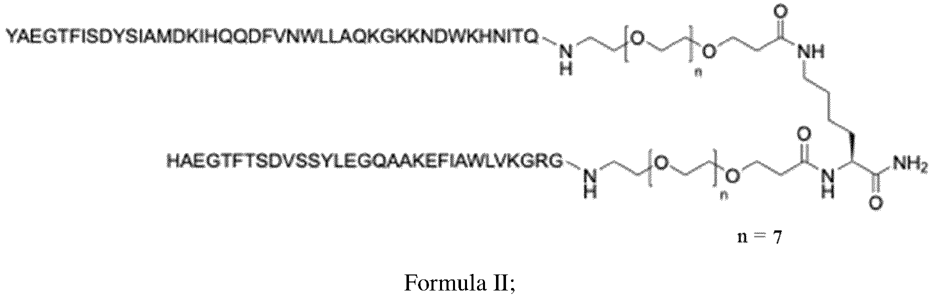

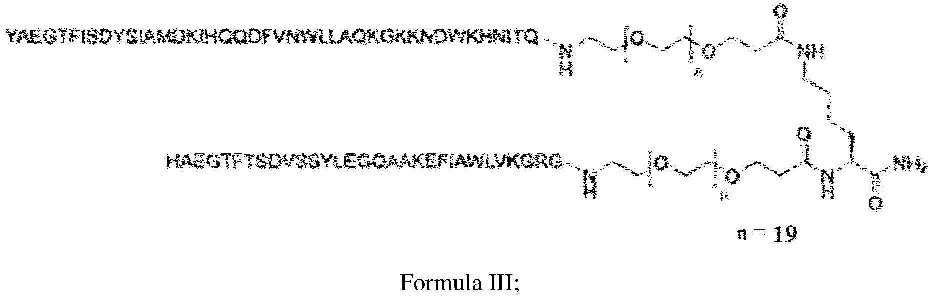

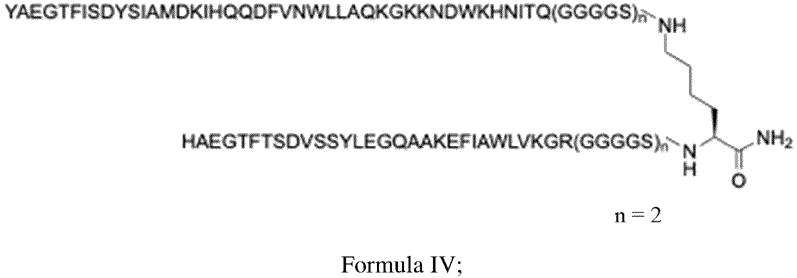

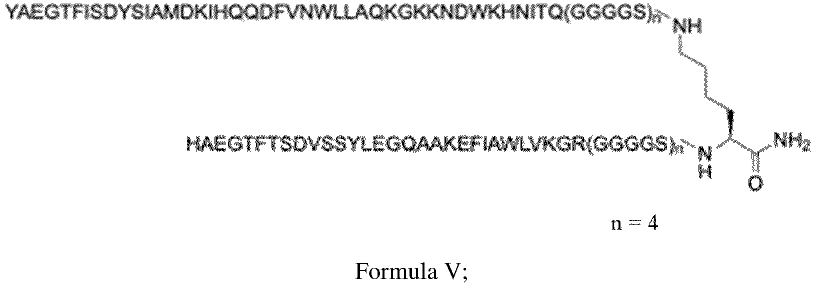

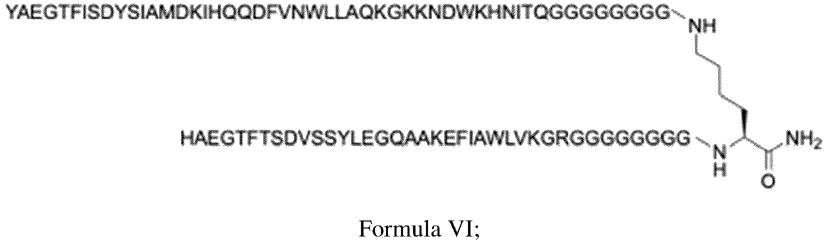

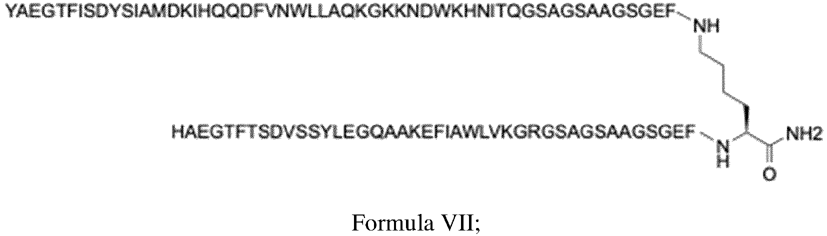

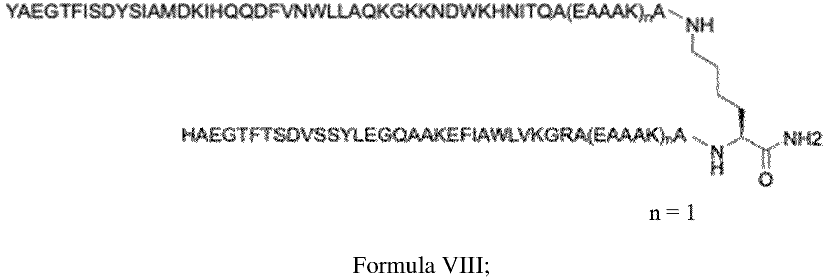

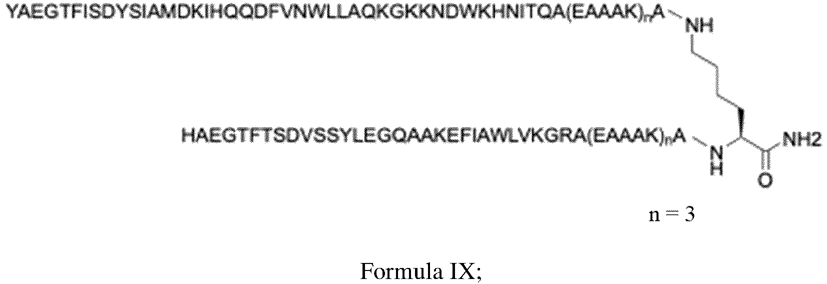

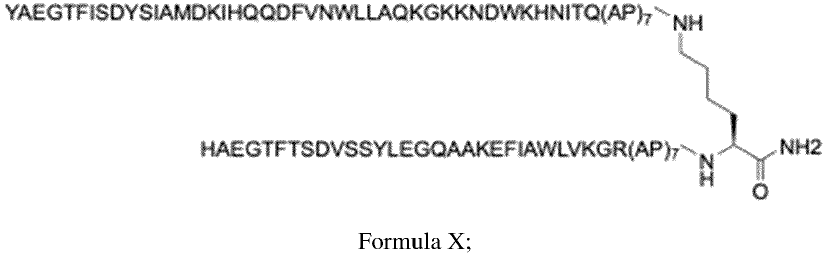

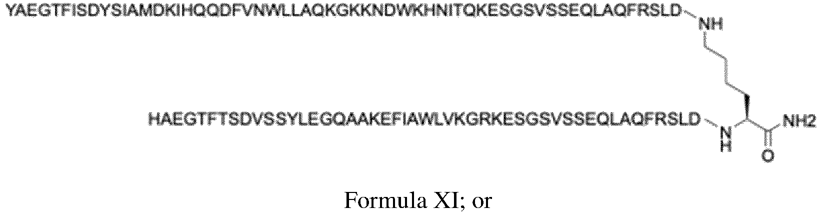

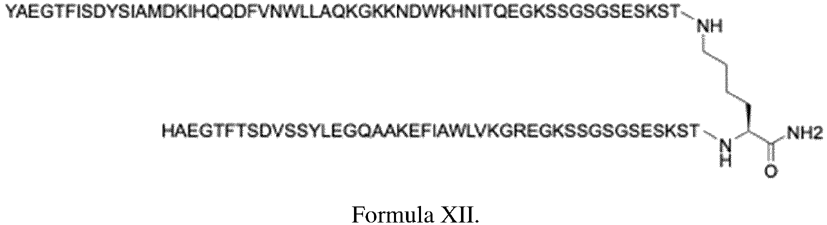

9. A multimeric polypeptide of claim 1 comprising a GLP-1 polypeptide unit linked via a linker moiety to a GIP polypeptide unit, an amylin polypeptide unit, or the combination thereof and further comprising a small-molecule SGLT2 inhibitor covalently linked to any one of the units, and wherein the linker moiety is bound to an amine of a C-terminal lysine appended to the C-terminus of each polypeptide unit.

10. A pharmaceutical composition comprising a multimeric polypeptide of any one of claims 1-9 and a pharmaceutically-acceptable excipient, diluent, vehicle or carrier.

11. The pharmaceutical composition of claim 10 comprising 0.05% polysorbate-80, 50 mM sodium phosphate, and 70 mM sodium chloride at a of pH 7.4.

12. The multimeric polypeptide of any of claims 1-9 or the pharmaceutical composition of any of claims 10-11 for use in modulating at least one GPCR in a subject

13. The multimeric polypeptide or the pharmaceutical composition for use according to claim 12, wherein GLP-1R and GIPR are modulated or wherein GLP-1R, GIPR and CRLR are modulated.

14. The multimeric polypeptide of any of claims 1-9 or the pharmaceutical composition of any of claims 10-11 for use in treating obesity, type 2 diabetes, or both obesity and type 2 diabetes, in a subject.

15. The multimeric polypeptide or pharmaceutical composition for use according to claim 14, wherein:
a. said multimeric polypeptide is administered at between 1 nmoles/kg to 80 nmoles/kg of patient body weight;
b. said multimeric polypeptide is administered at 1 nmol/kg, 2 nmol/kg, 4 nmol/kg, 5 nmol/kg, 10 nmol/kg, and 20 nmol/kg, or 40 nmol/kg of patient body weight;
c. wherein said multimeric polypeptide or pharmaceutical composition reduces fat without adversely affecting muscle mass; and/or
d. said multimeric polypeptide or pharmaceutical composition is administered subcutaneously.
